(19) Europäisches Patentamt
European Patent Office
Office européen des brevets

(11) EP 2 842 542 A1

# (12) EUROPEAN PATENT APPLICATION

(43) Date of publication:
**04.03.2015 Bulletin 2015/10**

(21) Application number: **14169650.0**

(22) Date of filing: **23.05.2014**

(51) Int Cl.:
***A61K 8/44*** *(2006.01)* ***C12N 9/00*** *(2006.01)*
***C12N 9/10*** *(2006.01)* ***C12P 13/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.08.2013 EP 13181841**

(71) Applicant: **Evonik Industries AG**
**45128 Essen (DE)**

(72) Inventors:
- **Grammann, Katrin**
  **45739 Oer-Erkenschwick (DE)**
- **Wolter, Jan**
  **40489 Düsseldorf (DE)**
- **Schaffer, Steffen**
  **45699 Herten (DE)**
- **Corthals, Jasmin**
  **44879 Bochum (DE)**
- **Haas, Thomas**
  **48161 Münster (DE)**
- **Potgrave, Nicole**
  **46286 Dorsten (DE)**

## (54) A method for producing acyl amino acids

(57) The present invention relates to a cell expressing an amino acid-N-acyl-transferase, which is preferably recombinant, and an acyl-CoA synthetase, which is preferably recombinant, wherein the cell has a reduced fatty acid degradation capacity, a method for producing acyl amino acids, comprising the step contacting an amino acid and an acyl CoA in the presence of an amino acid-N-acyl-transferase, which is preferably isolated and/or recombinant, wherein the amino acid-N-acyl-transferase is preferably a human amino acid-N-acyl-transferase, or culturing the cell and a reaction mixture comprising an amino acid-N-acyl-transferase, which is preferably isolated and/or recombinant, an acyl-CoA synthetase, which is preferably isolated and/or recombinant, an amino acid and either a fatty acid-CoA or a fatty acid and an acyl-CoA-synthase.

EP 2 842 542 A1

SEQUENCE LISTING

<110> Evonik Industries AG

<120> A method for producing acyl amino acids

<130> 2013P00087EP01

<160> 21

<170> PatentIn version 3.5

<210> 1
<211> 906
<212> DNA
<213> Umbellularia californica

<220>
<221> misc_feature
<223> synUcTE (an acyl-CoA thioesterase) gene (codon-optimized)

<400> 1
```
atgactctag agtggaaacc gaaaccaaaa ctgcctcaac tgctggatga tcacttcggt        60

ctgcacggtc tggtgtttcg tcgtactttc gcaattcgtt cttatgaagt gggtccagat       120

cgttctacct ccatcctggc cgtcatgaac cacatgcagg aagccaccct gaatcacgcg       180

aaatctgttg gtatcctggg tgatggtttc ggcactactc tggaaatgtc taaacgtgac       240

ctgatgtggg tagtgcgtcg cacccacgta gcagtagagc gctaccctac ttggggtgac       300

actgtggaag tcgagtgttg gattggcgcg tccggtaaca atggtatgcg tcgcgatttt       360

ctggtccgtg actgtaaaac gggcgaaatc ctgacgcgtt gcacctccct gagcgttctg       420

atgaacaccc gcactcgtcg cctgtctacc atcccggacg aagtgcgcgg tgagatcggt       480

cctgctttca tcgataacgt ggcagttaaa gacgacgaaa tcaagaaact gcaaaaactg       540

aacgactcca ccgcggacta catccagggc ggtctgactc cgcgctggaa cgacctggat       600

gttaatcagc atgtgaacaa cctgaaatac gttgcttggg tcttcgagac tgtgccggac       660

agcattttcg aaagccatca catttcctct tttactctgg agtaccgtcg cgaatgtact       720

cgcgactccg ttctgcgcag cctgaccacc gtaagcggcg gttctagcga ggcaggtctg       780

gtctgcgacc atctgctgca actggaaggc ggctccgaag tcctgcgtgc gcgtacggag       840

tggcgtccaa agctgacgga ttctttccgc ggcatctccg taattccggc ggaacctcgt       900

gtttaa                                                                   906
```

<210> 2
<211> 159
<212> DNA
<213> Artificial Sequence

<220>
<223> tac promotor for synUcTE expression

<400> 2
ggatccaatt gtgagcggat aacaattacg agcttcatgc acagtgatcg acgctgttga 60

caattaatca tcggctcgta taatgtgtgg atgtggaatt gtgagcgctc acaattccac 120

aacggtttcc ctctagaaat aattttgttt aacaggagg 159

<210> 3
<211> 4977
<212> DNA
<213> Artificial Sequence

<220>
<223> Vector pJ294[Ptac-synUcTE]

<400> 3
accaatgctt aatcagtgag gcacctatct cagcgatctg tctatttcgt tcatccatag 60

ttgcctgact ccccgtcgtg tagataacta cgatacggga gggcttacca tctggcccca 120

gcgctgcgat gataccgcga gaaccacgct caccggctcc ggatttatca gcaataaacc 180

agccagccgg aagggccgag cgcagaagtg gtcctgcaac tttatccgcc tccatccagt 240

ctattaattg ttgccgggaa gctagagtaa gtagttcgcc agttaatagt ttgcgcaacg 300

ttgttgccat cgctacaggc atcgtggtgt cacgctcgtc gtttggtatg gcttcattca 360

gctccggttc ccaacgatca aggcgagtta catgatcccc catgttgtgc aaaaaagcgg 420

ttagctcctt cggtcctccg atcgttgtca gaagtaagtt ggccgcagtg ttatcactca 480

tggttatggc agcactgcat aattctctta ctgtcatgcc atccgtaaga tgcttttctg 540

tgactggtga gtactcaacc aagtcattct gagaatagtg tatgcggcga ccgagttgct 600

cttgcccggc gtcaatacgg gataataccg cgccacatag cagaacttta aaagtgctca 660

tcattggaaa acgttcttcg gggcgaaaac tctcaaggat cttaccgctg ttgagatcca 720

gttcgatgta acccactcgt gcacccaact gatcttcagc atcttttact ttcaccagcg 780

tttctgggtg agcaaaaaca ggaaggcaaa atgccgcaaa aaagggaata agggcgacac 840

ggaaatgttg aatactcata ttcttccttt ttcaatatta ttgaagcatt tatcagggtt 900

attgtctcat gagcggatac atatttgaat gtatttagaa aaataaacaa ataggggtca 960

gtgttacaac caattaacca attctgaaca ttatcgcgag cccatttata cctgaatatg 1020

gctcataaca ccccttgttt gcctggcggc agtagcgcgg tggtcccacc tgaccccatg 1080

ccgaactcag aagtgaaacg ccgtagcgcc gatggtagtg tggggactcc ccatgcgaga 1140

gtagggaact gccaggcatc aaataaaacg aaaggctcag tcgaaagact gggcctttcg 1200

cccgggctaa ttatggggtg tcgcccttat tcgactctat agtgaagttc ctattctcta 1260

gaaagtatag gaacttctga agtgggggcg gccgcaaatt cagtaagcag aaagtcaaaa 1320

gcctccgacc ggaggctttt gactaaagct taaacacgag gttccgccgg aattacggag 1380

atgccgcgga aagaatccgt cagctttgga cgccactccg tacgcgcacg caggacttcg 1440
gagccgcctt ccagttgcag cagatggtcg cagaccagac ctgcctcgct agaaccgccg 1500
cttacggtgg tcaggctgcg cagaacggag tcgcgagtac attcgcgacg gtactccaga 1560
gtaaaagagg aaatgtgatg gctttcgaaa atgctgtccg gcacagtctc gaagacccaa 1620
gcaacgtatt tcaggttgtt cacatgctga ttaacatcca ggtcgttcca gcgcggagtc 1680
agaccgccct ggatgtagtc cgcggtggag tcgttcagtt tttgcagttt cttgatttcg 1740
tcgtctttaa ctgccacgtt atcgatgaaa gcaggaccga tctcaccgcg cacttcgtcc 1800
gggatggtag acaggcgacg agtgcgggtg ttcatcagaa cgctcaggga ggtgcaacgc 1860
gtcaggattt cgcccgtttt acagtcacgg accagaaaat cgcgacgcat accattgtta 1920
ccggacgcgc caatccaaca ctcgacttcc acagtgtcac cccaagtagg gtagcgctct 1980
actgctacgt gggtgcgacg cactacccac atcaggtcac gtttagacat ttccagagta 2040
gtgccgaaac catcacccag gataccaaca gatttcgcgt gattcagggt ggcttcctgc 2100
atgtggttca tgacggccag gatggaggta gaacgatctg gacccacttc ataagaacga 2160
attgcgaaag tacgacgaaa caccagaccg tgcagaccga agtgatcatc cagcagttga 2220
ggcagttttg gtttcggttt ccactctaga gtcattttat aatcctcctt gtaaacaaaa 2280
ttatttctag agggaaaccg ttgtggaatt gtgagcgctc acaattccac atccacacat 2340
tatacgagcc gatgattaat tgtcaacagc gtcgatcact gtgcatgaag ctcgtaattg 2400
ttatccgctc acaattggat ccaaaatgaa gggaagttcc tatactttct agagaatagg 2460
aacttctata gggagtcgaa taagggcgac acaaaaggta ttctaaatgc ataataaata 2520
ctgataacat cttatagttt gtattatatt ttgtattatc gttgacatgt ataattttga 2580
tatcaaaaac tgattttccc tttattattt tcgagattta ttttcttaat tctctttaac 2640
aaactagaaa tattgtatat acaaaaaatc ataaataata gatgaatagt ttaattatag 2700
gtgttcatca atcgaaaaag caacgtatct tatttaaagt gcgttgcttt tttctcattt 2760
ataaggttaa ataattctca tatatcaagc aaagtgacag gcgcccttaa atattctgac 2820
aaatgctctt tccctaaact ccccccataa aaaaacccgc cgaagcgggt ttttacgtta 2880
tttgcggatt aacgattact cgttatcaga accgcccagg atgcctggca gttccctact 2940
ctcgccgctg cgctcggtcg ttcggctgcg ggacctcagc gctagcggag tgtatactgg 3000
cttactatgt tggcactgat gagggtgtca gtgaagtgct tcatgtggca ggagaaaaaa 3060
ggctgcaccg gtgcgtcagc agaatatgtg atacaggata tattccgctt cctcgctcac 3120
tgactcgcta cgctcggtcg ttcgactgcg gcgagcggaa atggcttacg aacggggcgg 3180
agatttcctg gaagatgcca ggaagatact taacagggaa gtgagagggc cgcggcaaag 3240

```
ccgtttttcc ataggctccg cccccctgac aagcatcacg aaatctgacg ctcaaatcag      3300
tggtggcgaa acccgacagg actataaaga taccaggcgt ttccccctgg cggctccctc      3360
gtgcgctctc ctgttcctgc ctttcggttt accggtgtca ttccgctgtt atggccgcgt      3420
ttgtctcatt ccacgcctga cactcagttc cgggtaggca gttcgctcca agctggactg      3480
tatgcacgaa ccccccgttc agtccgaccg ctgcgcctta tccggtaact atcgtcttga      3540
gtccaacccg gaaagacatg caaaagcacc actggcagca gccactggta attgatttag      3600
aggagttagt cttgaagtca tgcgccggtt aaggctaaac tgaaaggaca agttttggtg      3660
actgcgctcc tccaagccag ttacctcggt tcaaagagtt ggtagctcag agaaccttcg      3720
aaaaaccgcc ctgcaaggcg gttttttcgt tttcagagca agagattacg cgcagaccaa      3780
aacgatctca agaagatcat cttattaatc actgcccgct ttccagtcgg gaaacctgtc      3840
gtgccagctg cattaatgaa tcggccaacg cgcggggaga ggcggtttgc gtattgggcg      3900
ccagggtggt ttttcttttc accagtgaga ctggcaacag ctgattgccc ttcaccgcct      3960
ggccctgaga gagttgcagc aagcggtcca cgctggtttg ccccagcagg cgaaaatcct      4020
gtttgatggt ggttaacggc gggatataac atgagctatc ttcggtatcg tcgtatccca      4080
ctaccgagat atccgcacca acgcgcagcc cggactcggt aatggcgcgc attgcgccca      4140
gcgccatctg atcgttggca accagcatcg cagtgggaac gatgccctca ttcagcattt      4200
gcatggtttg ttgaaaaccg gacatggcac tccagtcgcc ttcccgttcc gctatcggct      4260
gaatttgatt gcgagtgaga tatttatgcc agccagccag acgcagacgc gccgagacag      4320
aacttaatgg gcccgctaac agcgcgattt gctggtgacc caatgcgacc agatgctcca      4380
cgcccagtcg cgtaccgtcc tcatgggaga aaataatact gttgatgggt gtctggtcag      4440
agacatcaag aaataacgcc ggaacattag tgcaggcagc ttccacagca atggcatcct      4500
ggtcatccag cggatagtta atgatcagcc cactgacgcg ttgcgcgaga agattgtgca      4560
ccgccgcttt acaggcttcg acgccgcttc gttctaccat cgacaccacc acgctggcac      4620
ccagttgatc ggcgcgagat ttaatcgccg cgacaatttg cgacggcgcg tgcagggcca      4680
gactggaggt ggcaacgcca atcagcaacg actgtttgcc cgccagttgt tgtgccacgc      4740
ggttgggaat gtaattcagc tccgccatcg ccgcttccac tttttcccgc gttttcgcag      4800
aaacgtggct ggcctggttc accacgcggg aaacggtctg ataagagaca ccggcatact      4860
ctgcgacatc gtataacgtt actggtttca tattcaccac cctgaattga ctctcttccg      4920
ggcgctatca tgccataccg cgaaaggttt tgcgccattc gatggcgcgc cgcttttt        4977
```

<210> 4
<211> 882
<212> DNA
<213> Homo sapiens

<220>
<221> misc_feature
<223> hGLYAT2 (an amino acid N-acyl transferase) codon optimised for E.coli

<400> 4
atgctggttc tgcacaacag ccaaaaactg caaattctgt acaaatctct ggaaaaatct 60

attcctgaaa gcattaaagt ttatggtgcg atcttcaaca tcaaggataa aaaccctttc 120

aatatggagg tgctggtcga cgcgtggcct gattaccaaa tcgtcatcac ccgtccgcag 180

aagcaagaaa tgaaagatga ccaggaccac tatactaaca cctaccacat cttcactaaa 240

gcgccggaca aactggaaga agttctgagc tactccaatg ttatctcctg ggaacaaacg 300

ctgcagattc aaggttgcca ggaaggtctg gatgaggcga ttcgtaaggt cgctacctct 360

aagtccgttc aagtcgatta catgaaaacc atcctgttta tcccggagct gccgaagaaa 420

cacaaaacct cctctaacga caagatggag ctgttcgaag tagacgatga caacaaagag 480

ggtaactttt ccaatatgtt cctggacgcc tcccacgccg gtctggttaa tgagcactgg 540

gcgttcggta aaaacgaacg ctccctgaag tacattgagc gttgtctgca ggattttctg 600

ggttttggtg tcctgggtcc agagggtcaa ctggtctctt ggatcgttat ggaacagtct 660

tgtgaactgc gtatgggtta tactgtgcca aaataccgtc accaaggtaa tatgctgcaa 720

atcggttatc atctggagaa gtacctgagc cagaaggaaa tcccgttcta tttccatgtt 780

gccgataata acgaaaagag cctgcaagcc ctgaacaatc tgggcttcaa gatctgccct 840

tgcggttggc accagtggaa gtgcacgcct aaaaagtact gt 882

<210> 5
<211> 864
<212> DNA
<213> Homo sapiens

<220>
<221> misc_feature
<223> hGLYAT3 (another amino acid N-acyl transferase) (codon optimised for E.coli)

<400> 5
atgctggttc tgaattgctc tactaaactg ctgatcctgg agaaaatgct gaaatcctgt 60

ttcccggaat ctctgaaagt ttatggtgcg gtaatgaata tcaaccgtgg taatccgttt 120

caaaaagaag ttgttctgga ctcttggcca gatttcaagg cggttatcac ccgtcgtcag 180

cgtgaagcgg aaaccgacaa cctggaccac tataccaacg cgtatgcggt tttctataaa 240

gatgttcgcg cgtaccgtca gctgctggaa gaatgcgatg ttttcaactg ggaccaggtt 300

tttcagatcc aaggtctgca atccgagctg tacgacgttt ctaaggccgt ggcgaactct 360

aaacaactga acatcaagct gacctctttc aaagcggttc atttctctcc ggtatccagc 420

ctgccggaca cctccttcct gaaaggtccg tctccgcgtc tgacctacct gtctgttgcg 480

aatgcggatc tgctgaaccg tacttggtcc cgtggtggca acgaacagtg cctgcgttac 540

attgctaacc tgatctcttg cttcccgagc gtctgcgttc gtgatgaaaa aggtaaccca 600

gtatcttggt ctatcaccga ccagttcgcg accatgtgtc atggctacac cctgcctgaa 660

caccgtcgta agggttactc tcgtctggtt gcgctgaccc tggcgcgtaa gctgcagtct 720

cgtggttttc cgtcccaggg taacgtcctg gacgacaaca ctgcgtccat ctccctgctg 780

aagtccctgc atgccgaatt cctgccgtgc cgtttccacc gtctgattct gaccccagcg 840

accttctctg gtctgccgca cctg 864

<210> 6
<211> 1686
<212> DNA
<213> Escherichia coli

<220>
<221> misc_feature
<223> fadD (an acyl-CoA synthetase)

<400> 6
ttgaagaagg tttggcttaa ccgttatccc gcggacgttc cgacggagat caaccctgac 60

cgttatcaat ctctggtaga tatgtttgag cagtcggtcg cgcgctacgc cgatcaacct 120

gcgtttgtga atatggggga ggtaatgacc ttccgcaagc tggaagaacg cagtcgcgcg 180

tttgccgctt atttgcaaca agggttgggg ctgaagaaag gcgatcgcgt tgcgttgatg 240

atgcctaatt tattgcaata tccggtggcg ctgtttggca ttttgcgtgc cgggatgatc 300

gtcgtaaacg ttaacccgtt gtataccccg cgtgagcttg agcatcagct taacgatagc 360

ggcgcatcgg cgattgttat cgtgtctaac tttgctcaca cactggaaaa agtggttgat 420

aaaaccgccg ttcagcacgt aattctgacc cgtatgggcg atcagctatc tacggcaaaa 480

ggcacggtag tcaatttcgt tgttaaatac atcaagcgtt tggtgccgaa ataccatctg 540

ccagatgcca tttcatttcg tagcgcactg cataacggct accggatgca gtacgtcaaa 600

cccgaactgg tgccggaaga tttagctttt ctgcaataca ccggcggcac cactggtgtg 660

gcgaaaggcg cgatgctgac tcaccgcaat atgctggcga acctggaaca ggttaacgcg 720

acctatggtc cgctgttgca tccgggcaaa gagctggtgg tgacggcgct gccgctgtat 780

cacattttttg ccctgaccat taactgcctg ctgtttatcg aactgggtgg gcagaacctg 840

cttatcacta acccgcgcga tattccaggg ttggtaaaag agttagcgaa atatccgttt 900

accgctatca cgggcgttaa caccttgttc aatgcgttgc tgaacaataa agagttccag 960

cagctggatt tctccagtct gcatctttcc gcaggcggtg ggatgccagt gcagcaagtg 1020

gtggcagagc gttgggtgaa actgaccgga cagtatctgc tggaaggcta tggccttacc 1080

gagtgtgcgc cgctggtcag cgttaaccca tatgatattg attatcatag tggtagcatc 1140

ggtttgccgg tgccgtcgac ggaagccaaa ctggtggatg atgatgataa tgaagtacca 1200

ccaggtcaac cgggtgagct ttgtgtcaaa ggaccgcagg tgatgctggg ttactggcag 1260

cgtcccgatg ctaccgatga aatcatcaaa aatggctggt tacacaccgg cgacatcgcg 1320

gtaatggatg aagaaggatt cctgcgcatt gtcgatcgta aaaaagacat gattctggtt 1380

tccggtttta acgtctatcc caacgagatt gaagatgtcg tcatgcagca tcctggcgta 1440

caggaagtcg cggctgttgg cgtaccttcc ggctccagtg gtgaagcggt gaaaatcttc 1500

gtagtgaaaa aagatccatc gcttaccgaa gagtcactgg tgactttttg ccgccgtcag 1560

ctcacgggat acaaagtacc gaagctggtg gagtttcgtg atgagttacc gaaatctaac 1620

gtcggaaaaa ttttgcgacg agaattacgt gacgaagcgc gcggcaaagt ggacaataaa 1680

gcctga 1686

<210> 7
<211> 7138
<212> DNA
<213> Artificial Sequence

<220>
<223> Vector pCDF[atfA1_Ab(co_Ec)-fadD_Ec]

<220>
<221> misc_feature
<223> Vector pCDF[atfA1_Ab(co_Ec)-fadD_Ec]

<400> 7
cgggatctcg acgctctccc ttatgcgact cctgcgttta gggaaagagc atttgtcaga 60

atatttaagg gcgcctgtca ctttgcttga tatatgagaa ttatttaacc ttataaatga 120

gaaaaaagca acgcacttta aataagatac gttgcttttt cgattgatga acacctataa 180

ttaaactatt catctattat ttatgatttt ttgtatatac aatatttcta gtttgttaaa 240

gagaattaag aaaataaatc tcgaaaataa taaagggaaa atcagttttt gatatcaaaa 300

ttatacatgt caacgataat acaaaatata atacaaacta taagatgtta tcagtattta 360

ttatgcattt agaatacctt ttgtgtcgcc cttattcgac tccctataga agttcctatt 420

ctctagaaag tataggaact tcccttcatt ttggatccaa ttgtgagcgg ataacaatta 480

cgagcttcat gcacagtgat cgacgctgtt gacaattaat catcggctcg tataatgtgt 540

ggatgtggaa ttgtgagcgc tcacaattcc acaacggttt ccctctagaa ataattttgt 600

ttaacaggag gtaaaacata tgaaagcgct gtccccggtg gatcagctgt tcctgtggct 660

ggagaaacgt cagcaaccga tgcatgttgg tggtctgcag ctgtttagct tcccagaggg 720

tgcgggtcct aagtacgtta gcgagttggc tcagcaaatg cgtgactact gtcacccggt 780
ggccccgttc aatcagcgcc tgacccgtcg cctgggtcaa tactattgga cgcgcgacaa 840
acaatttgac attgaccacc attttcgcca tgaagcgctg ccgaagccgg gtcgcattcg 900
cgaactgctg tccttggtta gcgcggagca cagcaatctg ttggaccgcg agcgccctat 960
gtgggaagcg cacctgattg agggcatccg tggccgccaa ttcgctttgt actataagat 1020
ccaccatagc gtgatggatg gcatcagcgc tatgcgcatt gcgtctaaaa cgctgagcac 1080
cgacccgtct gagcgcgaaa tggcaccagc atgggccttc aataccaaga aacgtagccg 1140
tagcctgcct tccaatccag ttgatatggc gagcagcatg gcccgtctga cggcttctat 1200
cagcaaacag gcggcgaccg ttccgggtct ggctcgtgaa gtgtacaagg tcacccagaa 1260
agctaagaag gatgagaact acgtgagcat ttttcaagcg ccggatacca tcttgaacaa 1320
caccattacc ggttcccgtc gttttgcagc acagagcttt ccgctgccgc gtctgaaagt 1380
tatcgcgaag gcgtataact gcaccatcaa caccgtcgtc ctgtctatgt gtggccacgc 1440
gctgcgtgaa tatctgatta gccaacacgc actgccggat gagccgctga tcgcgatggt 1500
gccgatgagc ctgcgtcagg acgacagcac gggtggtaat cagattggca tgatcctggc 1560
caacctgggc acccatatct gcgatccggc gaatcgtttg cgtgttattc acgactccgt 1620
cgaggaagcg aaatcgcgtt tcagccagat gagcccggag gagatcctga actttaccgc 1680
actgactatg gccccgacgg gcttgaatct gctgaccggc ctggcaccga aatggcgtgc 1740
gttcaacgtc gtcattagca acattccggg tccgaaggaa ccgctgtatt ggaatggcgc 1800
ccagctgcaa ggtgtgtacc cggtaagcat tgcgttggac cgtattgcac tgaacatcac 1860
gctgactagc tacgtggatc agatggagtt cggtctgatc gcatgccgtc gcacgctgcc 1920
gagcatgcaa cgtctgctgg attatctgga acaaagcatt cgtgagttgg aaatcggtgc 1980
cggcatcaag taagcgttaa gtctgagagg tgaagaattg aagaaggttt ggcttaaccg 2040
ttatcccgcg gacgttccga cggagatcaa ccctgaccgt tatcaatctc tggtagatat 2100
gtttgagcag tcggtcgcgc gctacgccga tcaacctgcg tttgtgaata tgggggaggt 2160
aatgaccttc cgcaagctgg aagaacgcag tcgcgcgttt gccgcttatt tgcaacaagg 2220
gttggggctg aagaaaggcg atcgcgttgc gttgatgatg cctaatttat tgcaatatcc 2280
ggtggcgctg tttggcattt tgcgtgccgg gatgatcgtc gtaaacgtta acccgttgta 2340
taccccgcgt gagcttgagc atcagcttaa cgatagcggc gcatcggcga ttgttatcgt 2400
gtctaacttt gctcacacac tggaaaaagt ggttgataaa accgccgttc agcacgtaat 2460
tctgacccgt atgggcgatc agctatctac ggcaaaaggc acggtagtca atttcgttgt 2520
taaatacatc aagcgtttgg tgccgaaata ccatctgcca gatgccattt catttcgtag 2580

cgcactgcat aacggctacc ggatgcagta cgtcaaaccc gaactggtgc cggaagattt 2640
agcttttctg caatacaccg gcggcaccac tggtgtggcg aaaggcgcga tgctgactca 2700
ccgcaatatg ctggcgaacc tggaacaggt taacgcgacc tatggtccgc tgttgcatcc 2760
gggcaaagag ctggtggtga cggcgctgcc gctgtatcac atttttgccc tgaccattaa 2820
ctgcctgctg tttatcgaac tgggtgggca gaacctgctt atcactaacc cgcgcgatat 2880
tccagggttg gtaaaagagt tagcgaaata tccgtttacc gctatcacgg gcgttaacac 2940
cttgttcaat gcgttgctga acaataaaga gttccagcag ctggatttct ccagtctgca 3000
tctttccgca ggcggtggga tgccagtgca gcaagtggtg gcagagcgtt gggtgaaact 3060
gaccggacag tatctgctgg aaggctatgg ccttaccgag tgtgcgccgc tggtcagcgt 3120
taacccatat gatattgatt atcatagtgg tagcatcggt ttgccggtgc cgtcgacgga 3180
agccaaactg gtggatgatg atgataatga agtaccacca ggtcaaccgg gtgagctttg 3240
tgtcaaagga ccgcaggtga tgctgggtta ctggcagcgt cccgatgcta ccgatgaaat 3300
catcaaaaat ggctggttac acaccggcga catcgcggta atggatgaag aaggattcct 3360
gcgcattgtc gatcgtaaaa aagacatgat tctggtttcc ggttttaacg tctatcccaa 3420
cgagattgaa gatgtcgtca tgcagcatcc tggcgtacag gaagtcgcgg ctgttggcgt 3480
accttccggc tccagtggtg aagcggtgaa aatcttcgta gtgaaaaaag atccatcgct 3540
taccgaagag tcactggtga ctttttgccg ccgtcagctc acgggataca aagtaccgaa 3600
gctggtggag tttcgtgatg agttaccgaa atctaacgtc ggaaaaattt tgcgacgaga 3660
attacgtgac gaagcgcgcg gcaaagtgga caataaagcc tgagcgttaa gtcagtcgtc 3720
agacgccggt taatccggcg tttttttga cgcccactaa agagaaaaca atctcgagtc 3780
tggtaaagaa accgctgctg cgaaatttga acgccagcac atggactcgt ctactagcgc 3840
agcttaatta acctaggctg ctgccaccgc tgagcaataa ctagcataac cccttggggc 3900
ctctaaacgg gtcttgaggg gttttttgct gaaacctcag gcatttgaga agcacacggt 3960
cacactgctt ccggtagtca ataaaccggt aaaccagcaa tagacataag cggctattta 4020
acgaccctgc cctgaaccga cgaccgggtc atcgtggccg gatcttgcgg cccctcggct 4080
tgaacgaatt gttagacatt atttgccgac taccttggtg atctcgcctt tcacgtagtg 4140
gacaaattct tccaactgat ctgcgcgcga ggccaagcga tcttcttctt gtccaagata 4200
agcctgtcta gcttcaagta tgacgggctg atactgggcc ggcaggcgct ccattgccca 4260
gtcggcagcg acatccttcg gcgcgatttt gccggttact gcgctgtacc aaatgcggga 4320
caacgtaagc actacatttc gctcatcgcc agcccagtcg ggcggcgagt tccatagcgt 4380
taaggtttca tttagcgcct caaatagatc ctgttcagga accggatcaa agagttcctc 4440
cgccgctgga cctaccaagg caacgctatg ttctcttgct tttgtcagca agatagccag 4500

atcaatgtcg atcgtggctg gctcgaagat acctgcaaga atgtcattgc gctgccattc 4560
tccaaattgc agttcgcgct tagctggata acgccacgga atgatgtcgt cgtgcacaac 4620
aatggtgact tctacagcgc ggagaatctc gctctctcca ggggaagccg aagtttccaa 4680
aaggtcgttg atcaaagctc gccgcgttgt ttcatcaagc cttacggtca ccgtaaccag 4740
caaatcaata tcactgtgtg gcttcaggcc gccatccact gcggagccgt acaaatgtac 4800
ggccagcaac gtcggttcga gatggcgctc gatgacgcca actacctctg atagttgagt 4860
cgatacttcg gcgatcaccg cttccctcat actcttcctt tttcaatatt attgaagcat 4920
ttatcagggt tattgtctca tgagcggata catatttgaa tgtatttaga aaaataaaca 4980
aatagctagc tcactcggtc gctacgctcc gggcgtgaga ctgcggcggg cgctgcggac 5040
acatacaaag ttacccacag attccgtgga taagcagggg actaacatgt gaggcaaaac 5100
agcagggccg cgccggtggc gtttttccat aggctccgcc ctcctgccag agttcacata 5160
aacagacgct tttccggtgc atctgtggga gccgtgaggc tcaaccatga atctgacagt 5220
acgggcgaaa cccgacagga cttaaagatc cccaccgttt ccggcgggtc gctccctctt 5280
gcgctctcct gttccgaccc tgccgtttac cggatacctg ttccgccttt ctcccttacg 5340
ggaagtgtgg cgctttctca tagctcacac actggtatct cggctcggtg taggtcgttc 5400
gctccaagct gggctgtaag caagaactcc ccgttcagcc cgactgctgc gccttatccg 5460
gtaactgttc acttgagtcc aacccggaaa agcacggtaa aacgccactg gcagcagcca 5520
ttggtaactg ggagttcgca gaggatttgt ttagctaaac acgcggttgc tcttgaagtg 5580
tgcgccaaag tccggctaca ctggaaggac agatttggtt gctgtgctct gcgaaagcca 5640
gttaccacgg ttaagcagtt ccccaactga cttaaccttc gatcaaacca cctccccagg 5700
tggtttttc gtttacaggg caaaagatta cgcgcagaaa aaaaggatct caagaagatc 5760
ctttgatctt ttctactgaa ccgctctaga tttcagtgca atttatctct tcaaatgtag 5820
cacctgaagt cagccccata cgatataagt tgtaattctc atgttagtca tgccccgcgc 5880
ccaccggaag gagctgactg ggttgaaggc tctcaagggc atcggtcgag atcccggtgc 5940
ctaatgagtg agctaactta cattaattgc gttgcgctca ctgcccgctt tccagtcggg 6000
aaacctgtcg tgccagctgc attaatgaat cggccaacgc gcggggagag gcggtttgcg 6060
tattgggcgc cagggtggtt tttcttttca ccagtgagac gggcaacagc tgattgccct 6120
tcaccgcctg gccctgagag agttgcagca agcggtccac gctggtttgc cccagcaggc 6180
gaaaatcctg tttgatggtg gttaacggcg ggatataaca tgagctgtct tcggtatcgt 6240
cgtatcccac taccgagatg tccgcaccaa cgcgcagccc ggactcggta atggcgcgca 6300
ttgcgcccag cgccatctga tcgttggcaa ccagcatcgc agtgggaacg atgccctcat 6360

tcagcatttg catggtttgt tgaaaaccgg acatggcact ccagtcgcct tcccgttccg 6420

ctatcggctg aatttgattg cgagtgagat atttatgcca gccagccaga cgcagacgcg 6480

ccgagacaga acttaatggg cccgctaaca gcgcgatttg ctggtgaccc aatgcgacca 6540

gatgctccac gcccagtcgc gtaccgtctt catgggagaa aataatactg ttgatgggtg 6600

tctggtcaga gacatcaaga aataacgccg gaacattagt gcaggcagct tccacagcaa 6660

tggcatcctg gtcatccagc ggatagttaa tgatcagccc actgacgcgt tgcgcgagaa 6720

gattgtgcac cgccgcttta caggcttcga cgccgcttcg ttctaccatc gacaccacca 6780

cgctggcacc cagttgatcg gcgcgagatt taatcgccgc gacaatttgc gacggcgcgt 6840

gcagggccag actggaggtg gcaacgccaa tcagcaacga ctgtttgccc gccagttgtt 6900

gtgccacgcg gttgggaatg taattcagct ccgccatcgc cgcttccact ttttcccgcg 6960

ttttcgcaga aacgtggctg gcctggttca ccacgcggga aacggtctga taagagacac 7020

cggcatactc tgcgacatcg tataacgtta ctggtttcac attcaccacc ctgaattgac 7080

tctcttccgg gcgctatcat gccataccgc gaaaggtttt gcgccattcg atggtgtc 7138

<210> 8
<211> 6649
<212> DNA
<213> Artificial Sequence

<220>
<223> Vector pCDF{Ptac}[hGLYAT2(co_Ec)-fadD_Ec]

<220>
<221> misc_feature
<223> Vector pCDF{Ptac}[hGLYAT2(co_Ec)-fadD_Ec]

<400> 8
cgggatctcg acgctctccc ttatgcgact cctgcgttta gggaaagagc atttgtcaga 60

atatttaagg gcgcctgtca ctttgcttga tatatgagaa ttatttaacc ttataaatga 120

gaaaaaagca acgcacttta aataagatac gttgcttttt cgattgatga acacctataa 180

ttaaactatt catctattat ttatgatttt ttgtatatac aatatttcta gtttgttaaa 240

gagaattaag aaaataaatc tcgaaaataa taaagggaaa atcagttttt gatatcaaaa 300

ttatacatgt caacgataat acaaaatata atacaaacta taagatgtta tcagtattta 360

ttatgcattt agaatacctt ttgtgtcgcc cttattcgac tccctataga agttcctatt 420

ctctagaaag tataggaact tcccttcatt ttggatccaa ttgtgagcgg ataacaatta 480

cgagcttcat gcacagtgat cgacgctgtt gacaattaat catcggctcg tataatgtgt 540

ggatgtggaa ttgtgagcgc tcacaattcc acaacggttt ccctctagaa ataattttgt 600

ttaacaggag gtaaaacata tgctggttct gcacaacagc caaaaactgc aaattctgta 660

caaatctctg gaaaaatcta ttcctgaaag cattaaagtt tatggtgcga tcttcaacat 720
caaggataaa aaccctttca atatggaggt gctggtcgac gcgtggcctg attaccaaat 780
cgtcatcacc cgtccgcaga agcaagaaat gaaagatgac caggaccact atactaacac 840
ctaccacatc ttcactaaag cgccggacaa actggaagaa gttctgagct actccaatgt 900
tatctcctgg gaacaaacgc tgcagattca aggttgccag gaaggtctgg atgaggcgat 960
tcgtaaggtc gctacctcta agtccgttca agtcgattac atgaaaacca tcctgtttat 1020
cccggagctg ccgaagaaac acaaaacctc ctctaacgac aagatggagc tgttcgaagt 1080
agacgatgac aacaaagagg gtaacttttc caatatgttc ctggacgcct cccacgccgg 1140
tctggttaat gagcactggg cgttcggtaa aaacgaacgc tccctgaagt acattgagcg 1200
ttgtctgcag gattttctgg gttttggtgt cctgggtcca gagggtcaac tggtctcttg 1260
gatcgttatg gaacagtctt gtgaactgcg tatgggttat actgtgccaa aataccgtca 1320
ccaaggtaat atgctgcaaa tcggttatca tctggagaag tacctgagcc agaaggaaat 1380
cccgttctat ttccatgttg ccgataataa cgaaaagagc ctgcaagccc tgaacaatct 1440
gggcttcaag atctgccctt gcggttggca ccagtggaag tgcacgccta aaaagtactg 1500
ttaagcgtta agtctgagag gtgaagaatt gaagaaggtt tggcttaacc gttatcccgc 1560
ggacgttccg acggagatca accctgaccg ttatcaatct ctggtagata tgtttgagca 1620
gtcggtcgcg cgctacgccg atcaacctgc gtttgtgaat atgggggagg taatgacctt 1680
ccgcaagctg gaagaacgca gtcgcgcgtt tgccgcttat ttgcaacaag ggttggggct 1740
gaagaaaggc gatcgcgttg cgttgatgat gcctaattta ttgcaatatc cggtggcgct 1800
gtttggcatt ttgcgtgccg ggatgatcgt cgtaaacgtt aacccgttgt ataccccgcg 1860
tgagcttgag catcagctta acgatagcgg cgcatcggcg attgttatcg tgtctaactt 1920
tgctcacaca ctggaaaaag tggttgataa aaccgccgtt cagcacgtaa ttctgacccg 1980
tatgggcgat cagctatcta cggcaaaagg cacggtagtc aatttcgttg ttaaatacat 2040
caagcgtttg gtgccgaaat accatctgcc agatgccatt tcatttcgta gcgcactgca 2100
taacggctac cggatgcagt acgtcaaacc cgaactggtg ccggaagatt tagcttttct 2160
gcaatacacc ggcggcacca ctggtgtggc gaaaggcgcg atgctgactc accgcaatat 2220
gctggcgaac ctggaacagg ttaacgcgac ctatggtccg ctgttgcatc cgggcaaaga 2280
gctggtggtg acggcgctgc cgctgtatca catttttgcc ctgaccatta actgcctgct 2340
gtttatcgaa ctgggtgggc agaacctgct tatcactaac ccgcgcgata ttccagggtt 2400
ggtaaaagag ttagcgaaat atccgtttac cgctatcacg ggcgttaaca ccttgttcaa 2460
tgcgttgctg aacaataaag agttccagca gctggatttc tccagtctgc atctttccgc 2520
aggcggtggg atgccagtgc agcaagtggt ggcagagcgt tgggtgaaac tgaccggaca 2580

gtatctgctg gaaggctatg gccttaccga gtgtgcgccg ctggtcagcg ttaacccata 2640

tgatattgat tatcatagtg gtagcatcgg tttgccggtg ccgtcgacgg aagccaaact 2700

ggtggatgat gatgataatg aagtaccacc aggtcaaccg ggtgagcttt gtgtcaaagg 2760

accgcaggtg atgctgggtt actggcagcg tcccgatgct accgatgaaa tcatcaaaaa 2820

tggctggtta cacaccggcg acatcgcggt aatggatgaa gaaggattcc tgcgcattgt 2880

cgatcgtaaa aaagacatga ttctggtttc cggttttaac gtctatccca acgagattga 2940

agatgtcgtc atgcagcatc ctggcgtaca ggaagtcgcg gctgttggcg taccttccgg 3000

ctccagtggt gaagcggtga aaatcttcgt agtgaaaaaa gatccatcgc ttaccgaaga 3060

gtcactggtg actttttgcc gccgtcagct cacgggatac aaagtaccga agctggtgga 3120

gtttcgtgat gagttaccga aatctaacgt cggaaaaatt ttgcgacgag aattacgtga 3180

cgaagcgcgc ggcaaagtgg acaataaagc ctgagcgtta agtcagtcgt cagacgccgg 3240

ttaatccggc gtttttttg acgcccacta aagagaaaac aatctcgagt ctggtaaaga 3300

aaccgctgct gcgaaatttg aacgccagca catggactcg tctactagcg cagcttaatt 3360

aacctaggct gctgccaccg ctgagcaata actagcataa ccccttgggg cctctaaacg 3420

ggtcttgagg ggttttttgc tgaaacctca ggcatttgag aagcacacgg tcacactgct 3480

tccggtagtc aataaaccgg taaaccagca atagacataa gcggctattt aacgaccctg 3540

ccctgaaccg acgaccgggt catcgtggcc ggatcttgcg gcccctcggc ttgaacgaat 3600

tgttagacat tatttgccga ctaccttggt gatctcgcct ttcacgtagt ggacaaattc 3660

ttccaactga tctgcgcgcg aggccaagcg atcttcttct tgtccaagat aagcctgtct 3720

agcttcaagt atgacgggct gatactgggc cggcaggcgc tccattgccc agtcggcagc 3780

gacatccttc ggcgcgattt tgccggttac tgcgctgtac caaatgcggg acaacgtaag 3840

cactacattt cgctcatcgc cagcccagtc gggcggcgag ttccatagcg ttaaggtttc 3900

atttagcgcc tcaaatagat cctgttcagg aaccggatca aagagttcct ccgccgctgg 3960

acctaccaag gcaacgctat gttctcttgc ttttgtcagc aagatagcca gatcaatgtc 4020

gatcgtggct ggctcgaaga tacctgcaag aatgtcattg cgctgccatt ctccaaattg 4080

cagttcgcgc ttagctggat aacgccacgg aatgatgtcg tcgtgcacaa caatggtgac 4140

ttctacagcg cggagaatct cgctctctcc aggggaagcc gaagtttcca aaaggtcgtt 4200

gatcaaagct cgccgcgttg tttcatcaag ccttacggtc accgtaacca gcaaatcaat 4260

atcactgtgt ggcttcaggc cgccatccac tgcggagccg tacaaatgta cggccagcaa 4320

cgtcggttcg agatggcgct cgatgacgcc aactacctct gatagttgag tcgatacttc 4380

ggcgatcacc gcttccctca tactcttcct tttcaatat tattgaagca tttatcaggg 4440

ttattgtctc atgagcggat acatatttga atgtatttag aaaaataaac aaatagctag 4500
ctcactcggt cgctacgctc cgggcgtgag actgcggcgg gcgctgcgga cacatacaaa 4560
gttacccaca gattccgtgg ataagcaggg gactaacatg tgaggcaaaa cagcagggcc 4620
gcgccggtgg cgtttttcca taggctccgc cctcctgcca gagttcacat aaacagacgc 4680
ttttccggtg catctgtggg agccgtgagg ctcaaccatg aatctgacag tacgggcgaa 4740
acccgacagg acttaaagat ccccaccgtt tccggcgggt cgctccctct tgcgctctcc 4800
tgttccgacc ctgccgttta ccggatacct gttccgcctt tctcccttac gggaagtgtg 4860
gcgctttctc atagctcaca cactggtatc tcggctcggt gtaggtcgtt cgctccaagc 4920
tgggctgtaa gcaagaactc cccgttcagc ccgactgctg cgccttatcc ggtaactgtt 4980
cacttgagtc caacccggaa aagcacggta aaacgccact ggcagcagcc attggtaact 5040
gggagttcgc agaggatttg tttagctaaa cacgcggttg ctcttgaagt gtgcgccaaa 5100
gtccggctac actggaagga cagatttggt tgctgtgctc tgcgaaagcc agttaccacg 5160
gttaagcagt tccccaactg acttaacctt cgatcaaacc acctccccag gtggtttttt 5220
cgtttacagg gcaaaagatt acgcgcagaa aaaaaggatc tcaagaagat cctttgatct 5280
tttctactga accgctctag atttcagtgc aatttatctc ttcaaatgta gcacctgaag 5340
tcagccccat acgatataag ttgtaattct catgttagtc atgccccgcg cccaccggaa 5400
ggagctgact gggttgaagg ctctcaaggg catcggtcga gatcccggtg cctaatgagt 5460
gagctaactt acattaattg cgttgcgctc actgcccgct ttccagtcgg gaaacctgtc 5520
gtgccagctg cattaatgaa tcggccaacg cgcggggaga ggcggtttgc gtattgggcg 5580
ccagggtggt ttttcttttc accagtgaga cgggcaacag ctgattgccc ttcaccgcct 5640
ggccctgaga gagttgcagc aagcggtcca cgctggtttg ccccagcagg cgaaaatcct 5700
gtttgatggt ggttaacggc gggatataac atgagctgtc ttcggtatcg tcgtatccca 5760
ctaccgagat gtccgcacca acgcgcagcc cggactcggt aatggcgcgc attgcgccca 5820
gcgccatctg atcgttggca accagcatcg cagtgggaac gatgccctca ttcagcattt 5880
gcatggtttg ttgaaaaccg gacatggcac tccagtcgcc ttcccgttcc gctatcggct 5940
gaatttgatt gcgagtgaga tatttatgcc agccagccag acgcagacgc gccgagacag 6000
aacttaatgg gcccgctaac agcgcgattt gctggtgacc caatgcgacc agatgctcca 6060
cgcccagtcg cgtaccgtct tcatgggaga aaataatact gttgatgggt gtctggtcag 6120
agacatcaag aaataacgcc ggaacattag tgcaggcagc ttccacagca atggcatcct 6180
ggtcatccag cggatagtta atgatcagcc cactgacgcg ttgcgcgaga agattgtgca 6240
ccgccgcttt acaggcttcg acgccgcttc gttctaccat cgacaccacc acgctggcac 6300
ccagttgatc ggcgcgagat ttaatcgccg cgacaatttg cgacggcgcg tgcagggcca 6360

gactggaggt ggcaacgcca atcagcaacg actgtttgcc cgccagttgt tgtgccacgc 6420

ggttgggaat gtaattcagc tccgccatcg ccgcttccac tttttcccgc gttttcgcag 6480

aaacgtggct ggcctggttc accacgcggg aaacggtctg ataagagaca ccggcatact 6540

ctgcgacatc gtataacgtt actggtttca cattcaccac cctgaattga ctctcttccg 6600

ggcgctatca tgccataccg cgaaaggttt tgcgccattc gatggtgtc 6649

<210> 9
<211> 6631
<212> DNA
<213> Artificial Sequence

<220>
<223> Vector pCDF{Ptac}[hGLYAT3(co_Ec)-fadD_Ec]

<220>
<221> misc_feature
<223> Vector pCDF{Ptac}[hGLYAT3(co_Ec)-fadD_Ec]

<400> 9
cgggatctcg acgctctccc ttatgcgact cctgcgttta gggaaagagc atttgtcaga 60

atatttaagg gcgcctgtca ctttgcttga tatatgagaa ttatttaacc ttataaatga 120

gaaaaaagca acgcacttta aataagatac gttgcttttt cgattgatga acacctataa 180

ttaaactatt catctattat ttatgatttt ttgtatatac aatatttcta gtttgttaaa 240

gagaattaag aaaataaatc tcgaaaataa taaagggaaa atcagttttt gatatcaaaa 300

ttatacatgt caacgataat acaaaatata atacaaacta taagatgtta tcagtattta 360

ttatgcattt agaatacctt ttgtgtcgcc cttattcgac tccctataga agttcctatt 420

ctctagaaag tataggaact tcccttcatt ttggatccaa ttgtgagcgg ataacaatta 480

cgagcttcat gcacagtgat cgacgctgtt gacaattaat catcggctcg tataatgtgt 540

ggatgtggaa ttgtgagcgc tcacaattcc acaacggttt ccctctagaa ataattttgt 600

ttaacaggag gtaaaacata tgctggttct gaattgctct actaaactgc tgatcctgga 660

gaaaatgctg aaatcctgtt tcccggaatc tctgaaagtt tatggtgcgg taatgaatat 720

caaccgtggt aatccgtttc aaaaagaagt tgttctggac tcttggccag atttcaaggc 780

ggttatcacc cgtcgtcagc gtgaagcgga aaccgacaac ctggaccact ataccaacgc 840

gtatgcggtt ttctataaag atgttcgcgc gtaccgtcag ctgctggaag aatgcgatgt 900

tttcaactgg gaccaggttt ttcagatcca aggtctgcaa tccgagctgt acgacgtttc 960

taaggccgtg gcgaactcta aacaactgaa catcaagctg acctctttca aagcggttca 1020

tttctctccg gtatccagcc tgccggacac ctccttcctg aaaggtccgt ctccgcgtct 1080

gacctacctg tctgttgcga atgcggatct gctgaaccgt acttggtccc gtggtggcaa 1140

cgaacagtgc ctgcgttaca ttgctaacct gatctcttgc ttcccgagcg tctgcgttcg 1200

tgatgaaaaa ggtaacccag tatcttggtc tatcaccgac cagttcgcga ccatgtgtca 1260

tggctacacc ctgcctgaac accgtcgtaa gggttactct cgtctggttg cgctgaccct 1320

ggcgcgtaag ctgcagtctc gtggttttcc gtcccagggt aacgtcctgg acgacaacac 1380

tgcgtccatc tccctgctga agtccctgca tgccgaattc ctgccgtgcc gtttccaccg 1440

tctgattctg accccagcga ccttctctgg tctgccgcac ctgtaagcgt taagtctgag 1500

aggtgaagaa ttgaagaagg tttggcttaa ccgttatccc gcggacgttc cgacggagat 1560

caaccctgac cgttatcaat ctctggtaga tatgtttgag cagtcggtcg cgcgctacgc 1620

cgatcaacct gcgtttgtga atatggggga ggtaatgacc ttccgcaagc tggaagaacg 1680

cagtcgcgcg tttgccgctt atttgcaaca agggttgggg ctgaagaaag gcgatcgcgt 1740

tgcgttgatg atgcctaatt tattgcaata tccggtggcg ctgtttggca ttttgcgtgc 1800

cgggatgatc gtcgtaaacg ttaacccgtt gtataccccg cgtgagcttg agcatcagct 1860

taacgatagc ggcgcatcgg cgattgttat cgtgtctaac tttgctcaca cactggaaaa 1920

agtggttgat aaaaccgccg ttcagcacgt aattctgacc cgtatgggcg atcagctatc 1980

tacggcaaaa ggcacggtag tcaatttcgt tgttaaatac atcaagcgtt tggtgccgaa 2040

ataccatctg ccagatgcca tttcatttcg tagcgcactg cataacggct accggatgca 2100

gtacgtcaaa cccgaactgg tgccggaaga tttagctttt ctgcaataca ccggcggcac 2160

cactggtgtg gcgaaaggcg cgatgctgac tcaccgcaat atgctggcga acctggaaca 2220

ggttaacgcg acctatggtc cgctgttgca tccgggcaaa gagctggtgg tgacggcgct 2280

gccgctgtat cacatttttg ccctgaccat taactgcctg ctgtttatcg aactgggtgg 2340

gcagaacctg cttatcacta acccgcgcga tattccaggg ttggtaaaag agttagcgaa 2400

atatccgttt accgctatca cgggcgttaa caccttgttc aatgcgttgc tgaacaataa 2460

agagttccag cagctggatt tctccagtct gcatctttcc gcaggcggtg ggatgccagt 2520

gcagcaagtg gtggcagagc gttgggtgaa actgaccgga cagtatctgc tggaaggcta 2580

tggccttacc gagtgtgcgc cgctggtcag cgttaaccca tatgatattg attatcatag 2640

tggtagcatc ggtttgccgg tgccgtcgac ggaagccaaa ctggtggatg atgatgataa 2700

tgaagtacca ccaggtcaac cgggtgagct ttgtgtcaaa ggaccgcagg tgatgctggg 2760

ttactggcag cgtcccgatg ctaccgatga aatcatcaaa aatggctggt tacacaccgg 2820

cgacatcgcg gtaatggatg aagaaggatt cctgcgcatt gtcgatcgta aaaaagacat 2880

gattctggtt tccggtttta acgtctatcc caacgagatt gaagatgtcg tcatgcagca 2940

tcctggcgta caggaagtcg cggctgttgg cgtaccttcc ggctccagtg gtgaagcggt 3000

gaaaatcttc gtagtgaaaa aagatccatc gcttaccgaa gagtcactgg tgactttttg 3060
ccgccgtcag ctcacgggat acaaagtacc gaagctggtg gagtttcgtg atgagttacc 3120
gaaatctaac gtcggaaaaa ttttgcgacg agaattacgt gacgaagcgc gcggcaaagt 3180
ggacaataaa gcctgagcgt taagtcagtc gtcagacgcc ggttaatccg gcgttttttt 3240
tgacgcccac taaagagaaa acaatctcga gtctggtaaa gaaaccgctg ctgcgaaatt 3300
tgaacgccag cacatggact cgtctactag cgcagcttaa ttaacctagg ctgctgccac 3360
cgctgagcaa taactagcat aaccccttgg ggcctctaaa cgggtcttga ggggtttttt 3420
gctgaaacct caggcatttg agaagcacac ggtcacactg cttccggtag tcaataaacc 3480
ggtaaaccag caatagacat aagcggctat ttaacgaccc tgccctgaac cgacgaccgg 3540
gtcatcgtgg ccggatcttg cggcccctcg gcttgaacga attgttagac attatttgcc 3600
gactaccttg gtgatctcgc ctttcacgta gtggacaaat tcttccaact gatctgcgcg 3660
cgaggccaag cgatcttctt cttgtccaag ataagcctgt ctagcttcaa gtatgacggg 3720
ctgatactgg gccggcaggc gctccattgc ccagtcggca gcgacatcct tcggcgcgat 3780
tttgccggtt actgcgctgt accaaatgcg ggacaacgta agcactacat ttcgctcatc 3840
gccagcccag tcgggcggcg agttccatag cgttaaggtt tcatttagcg cctcaaatag 3900
atcctgttca ggaaccggat caaagagttc ctccgccgct ggacctacca aggcaacgct 3960
atgttctctt gcttttgtca gcaagatagc cagatcaatg tcgatcgtgg ctggctcgaa 4020
gatacctgca agaatgtcat tgcgctgcca ttctccaaat tgcagttcgc gcttagctgg 4080
ataacgccac ggaatgatgt cgtcgtgcac aacaatggtg acttctacag cgcggagaat 4140
ctcgctctct ccaggggaag ccgaagtttc caaaaggtcg ttgatcaaag ctcgccgcgt 4200
tgtttcatca agccttacgg tcaccgtaac cagcaaatca atatcactgt gtggcttcag 4260
gccgccatcc actgcggagc cgtacaaatg tacggccagc aacgtcggtt cgagatggcg 4320
ctcgatgacg ccaactacct ctgatagttg agtcgatact tcggcgatca ccgcttccct 4380
catactcttc ctttttcaat attattgaag catttatcag ggttattgtc tcatgagcgg 4440
atacatattt gaatgtattt agaaaaataa acaaatagct agctcactcg gtcgctacgc 4500
tccgggcgtg agactgcggc gggcgctgcg gacacataca aagttaccca cagattccgt 4560
ggataagcag gggactaaca tgtgaggcaa aacagcaggg ccgcgccggt ggcgtttttc 4620
cataggctcc gccctcctgc cagagttcac ataaacagac gcttttccgg tgcatctgtg 4680
ggagccgtga ggctcaacca tgaatctgac agtacgggcg aaacccgaca ggacttaaag 4740
atccccaccg tttccggcgg gtcgctccct cttgcgctct cctgttccga ccctgccgtt 4800
taccggatac ctgttccgcc tttctccctt acgggaagtg tggcgctttc tcatagctca 4860
cacactggta tctcggctcg gtgtaggtcg ttcgctccaa gctgggctgt aagcaagaac 4920

tccccgttca gcccgactgc tgcgccttat ccggtaactg ttcacttgag tccaacccgg 4980

aaaagcacgg taaaacgcca ctggcagcag ccattggtaa ctgggagttc gcagaggatt 5040

tgtttagcta aacacgcggt tgctcttgaa gtgtgcgcca aagtccggct acactggaag 5100

gacagatttg gttgctgtgc tctgcgaaag ccagttacca cggttaagca gttccccaac 5160

tgacttaacc ttcgatcaaa ccacctcccc aggtggtttt ttcgtttaca gggcaaaaga 5220

ttacgcgcag aaaaaaagga tctcaagaag atcctttgat cttttctact gaaccgctct 5280

agatttcagt gcaatttatc tcttcaaatg tagcacctga agtcagcccc atacgatata 5340

agttgtaatt ctcatgttag tcatgccccg cgcccaccgg aaggagctga ctgggttgaa 5400

ggctctcaag ggcatcggtc gagatcccgg tgcctaatga gtgagctaac ttacattaat 5460

tgcgttgcgc tcactgcccg ctttccagtc gggaaacctg tcgtgccagc tgcattaatg 5520

aatcggccaa cgcgcgggga gaggcggttt gcgtattggg cgccagggtg gtttttcttt 5580

tcaccagtga gacgggcaac agctgattgc ccttcaccgc ctggccctga gagagttgca 5640

gcaagcggtc cacgctggtt tgccccagca ggcgaaaatc ctgtttgatg gtggttaacg 5700

gcgggatata acatgagctg tcttcggtat cgtcgtatcc cactaccgag atgtccgcac 5760

caacgcgcag cccggactcg gtaatggcgc gcattgcgcc cagcgccatc tgatcgttgg 5820

caaccagcat cgcagtggga acgatgccct cattcagcat ttgcatggtt tgttgaaaac 5880

cggacatggc actccagtcg ccttcccgtt ccgctatcgg ctgaatttga ttgcgagtga 5940

gatatttatg ccagccagcc agacgcagac gcgccgagac agaacttaat gggcccgcta 6000

acagcgcgat ttgctggtga cccaatgcga ccagatgctc cacgcccagt cgcgtaccgt 6060

cttcatggga gaaaataata ctgttgatgg gtgtctggtc agagacatca agaaataacg 6120

ccggaacatt agtgcaggca gcttccacag caatggcatc ctggtcatcc agcggatagt 6180

taatgatcag cccactgacg cgttgcgcga gaagattgtg caccgccgct ttacaggctt 6240

cgacgccgct tcgttctacc atcgacacca ccacgctggc acccagttga tcggcgcgag 6300

atttaatcgc cgcgacaatt tgcgacggcg cgtgcagggc cagactggag gtggcaacgc 6360

caatcagcaa cgactgtttg cccgccagtt gttgtgccac gcggttggga atgtaattca 6420

gctccgccat cgccgcttcc actttttccc gcgttttcgc agaaacgtgg ctggcctggt 6480

tcaccacgcg ggaaacggtc tgataagaga caccggcata ctctgcgaca tcgtataacg 6540

ttactggttt cacattcacc accctgaatt gactctcttc cgggcgctat catgccatac 6600

cgcgaaaggt tttgcgccat tcgatggtgt c 6631

<210> 10
<211> 693
<212> DNA

<213> Pseudomonas putida

<220>
<221> misc_feature
<223> alkL (an importer facilitating transport of hydrophobic acyl across cell membranes) gene

<400> 10

```
gtgagttttt ctaattataa agtaatcgcg atgccggtgt tggttgctaa ttttgttttg      60
ggggcggcca ctgcatgggc gaatgaaaat tatccggcga aatctgctgg ctataatcag     120
ggtgactggg tcgctagctt caatttttct aaggtctatg tgggtgagga gcttggcgat     180
ctaaatgttg gagggggggc tttgccaaat gctgatgtaa gtattggtaa tgatacaaca     240
cttacgtttg atatcgccta ttttgttagc tcaaatatag cggtggattt ttttgttggg     300
gtgccagcta gggctaaatt tcaaggtgag aaatcaatct cctcgctggg aagagtcagt     360
gaagttgatt acggccctgc aattctttcg cttcaatatc attacgatag ctttgagcga     420
ctttatccat atgttggggt tggtgttggt cgggtgctat tttttgataa aaccgacggt     480
gctttgagtt cgtttgatat taaggataaa tgggcgcctg cttttcaggt tggccttaga     540
tatgaccttg gtaactcatg gatgctaaat tcagatgtgc gttatattcc tttcaaaacg     600
gacgtcacag gtactcttgg cccggttcct gtttctacta aaattgaggt tgatcctttc     660
attctcagtc ttggtgcgtc atatgttttc taa                                  693
```

<210> 11
<211> 153
<212> DNA
<213> Artificial Sequence

<220>
<223> lacuv5 promoter

<220>
<221> misc_feature
<223> lacuv5 promoter

<400> 11

```
ggcagtgagc gcaacgcaat taatgtaagt tagctcactc attaggcacc ccaggcttga      60
cactttatgc ttccggctcg tataatgtgt ggaattgtga gcggataaca ataacaattt     120
cacacaggat ctaggaacca aggagagtgg cat                                  153
```

<210> 12
<211> 4876
<212> DNA
<213> Artificial Sequence

<220>
<223> Vector pCDF[alkLmod1]

<220>
<221> misc_feature
<223> Vector pCDF[alkLmod1]

<400> 12
ggggaattgt gagcggataa caattcccct gtagaaataa ttttgtttaa ctttaataag 60

gagatatacc atgggcagca gccatcacca tcatcaccac agccaggatc cttcaatatt 120

attgaagcat ttatcagggt tattgtctca tgagcggata catatttgaa tgtatttaga 180

aaaataaaca aataggggtc agtgttacaa ccaattaacc aattctgaac attatcgcga 240

gcccatttat acctgaatat ggctcataac accccttgtt tgcctggcgg cagtagcgcg 300

gtggtcccac ctgaccccat gccgaactca gaagtgaaac gccgtagcgc cgatggtagt 360

gtggggactc cccatgcgag agtagggaac tgccaggcat caaataaaac gaaaggctca 420

gtcgaaagac tgggcctttc gcccgggcta attatggggt gtcgccctta ttcgactcta 480

tagtgaagtt cctattctct agaaagtata ggaacttctg aagtggggat ccggccggcc 540

tgcagccccg cagggcctgt ctcggtcgat cattcagccc ggctcataga tatgcgggca 600

gtgagcgcaa cgcaattaat gtaagttagc tcactcatta ggcaccccag gcttgacact 660

ttatgcttcc ggctcgtata atgtgtggaa ttgtgagcgg ataacaataa caatttcaca 720

caggatctag gaaccaagga gagtggcatg tgagtttttc taattataaa gtaatcgcga 780

tgccggtgtt ggttgctaat tttgttttgg gggcggccac tgcatgggcg aatgaaaatt 840

atccggcgaa atctgctggc tataatcagg gtgactgggt cgctagcttc aatttttcta 900

aggtctatgt gggtgaggag cttggcgatc taaatgttgg aggggggggct ttgccaaatg 960

ctgatgtaag tattggtaat gatacaacac ttacgtttga tatcgcctat tttgttagct 1020

caaatatagc ggtggatttt tttgttgggg tgccagctag ggctaaattt caaggtgaga 1080

aatcaatctc ctcgctggga agagtcagtg aagttgatta cggccctgca attctttcgc 1140

ttcaatatca ttacgatagc tttgagcgac tttatccata tgttggggtt ggtgttggtc 1200

gggtgctatt ttttgataaa accgacggtg ctttgagttc gtttgatatt aaggataaat 1260

gggcgcctgc ttttcaggtt ggccttagat atgaccttgg taactcatgg atgctaaatt 1320

cagatgtgcg ttatattcct ttcaaaacgg acgtcacagg tactcttggc ccggttcctg 1380

tttctactaa aattgaggtt gatcctttca ttctcagtct tggtgcgtca tatgttttct 1440

aaggtaccct cgagtctggt aaagaaaccg ctgctgcgaa atttgaacgc cagcacatgg 1500

actcgtctac tagcgcagct taattaacct aggctgctgc caccgctgag caataactag 1560

cataacccct tggggcctct aaacgggtct tgaggggttt tttgctgaaa cctcaggcat 1620

ttgagaagca cacggtcaca ctgcttccgg tagtcaataa accggtaaac cagcaataga 1680

cataagcggc tatttaacga ccctgccctg aaccgacgac cgggtcatcg tggccggatc 1740

ttgcggcccc tcggcttgaa cgaattgtta gacattattt gccgactacc ttggtgatct 1800

cgcctttcac gtagtggaca aattcttcca actgatctgc gcgcgaggcc aagcgatctt 1860

cttcttgtcc aagataagcc tgtctagctt caagtatgac gggctgatac tgggccggca 1920

ggcgctccat tgcccagtcg gcagcgacat ccttcggcgc gattttgccg gttactgcgc 1980

tgtaccaaat gcgggacaac gtaagcacta catttcgctc atcgccagcc cagtcgggcg 2040

gcgagttcca tagcgttaag gtttcattta gcgcctcaaa tagatcctgt tcaggaaccg 2100

gatcaaagag ttcctccgcc gctggaccta ccaaggcaac gctatgttct cttgcttttg 2160

tcagcaagat agccagatca atgtcgatcg tggctggctc gaagatacct gcaagaatgt 2220

cattgcgctg ccattctcca aattgcagtt cgcgcttagc tggataacgc cacggaatga 2280

tgtcgtcgtg cacaacaatg gtgacttcta cagcgcggag aatctcgctc tctccagggg 2340

aagccgaagt ttccaaaagg tcgttgatca aagctcgccg cgttgtttca tcaagcctta 2400

cggtcaccgt aaccagcaaa tcaatatcac tgtgtggctt caggccgcca tccactgcgg 2460

agccgtacaa atgtacggcc agcaacgtcg gttcgagatg gcgctcgatg acgccaacta 2520

cctctgatag ttgagtcgat acttcggcga tcaccgcttc cctcatactc ttcctttttc 2580

aatattattg aagcatttat cagggttatt gtctcatgag cggatacata tttgaatgta 2640

tttagaaaaa taaacaaata gctagctcac tcggtcgcta cgctccgggc gtgagactgc 2700

ggcgggcgct gcggacacat acaaagttac ccacagattc cgtggataag caggggacta 2760

acatgtgagg caaaacagca gggccgcgcc ggtggcgttt ttccataggc tccgccctcc 2820

tgccagagtt cacataaaca gacgcttttc cggtgcatct gtgggagccg tgaggctcaa 2880

ccatgaatct gacagtacgg gcgaaacccg acaggactta aagatcccca ccgtttccgg 2940

cgggtcgctc cctcttgcgc tctcctgttc cgaccctgcc gtttaccgga tacctgttcc 3000

gcctttctcc cttacgggaa gtgtggcgct ttctcatagc tcacacactg gtatctcggc 3060

tcggtgtagg tcgttcgctc caagctgggc tgtaagcaag aactccccgt tcagcccgac 3120

tgctgcgcct tatccggtaa ctgttcactt gagtccaacc cggaaaagca cggtaaaacg 3180

ccactggcag cagccattgg taactgggag ttcgcagagg atttgtttag ctaaacacgc 3240

ggttgctctt gaagtgtgcg ccaaagtccg gctacactgg aaggacagat ttggttgctg 3300

tgctctgcga aagccagtta ccacggttaa gcagttcccc aactgactta accttcgatc 3360

aaaccacctc cccaggtggt tttttcgttt acagggcaaa agattacgcg cagaaaaaaa 3420

ggatctcaag aagatccttt gatcttttct actgaaccgc tctagatttc agtgcaattt 3480

atctcttcaa atgtagcacc tgaagtcagc cccatacgat ataagttgta attctcatgt 3540

tagtcatgcc ccgcgcccac cggaaggagc tgactgggtt gaaggctctc aagggcatcg 3600

gtcgagatcc cggtgcctaa tgagtgagct aacttacatt aattgcgttg cgctcactgc 3660

ccgctttcca gtcgggaaac ctgtcgtgcc agctgcatta atgaatcggc caacgcgcgg 3720

ggagaggcgg tttgcgtatt gggcgccagg gtggtttttc ttttcaccag tgagacgggc 3780

aacagctgat tgcccttcac cgcctggccc tgagagagtt gcagcaagcg gtccacgctg 3840

gtttgcccca gcaggcgaaa atcctgtttg atggtggtta acggcgggat ataacatgag 3900

ctgtcttcgg tatcgtcgta tcccactacc gagatgtccg caccaacgcg cagcccggac 3960

tcggtaatgg cgcgcattgc gcccagcgcc atctgatcgt tggcaaccag catcgcagtg 4020

ggaacgatgc cctcattcag catttgcatg gtttgttgaa aaccggacat ggcactccag 4080

tcgccttccc gttccgctat cggctgaatt tgattgcgag tgagatattt atgccagcca 4140

gccagacgca gacgcgccga gacagaactt aatgggcccg ctaacagcgc gatttgctgg 4200

tgacccaatg cgaccagatg ctccacgccc agtcgcgtac cgtcttcatg ggagaaaata 4260

atactgttga tgggtgtctg gtcagagaca tcaagaaata acgccggaac attagtgcag 4320

gcagcttcca cagcaatggc atcctggtca tccagcggat agttaatgat cagcccactg 4380

acgcgttgcg cgagaagatt gtgcaccgcc gctttacagg cttcgacgcc gcttcgttct 4440

accatcgaca ccaccacgct ggcacccagt tgatcggcgc gagatttaat cgccgcgaca 4500

atttgcgacg gcgcgtgcag ggccagactg gaggtggcaa cgccaatcag caacgactgt 4560

ttgcccgcca gttgttgtgc cacgcggttg ggaatgtaat tcagctccgc catcgccgct 4620

tccacttttt cccgcgtttt cgcagaaacg tggctggcct ggttcaccac gcgggaaacg 4680

gtctgataag agacaccggc atactctgcg acatcgtata acgttactgg tttcacattc 4740

accaccctga attgactctc ttccgggcgc tatcatgcca taccgcgaaa ggttttgcgc 4800

cattcgatgg tgtccgggat ctcgacgctc tcccttatgc gactcctgca ttaggaaatt 4860

aatacgactc actata 4876

<210> 13
<211> 7468
<212> DNA
<213> Artificial Sequence

<220>
<223> Vector pCDF{Ptac}[hGLYAT2(co_Ec)-fadD_Ec]{Placuv5}[alkLmod1]

<220>
<221> misc_feature
<223> Vector pCDF{Ptac}[hGLYAT2(co_Ec)-fadD_Ec]{Placuv5}[alkLmod1]

<400> 13
cgggatctcg acgctctccc ttatgcgact cctgcgttta gggaaagagc atttgtcaga 60

atatttaagg gcgcctgtca ctttgcttga tatatgagaa ttatttaacc ttataaatga 120

gaaaaaagca acgcacttta aataagatac gttgcttttt cgattgatga acacctataa 180
ttaaactatt catctattat ttatgatttt ttgtatatac aatatttcta gtttgttaaa 240
gagaattaag aaaataaatc tcgaaaataa taaagggaaa atcagttttt gatatcaaaa 300
ttatacatgt caacgataat acaaaatata atacaaacta taagatgtta tcagtattta 360
ttatgcattg tctcggtcga tcattcagcc cggctcatag atatgcgggc agtgagcgca 420
acgcaattaa tgtaagttag ctcactcatt aggcacccca ggcttgacac tttatgcttc 480
cggctcgtat aatgtgtgga attgtgagcg gataacaata acaatttcac acaggatcta 540
ggaaccaagg agagtggcat gtgagttttt ctaattataa agtaatcgcg atgccggtgt 600
tggttgctaa ttttgttttg gggcggcca ctgcatgggc gaatgaaaat tatccggcga 660
aatctgctgg ctataatcag ggtgactggg tcgctagctt caatttttct aaggtctatg 720
tgggtgagga gcttggcgat ctaaatgttg gaggggggc tttgccaaat gctgatgtaa 780
gtattggtaa tgatacaaca cttacgtttg atatcgccta ttttgttagc tcaaatatag 840
cggtggattt ttttgttggg gtgccagcta gggctaaatt tcaaggtgag aaatcaatct 900
cctcgctggg aagagtcagt gaagttgatt acggccctgc aattctttcg cttcaatatc 960
attacgatag ctttgagcga ctttatccat atgttggggt tggtgttggt cgggtgctat 1020
tttttgataa aaccgacggt gctttgagtt cgtttgatat taaggataaa tgggcgcctg 1080
cttttcaggt tggccttaga tatgaccttg gtaactcatg gatgctaaat tcagatgtgc 1140
gttatattcc tttcaaaacg gacgtcacag gtactcttgg cccggttcct gtttctacta 1200
aaattgaggt tgatcctttc attctcagtc ttggtgcgtc atatgttttc taaggtaccc 1260
tcgagtctgg tggatccaat tgtgagcgga taacaattac gagcttcatg cacagtgatc 1320
gacgctgttg acaattaatc atcggctcgt ataatgtgtg gatgtggaat tgtgagcgct 1380
cacaattcca caacggtttc cctctagaaa taattttgtt taacaggagg taaaacatat 1440
gctggttctg cacaacagcc aaaaactgca aattctgtac aaatctctgg aaaaatctat 1500
tcctgaaagc attaaagttt atggtgcgat cttcaacatc aaggataaaa accctttcaa 1560
tatggaggtg ctggtcgacg cgtggcctga ttaccaaatc gtcatcaccc gtccgcagaa 1620
gcaagaaatg aaagatgacc aggaccacta tactaacacc taccacatct tcactaaagc 1680
gccggacaaa ctggaagaag ttctgagcta ctccaatgtt atctcctggg aacaaacgct 1740
gcagattcaa ggttgccagg aaggtctgga tgaggcgatt cgtaaggtcg ctacctctaa 1800
gtccgttcaa gtcgattaca tgaaaaccat cctgtttatc ccggagctgc cgaagaaaca 1860
caaaacctcc tctaacgaca agatggagct gttcgaagta gacgatgaca acaaagaggg 1920
taacttttcc aatatgttcc tggacgcctc ccacgccggt ctggttaatg agcactgggc 1980
gttcggtaaa aacgaacgct ccctgaagta cattgagcgt tgtctgcagg attttctggg 2040

ttttggtgtc ctgggtccag agggtcaact ggtctcttgg atcgttatgg aacagtcttg 2100

tgaactgcgt atgggttata ctgtgccaaa ataccgtcac caaggtaata tgctgcaaat 2160

cggttatcat ctggagaagt acctgagcca gaaggaaatc ccgttctatt tccatgttgc 2220

cgataataac gaaaagagcc tgcaagccct gaacaatctg ggcttcaaga tctgcccttg 2280

cggttggcac cagtggaagt gcacgcctaa aaagtactgt taagcgttaa gtctgagagg 2340

tgaagaattg aagaaggttt ggcttaaccg ttatcccgcg gacgttccga cggagatcaa 2400

ccctgaccgt tatcaatctc tggtagatat gtttgagcag tcggtcgcgc gctacgccga 2460

tcaacctgcg tttgtgaata tgggggaggt aatgaccttc cgcaagctgg aagaacgcag 2520

tcgcgcgttt gccgcttatt tgcaacaagg gttggggctg aagaaaggcg atcgcgttgc 2580

gttgatgatg cctaatttat tgcaatatcc ggtggcgctg tttggcattt tgcgtgccgg 2640

gatgatcgtc gtaaacgtta acccgttgta taccccgcgt gagcttgagc atcagcttaa 2700

cgatagcggc gcatcggcga ttgttatcgt gtctaacttt gctcacacac tggaaaaagt 2760

ggttgataaa accgccgttc agcacgtaat tctgacccgt atgggcgatc agctatctac 2820

ggcaaaaggc acggtagtca atttcgttgt taaatacatc aagcgtttgg tgccgaaata 2880

ccatctgcca gatgccattt catttcgtag cgcactgcat aacggctacc ggatgcagta 2940

cgtcaaaccc gaactggtgc cggaagattt agcttttctg caatacaccg gcggcaccac 3000

tggtgtggcg aaaggcgcga tgctgactca ccgcaatatg ctggcgaacc tggaacaggt 3060

taacgcgacc tatggtccgc tgttgcatcc gggcaaagag ctggtggtga cggcgctgcc 3120

gctgtatcac atttttgccc tgaccattaa ctgcctgctg tttatcgaac tgggtgggca 3180

gaacctgctt atcactaacc cgcgcgatat tccagggttg gtaaaagagt tagcgaaata 3240

tccgtttacc gctatcacgg gcgttaacac cttgttcaat gcgttgctga acaataaaga 3300

gttccagcag ctggatttct ccagtctgca tctttccgca ggcggtggga tgccagtgca 3360

gcaagtggtg gcagagcgtt gggtgaaact gaccggacag tatctgctgg aaggctatgg 3420

ccttaccgag tgtgcgccgc tggtcagcgt taacccatat gatattgatt atcatagtgg 3480

tagcatcggt ttgccggtgc cgtcgacgga agccaaactg gtggatgatg atgataatga 3540

agtaccacca ggtcaaccgg gtgagctttg tgtcaaagga ccgcaggtga tgctgggtta 3600

ctggcagcgt cccgatgcta ccgatgaaat catcaaaaat ggctggttac acaccggcga 3660

catcgcggta atggatgaag aaggattcct gcgcattgtc gatcgtaaaa aagacatgat 3720

tctggtttcc ggttttaacg tctatcccaa cgagattgaa gatgtcgtca tgcagcatcc 3780

tggcgtacag gaagtcgcgg ctgttggcgt accttccggc tccagtggtg aagcggtgaa 3840

aatcttcgta gtgaaaaaag atccatcgct taccgaagag tcactggtga ctttttgccg 3900

ccgtcagctc acgggataca aagtaccgaa gctggtggag tttcgtgatg agttaccgaa 3960

atctaacgtc ggaaaaattt tgcgacgaga attacgtgac gaagcgcgcg gcaaagtgga 4020

caataaagcc tgagcgttaa gtcagtcgtc agacgccggt taatccggcg ttttttttga 4080

cgcccactaa agagaaaaca atctcgagtc tggtaaagaa accgctgctg cgaaatttga 4140

acgccagcac atggactcgt ctactagcgc agcttaatta acctaggctg ctgccaccgc 4200

tgagcaataa ctagcataac cccttggggc ctctaaacgg gtcttgaggg gttttttgct 4260

gaaacctcag gcatttgaga agcacacggt cacactgctt ccggtagtca ataaaccggt 4320

aaaccagcaa tagacataag cggctattta acgaccctgc cctgaaccga cgaccgggtc 4380

atcgtggccg gatcttgcgg cccctcggct tgaacgaatt gttagacatt atttgccgac 4440

taccttggtg atctcgcctt tcacgtagtg gacaaattct tccaactgat ctgcgcgcga 4500

ggccaagcga tcttcttctt gtccaagata agcctgtcta gcttcaagta tgacgggctg 4560

atactgggcc ggcaggcgct ccattgccca gtcggcagcg acatccttcg gcgcgatttt 4620

gccggttact gcgctgtacc aaatgcggga caacgtaagc actacatttc gctcatcgcc 4680

agcccagtcg ggcggcgagt tccatagcgt taaggtttca tttagcgcct caaatagatc 4740

ctgttcagga accggatcaa agagttcctc cgccgctgga cctaccaagg caacgctatg 4800

ttctcttgct tttgtcagca agatagccag atcaatgtcg atcgtggctg gctcgaagat 4860

acctgcaaga atgtcattgc gctgccattc tccaaattgc agttcgcgct tagctggata 4920

acgccacgga atgatgtcgt cgtgcacaac aatggtgact tctacagcgc ggagaatctc 4980

gctctctcca ggggaagccg aagtttccaa aaggtcgttg atcaaagctc gccgcgttgt 5040

ttcatcaagc cttacggtca ccgtaaccag caaatcaata tcactgtgtg gcttcaggcc 5100

gccatccact gcggagccgt acaaatgtac ggccagcaac gtcggttcga gatggcgctc 5160

gatgacgcca actacctctg atagttgagt cgatacttcg gcgatcaccg cttccctcat 5220

actcttcctt tttcaatatt attgaagcat ttatcagggt tattgtctca tgagcggata 5280

catatttgaa tgtatttaga aaaataaaca aatagctagc tcactcggtc gctacgctcc 5340

gggcgtgaga ctgcggcggg cgctgcggac acatacaaag ttacccacag attccgtgga 5400

taagcagggg actaacatgt gaggcaaaac agcagggccg cgccggtggc gtttttccat 5460

aggctccgcc ctcctgccag agttcacata aacagacgct tttccggtgc atctgtggga 5520

gccgtgaggc tcaaccatga atctgacagt acgggcgaaa cccgacagga cttaaagatc 5580

cccaccgttt ccggcgggtc gctccctctt gcgctctcct gttccgaccc tgccgtttac 5640

cggatacctg ttccgccttt ctcccttacg ggaagtgtgg cgctttctca tagctcacac 5700

actggtatct cggctcggtg taggtcgttc gctccaagct gggctgtaag caagaactcc 5760

ccgttcagcc cgactgctgc gccttatccg gtaactgttc acttgagtcc aacccggaaa 5820

agcacggtaa aacgccactg gcagcagcca ttggtaactg ggagttcgca gaggatttgt 5880

ttagctaaac acgcggttgc tcttgaagtg tgcgccaaag tccggctaca ctggaaggac 5940

agatttggtt gctgtgctct gcgaaagcca gttaccacgg ttaagcagtt ccccaactga 6000

cttaaccttc gatcaaacca cctccccagg tggttttttc gtttacaggg caaaagatta 6060

cgcgcagaaa aaaaggatct caagaagatc ctttgatctt ttctactgaa ccgctctaga 6120

tttcagtgca atttatctct tcaaatgtag cacctgaagt cagccccata cgatataagt 6180

tgtaattctc atgttagtca tgccccgcgc ccaccggaag gagctgactg ggttgaaggc 6240

tctcaagggc atcggtcgag atcccggtgc ctaatgagtg agctaactta cattaattgc 6300

gttgcgctca ctgcccgctt tccagtcggg aaacctgtcg tgccagctgc attaatgaat 6360

cggccaacgc gcggggagag gcggtttgcg tattgggcgc cagggtggtt tttcttttca 6420

ccagtgagac gggcaacagc tgattgccct tcaccgcctg gccctgagag agttgcagca 6480

agcggtccac gctggtttgc cccagcaggc gaaaatcctg tttgatggtg gttaacggcg 6540

ggatataaca tgagctgtct tcggtatcgt cgtatcccac taccgagatg tccgcaccaa 6600

cgcgcagccc ggactcggta atggcgcgca ttgcgcccag cgccatctga tcgttggcaa 6660

ccagcatcgc agtgggaacg atgccctcat tcagcatttg catggtttgt tgaaaaccgg 6720

acatggcact ccagtcgcct tcccgttccg ctatcggctg aatttgattg cgagtgagat 6780

atttatgcca gccagccaga cgcagacgcg ccgagacaga acttaatggg cccgctaaca 6840

gcgcgatttg ctggtgaccc aatgcgacca gatgctccac gcccagtcgc gtaccgtctt 6900

catgggagaa aataatactg ttgatgggtg tctggtcaga gacatcaaga aataacgccg 6960

gaacattagt gcaggcagct tccacagcaa tggcatcctg gtcatccagc ggatagttaa 7020

tgatcagccc actgacgcgt tgcgcgagaa gattgtgcac cgccgcttta caggcttcga 7080

cgccgcttcg ttctaccatc gacaccacca cgctggcacc cagttgatcg gcgcgagatt 7140

taatcgccgc gacaatttgc gacggcgcgt gcagggccag actggaggtg gcaacgccaa 7200

tcagcaacga ctgtttgccc gccagttgtt gtgccacgcg gttgggaatg taattcagct 7260

ccgccatcgc cgcttccact ttttcccgcg ttttcgcaga aacgtggctg gcctggttca 7320

ccacgcggga aacggtctga taagagacac cggcatactc tgcgacatcg tataacgtta 7380

ctggtttcac attcaccacc ctgaattgac tctcttccgg gcgctatcat gccataccgc 7440

gaaaggtttt gcgccattcg atggtgtc 7468

<210> 14
<211> 5367
<212> DNA
<213> Artificial Sequence

```
<220>
<223>  Vector pET-28b


<220>
<221>  misc_feature
<223>  Vector pET-28b

<400>  14
ggcgaatggg acgcgccctg tagcggcgca ttaagcgcgg cgggtgtggt ggttacgcgc      60

agcgtgaccg ctacacttgc cagcgcccta gcgcccgctc ctttcgcttt cttcccttcc     120

tttctcgcca cgttcgccgg ctttccccgt caagctctaa atcgggggct ccctttaggg     180

ttccgattta gtgctttacg gcacctcgac cccaaaaaac ttgattaggg tgatggttca     240

cgtagtgggc catcgccctg atagacggtt tttcgccctt tgacgttgga gtccacgttc     300

tttaatagtg gactcttgtt ccaaactgga acaacactca accctatctc ggtctattct     360

tttgatttat aagggatttt gccgatttcg gcctattggt taaaaaatga gctgatttaa     420

caaaaattta acgcgaattt taacaaaata ttaacgttta caatttcagg tggcactttt     480

cggggaaatg tgcgcggaac ccctatttgt ttatttttct aaatacattc aaatatgtat     540

ccgctcatga attaattctt agaaaaactc atcgagcatc aaatgaaact gcaatttatt     600

catatcagga ttatcaatac catatttttg aaaaagccgt ttctgtaatg aaggagaaaa     660

ctcaccgagg cagttccata ggatggcaag atcctggtat cggtctgcga ttccgactcg     720

tccaacatca atacaaccta ttaatttccc ctcgtcaaaa ataaggttat caagtgagaa     780

atcaccatga gtgacgactg aatccggtga gaatggcaaa agtttatgca tttctttcca     840

gacttgttca acaggccagc cattacgctc gtcatcaaaa tcactcgcat caaccaaacc     900

gttattcatt cgtgattgcg cctgagcgag acgaaatacg cgatcgctgt taaaaggaca     960

attacaaaca ggaatcgaat gcaaccggcg caggaacact gccagcgcat caacaatatt    1020

ttcacctgaa tcaggatatt cttctaatac ctggaatgct gttttcccgg ggatcgcagt    1080

ggtgagtaac catgcatcat caggagtacg gataaaatgc ttgatggtcg gaagaggcat    1140

aaattccgtc agccagttta gtctgaccat ctcatctgta acatcattgg caacgctacc    1200

tttgccatgt ttcagaaaca actctggcgc atcgggcttc ccatacaatc gatagattgt    1260

cgcacctgat tgcccgacat tatcgcgagc ccatttatac ccatataaat cagcatccat    1320

gttggaattt aatcgcggcc tagagcaaga cgtttcccgt tgaatatggc tcataacacc    1380

ccttgtatta ctgtttatgt aagcagacag ttttattgtt catgaccaaa atcccttaac    1440

gtgagttttc gttccactga gcgtcagacc ccgtagaaaa gatcaaagga tcttcttgag    1500

atcctttttt tctgcgcgta atctgctgct tgcaaacaaa aaaaccaccg ctaccagcgg    1560

tggtttgttt gccggatcaa gagctaccaa ctctttttcc gaaggtaact ggcttcagca    1620
```

gagcgcagat accaaatact gtccttctag tgtagccgta gttaggccac cacttcaaga 1680
actctgtagc accgcctaca tacctcgctc tgctaatcct gttaccagtg gctgctgcca 1740
gtggcgataa gtcgtgtctt accgggttgg actcaagacg atagttaccg gataaggcgc 1800
agcggtcggg ctgaacgggg ggttcgtgca cacagcccag cttggagcga acgacctaca 1860
ccgaactgag atacctacag cgtgagctat gagaaagcgc cacgcttccc gaagggagaa 1920
aggcggacag gtatccggta agcggcaggg tcggaacagg agagcgcacg agggagcttc 1980
caggggggaaa cgcctggtat ctttatagtc ctgtcgggtt tcgccacctc tgacttgagc 2040
gtcgattttt gtgatgctcg tcaggggggc ggagcctatg gaaaaacgcc agcaacgcgg 2100
cctttttacg gttcctggcc ttttgctggc cttttgctca catgttcttt cctgcgttat 2160
cccctgattc tgtggataac cgtattaccg cctttgagtg agctgatacc gctcgccgca 2220
gccgaacgac cgagcgcagc gagtcagtga gcgaggaagc ggaagagcgc ctgatgcggt 2280
attttctcct tacgcatctg tgcggtattt cacaccgcat atatggtgca ctctcagtac 2340
aatctgctct gatgccgcat agttaagcca gtatacactc cgctatcgct acgtgactgg 2400
gtcatggctg cgccccgaca cccgccaaca cccgctgacg cgccctgacg ggcttgtctg 2460
ctcccggcat ccgcttacag acaagctgtg accgtctccg ggagctgcat gtgtcagagg 2520
ttttcaccgt catcaccgaa acgcgcgagg cagctgcggt aaagctcatc agcgtggtcg 2580
tgaagcgatt cacagatgtc tgcctgttca tccgcgtcca gctcgttgag tttctccaga 2640
agcgttaatg tctggcttct gataaagcgg gccatgttaa gggcggtttt ttcctgtttg 2700
gtcactgatg cctccgtgta agggggattt ctgttcatgg gggtaatgat accgatgaaa 2760
cgagagagga tgctcacgat acgggttact gatgatgaac atgcccggtt actggaacgt 2820
tgtgagggta aacaactggc ggtatggatg cggcgggacc agagaaaaat cactcagggt 2880
caatgccagc gcttcgttaa tacagatgta ggtgttccac agggtagcca gcagcatcct 2940
gcgatgcaga tccggaacat aatggtgcag ggcgctgact tccgcgtttc cagactttac 3000
gaaacacgga aaccgaagac cattcatgtt gttgctcagg tcgcagacgt tttgcagcag 3060
cagtcgcttc acgttcgctc gcgtatcggt gattcattct gctaaccagt aaggcaaccc 3120
cgccagccta gccgggtcct caacgacagg agcacgatca tgcgcacccg tggggccgcc 3180
atgccggcga taatggcctg cttctcgccg aaacgtttgg tggcgggacc agtgacgaag 3240
gcttgagcga gggcgtgcaa gattccgaat accgcaagcg acaggccgat catcgtcgcg 3300
ctccagcgaa agcggtcctc gccgaaaatg acccagagcg ctgccggcac ctgtcctacg 3360
agttgcatga taaagaagac agtcataagt gcggcgacga tagtcatgcc ccgcgcccac 3420
cggaaggagc tgactgggtt gaaggctctc aagggcatcg gtcgagatcc cggtgcctaa 3480
tgagtgagct aacttacatt aattgcgttg cgctcactgc ccgctttcca gtcgggaaac 3540

ctgtcgtgcc agctgcatta atgaatcggc caacgcgcgg ggagaggcgg tttgcgtatt 3600
gggcgccagg gtggtttttc ttttcaccag tgagacgggc aacagctgat tgcccttcac 3660
cgcctggccc tgagagagtt gcagcaagcg gtccacgctg gtttgcccca gcaggcgaaa 3720
atcctgtttg atggtggtta acggcgggat ataacatgag ctgtcttcgg tatcgtcgta 3780
tcccactacc gagatatccg caccaacgcg cagcccggac tcggtaatgg cgcgcattgc 3840
gcccagcgcc atctgatcgt tggcaaccag catcgcagtg ggaacgatgc cctcattcag 3900
catttgcatg gtttgttgaa aaccggacat ggcactccag tcgccttccc gttccgctat 3960
cggctgaatt tgattgcgag tgagatattt atgccagcca gccagacgca gacgcgccga 4020
gacagaactt aatgggcccg ctaacagcgc gatttgctgg tgacccaatg cgaccagatg 4080
ctccacgccc agtcgcgtac cgtcttcatg ggagaaaata atactgttga tgggtgtctg 4140
gtcagagaca tcaagaaata acgccggaac attagtgcag gcagcttcca cagcaatggc 4200
atcctggtca tccagcggat agttaatgat cagcccactg acgcgttgcg cgagaagatt 4260
gtgcaccgcc gctttacagg cttcgacgcc gcttcgttct accatcgaca ccaccacgct 4320
ggcacccagt tgatcggcgc gagatttaat cgccgcgaca atttgcgacg gcgcgtgcag 4380
ggccagactg gaggtggcaa cgccaatcag caacgactgt ttgcccgcca gttgttgtgc 4440
cacgcggttg ggaatgtaat tcagctccgc catcgccgct tccacttttt cccgcgtttt 4500
cgcagaaacg tggctggcct ggttcaccac gcgggaaacg gtctgataag agacaccggc 4560
atactctgcg acatcgtata acgttactgg tttcacattc accaccctga attgactctc 4620
ttccgggcgc tatcatgcca taccgcgaaa ggttttgcgc cattcgatgg tgtccgggat 4680
ctcgacgctc tcccttatgc gactcctgca ttaggaagca gcccagtagt aggttgaggc 4740
cgttgagcac cgccgccgca aggaatggtg catgcaagga gatggcgccc aacagtcccc 4800
cggccacggg gcctgccacc atacccacgc cgaaacaagc gctcatgagc ccgaagtggc 4860
gagcccgatc ttccccatcg gtgatgtcgg cgatataggc gccagcaacc gcacctgtgg 4920
cgccggtgat gccggccacg atgcgtccgg cgtagaggat cgagatctcg atcccgcgaa 4980
attaatacga ctcactatag gggaattgtg agcggataac aattcccctc tagaaataat 5040
tttgtttaac tttaagaagg agatatacca tgggcagcag ccatcatcat catcatcaca 5100
gcagcggcct ggtgccgcgc ggcagccata tggctagcat gactggtgga cagcaaatgg 5160
gtcgggatcc gaattcgagc tccgtcgaca agcttgcggc cgcactcgag caccaccacc 5220
accaccactg agatccggct gctaacaaag cccgaaagga agctgagttg gctgctgcca 5280
ccgctgagca ataactagca taaccccttg gggcctctaa acgggtcttg aggggttttt 5340
tgctgaaagg aggaactata tccggat 5367

<210> 15
<211> 6432
<212> DNA
<213> Artificial Sequence

<220>
<223> Vector pET-28b{Ptac}[hGLYAT2(co_Ec)]

<220>
<221> misc_feature
<223> Vector pET-28b{Ptac}[hGLYAT2(co_Ec)]

<400> 15
ggccgctctg gtggatccaa ttgtgagcgg ataacaatta cgagcttcat gcacagtgat 60

cgacgctgtt gacaattaat catcggctcg tataatgtgt ggatgtggaa ttgtgagcgc 120

tcacaattcc acaacggttt ccctctagaa ataattttgt ttaacaggag gtaaaacata 180

tgctggttct gcacaacagc caaaaactgc aaattctgta caaatctctg gaaaaatcta 240

ttcctgaaag cattaaagtt tatggtgcga tcttcaacat caaggataaa aacccttca 300

atatggaggt gctggtcgac gcgtggcctg attaccaaat cgtcatcacc cgtccgcaga 360

agcaagaaat gaaagatgac caggaccact atactaacac ctaccacatc ttcactaaag 420

cgccggacaa actggaagaa gttctgagct actccaatgt tatctcctgg gaacaaacgc 480

tgcagattca aggttgccag gaaggtctgg atgaggcgat tcgtaaggtc gctacctcta 540

agtccgttca agtcgattac atgaaaacca tcctgtttat cccggagctg ccgaagaaac 600

acaaaacctc ctctaacgac aagatggagc tgttcgaagt agacgatgac aacaaagagg 660

gtaacttttc caatatgttc ctggacgcct cccacgccgg tctggttaat gagcactggg 720

cgttcggtaa aaacgaacgc tccctgaagt acattgagcg ttgtctgcag gattttctgg 780

gttttggtgt cctgggtcca gagggtcaac tggtctcttg gatcgttatg gaacagtctt 840

gtgaactgcg tatgggttat actgtgccaa aataccgtca ccaaggtaat atgctgcaaa 900

tcggttatca tctggagaag tacctgagcc agaaggaaat cccgttctat ttccatgttg 960

ccgataataa cgaaaagagc ctgcaagccc tgaacaatct gggcttcaag atctgccctt 1020

gcggttggca ccagtggaag tgcacgccta aaaagtactg ttaagcgtta agtctgagag 1080

gtgagagctc gaattcggat cccgacccat ttgctgtcca ccagtcatgc tagccatatg 1140

gctgccgcgc ggcaccaggc cgctgctgtg atgatgatga tgatggctgc tgcccatggt 1200

atatctcctt cttaaagtta aacaaaatta tttctagagg ggaattgtta tccgctcaca 1260

attcccctat agtgagtcgt attaatttcg cgggatcgag atctcgatcc tctacgccgg 1320

acgcatcgtg gccggcatca ccggcgccac aggtgcggtt gctggcgcct atatcgccga 1380

catcaccgat ggggaagatc gggctcgcca cttcgggctc atgagcgctt gtttcggcgt 1440

gggtatggtg gcaggccccg tggccggggg actgttgggc gccatctcct tgcatgcacc 1500
attccttgcg gcggcggtgc tcaacggcct caacctacta ctgggctgct tcctaatgca 1560
ggagtcgcat aagggagagc gtcgagatcc cggacaccat cgaatggcgc aaaacctttc 1620
gcggtatggc atgatagcgc ccggaagaga gtcaattcag ggtggtgaat gtgaaaccag 1680
taacgttata cgatgtcgca gagtatgccg gtgtctctta tcagaccgtt tcccgcgtgg 1740
tgaaccaggc cagccacgtt tctgcgaaaa cgcgggaaaa agtggaagcg gcgatggcgg 1800
agctgaatta cattcccaac cgcgtggcac aacaactggc gggcaaacag tcgttgctga 1860
ttggcgttgc cacctccagt ctggccctgc acgcgccgtc gcaaattgtc gcggcgatta 1920
aatctcgcgc cgatcaactg ggtgccagcg tggtggtgtc gatggtagaa cgaagcggcg 1980
tcgaagcctg taaagcggcg gtgcacaatc ttctcgcgca acgcgtcagt gggctgatca 2040
ttaactatcc gctggatgac caggatgcca ttgctgtgga agctgcctgc actaatgttc 2100
cggcgttatt tcttgatgtc tctgaccaga cacccatcaa cagtattatt ttctcccatg 2160
aagacggtac gcgactgggc gtggagcatc tggtcgcatt gggtcaccag caaatcgcgc 2220
tgttagcggg cccattaagt tctgtctcgg cgcgtctgcg tctggctggc tggcataaat 2280
atctcactcg caatcaaatt cagccgatag cggaacggga aggcgactgg agtgccatgt 2340
ccggttttca acaaaccatg caaatgctga atgagggcat cgttcccact gcgatgctgg 2400
ttgccaacga tcagatggcg ctgggcgcaa tgcgcgccat taccgagtcc gggctgcgcg 2460
ttggtgcgga tatctcggta gtgggatacg acgataccga agacagctca tgttatatcc 2520
cgccgttaac caccatcaaa caggattttc gcctgctggg gcaaaccagc gtggaccgct 2580
tgctgcaact ctctcagggc caggcggtga agggcaatca gctgttgccc gtctcactgg 2640
tgaaaagaaa aaccaccctg gcgcccaata cgcaaaccgc ctctccccgc gcgttggccg 2700
attcattaat gcagctggca cgacaggttt cccgactgga aagcgggcag tgagcgcaac 2760
gcaattaatg taagttagct cactcattag gcaccgggat ctcgaccgat gcccttgaga 2820
gccttcaacc cagtcagctc cttccggtgg gcgcggggca tgactatcgt cgccgcactt 2880
atgactgtct tctttatcat gcaactcgta ggacaggtgc cggcagcgct ctgggtcatt 2940
ttcggcgagg accgctttcg ctggagcgcg acgatgatcg gcctgtcgct tgcggtattc 3000
ggaatcttgc acgccctcgc tcaagccttc gtcactggtc ccgccaccaa acgtttcggc 3060
gagaagcagg ccattatcgc cggcatggcg gccccacggg tgcgcatgat cgtgctcctg 3120
tcgttgagga cccggctagg ctggcggggt tgccttactg gttagcagaa tgaatcaccg 3180
atacgcgagc gaacgtgaag cgactgctgc tgcaaaacgt ctgcgacctg agcaacaaca 3240
tgaatggtct tcggtttccg tgtttcgtaa agtctggaaa cgcggaagtc agcgccctgc 3300
accattatgt tccggatctg catcgcagga tgctgctggc taccctgtgg aacacctaca 3360

tctgtattaa cgaagcgctg gcattgaccc tgagtgattt ttctctggtc ccgccgcatc 3420

cataccgcca gttgtttacc ctcacaacgt tccagtaacc gggcatgttc atcatcagta 3480

acccgtatcg tgagcatcct ctctcgtttc atcggtatca ttacccccat gaacagaaat 3540

cccccttaca cggaggcatc agtgaccaaa caggaaaaaa ccgcccttaa catggcccgc 3600

tttatcagaa gccagacatt aacgcttctg gagaaactca acgagctgga cgcggatgaa 3660

caggcagaca tctgtgaatc gcttcacgac cacgctgatg agctttaccg cagctgcctc 3720

gcgcgtttcg gtgatgacgg tgaaaacctc tgacacatgc agctcccgga gacggtcaca 3780

gcttgtctgt aagcggatgc cgggagcaga caagcccgtc agggcgcgtc agcgggtgtt 3840

ggcgggtgtc ggggcgcagc catgacccag tcacgtagcg atagcggagt gtatactggc 3900

ttaactatgc ggcatcagag cagattgtac tgagagtgca ccatatatgc ggtgtgaaat 3960

accgcacaga tgcgtaagga gaaaataccg catcaggcgc tcttccgctt cctcgctcac 4020

tgactcgctg cgctcggtcg ttcggctgcg gcgagcggta tcagctcact caaaggcggt 4080

aatacggtta tccacagaat caggggataa cgcaggaaag aacatgtgag caaaaggcca 4140

gcaaaaggcc aggaaccgta aaaaggccgc gttgctggcg tttttccata ggctccgccc 4200

ccctgacgag catcacaaaa atcgacgctc aagtcagagg tggcgaaacc cgacaggact 4260

ataaagatac caggcgtttc cccctggaag ctccctcgtg cgctctcctg ttccgaccct 4320

gccgcttacc ggatacctgt ccgcctttct cccttcggga agcgtggcgc tttctcatag 4380

ctcacgctgt aggtatctca gttcggtgta ggtcgttcgc tccaagctgg gctgtgtgca 4440

cgaacccccc gttcagcccg accgctgcgc cttatccggt aactatcgtc ttgagtccaa 4500

cccggtaaga cacgacttat cgccactggc agcagccact ggtaacagga ttagcagagc 4560

gaggtatgta ggcggtgcta cagagttctt gaagtggtgg cctaactacg gctacactag 4620

aaggacagta tttggtatct gcgctctgct gaagccagtt accttcggaa aaagagttgg 4680

tagctcttga tccggcaaac aaaccaccgc tggtagcggt ggtttttttg tttgcaagca 4740

gcagattacg cgcagaaaaa aaggatctca agaagatcct ttgatctttt ctacggggtc 4800

tgacgctcag tggaacgaaa actcacgtta agggattttg gtcatgaaca ataaaactgt 4860

ctgcttacat aaacagtaat acaaggggtg ttatgagcca tattcaacgg gaaacgtctt 4920

gctctaggcc gcgattaaat tccaacatgg atgctgattt atatgggtat aaatgggctc 4980

gcgataatgt cgggcaatca ggtgcgacaa tctatcgatt gtatgggaag cccgatgcgc 5040

cagagttgtt tctgaaacat ggcaaaggta gcgttgccaa tgatgttaca gatgagatgg 5100

tcagactaaa ctggctgacg gaatttatgc ctcttccgac catcaagcat tttatccgta 5160

ctcctgatga tgcatggtta ctcaccactg cgatccccgg gaaaacagca ttccaggtat 5220

tagaagaata tcctgattca ggtgaaaata ttgttgatgc gctggcagtg ttcctgcgcc 5280

ggttgcattc gattcctgtt tgtaattgtc cttttaacag cgatcgcgta tttcgtctcg 5340

ctcaggcgca atcacgaatg aataacggtt tggttgatgc gagtgatttt gatgacgagc 5400

gtaatggctg gcctgttgaa caagtctgga aagaaatgca taaacttttg ccattctcac 5460

cggattcagt cgtcactcat ggtgatttct cacttgataa ccttattttt gacgagggga 5520

aattaatagg ttgtattgat gttggacgag tcggaatcgc agaccgatac caggatcttg 5580

ccatcctatg gaactgcctc ggtgagtttt ctccttcatt acagaaacgg ctttttcaaa 5640

aatatggtat tgataatcct gatatgaata aattgcagtt tcatttgatg ctcgatgagt 5700

ttttctaaga attaattcat gagcggatac atatttgaat gtatttagaa aaataaacaa 5760

ataggggttc cgcgcacatt tccccgaaaa gtgccacctg aaattgtaaa cgttaatatt 5820

ttgttaaaat tcgcgttaaa tttttgttaa atcagctcat tttttaacca ataggccgaa 5880

atcggcaaaa tcccttataa atcaaaagaa tagaccgaga tagggttgag tgttgttcca 5940

gtttggaaca agagtccact attaaagaac gtggactcca acgtcaaagg gcgaaaaacc 6000

gtctatcagg gcgatggccc actacgtgaa ccatcaccct aatcaagttt tttggggtcg 6060

aggtgccgta aagcactaaa tcggaaccct aaagggagcc cccgatttag agcttgacgg 6120

ggaaagccgg cgaacgtggc gagaaaggaa gggaagaaag cgaaaggagc gggcgctagg 6180

gcgctggcaa gtgtagcggt cacgctgcgc gtaaccacca cacccgccgc gcttaatgcg 6240

ccgctacagg gcgcgtccca ttcgccatcc ggatatagtt cctcctttca gcaaaaaacc 6300

cctcaagacc cgtttagagg ccccaagggg ttatgctagt tattgctcag cggtggcagc 6360

agccaactca gcttcctttc gggctttgtt agcagccgga tctcagtggt ggtggtggtg 6420

gtgctcgagt gc 6432

<210> 16
<211> 6418
<212> DNA
<213> Artificial Sequence

<220>
<223> pET-28b{Ptac}[hGLYAT3(co_Ec)]

<220>
<221> misc_feature
<223> pET-28b{Ptac}[hGLYAT3(co_Ec)]

<400> 16
ggccgccctt cattttggat ccaattgtga gcggataaca attacgagct tcatgcacag 60

tgatcgacgc tgttgacaat taatcatcgg ctcgtataat gtgtggatgt ggaattgtga 120

gcgctcacaa ttccacaacg gtttccctct agaaataatt ttgtttaaca ggaggtaaaa 180

catatgctgg ttctgaattg ctctactaaa ctgctgatcc tggagaaaat gctgaaatcc 240
tgtttcccgg aatctctgaa agtttatggt gcggtaatga atatcaaccg tggtaatccg 300
tttcaaaaag aagttgttct ggactcttgg ccagatttca aggcggttat cacccgtcgt 360
cagcgtgaag cggaaaccga caacctggac cactatacca acgcgtatgc ggttttctat 420
aaagatgttc gcgcgtaccg tcagctgctg gaagaatgcg atgttttcaa ctgggaccag 480
gtttttcaga tccaaggtct gcaatccgag ctgtacgacg tttctaaggc cgtggcgaac 540
tctaaacaac tgaacatcaa gctgacctct ttcaaagcgg ttcatttctc tccggtatcc 600
agcctgccgg acacctcctt cctgaaaggt ccgtctccgc gtctgaccta cctgtctgtt 660
gcgaatgcgg atctgctgaa ccgtacttgg tcccgtggtg gcaacgaaca gtgcctgcgt 720
tacattgcta acctgatctc ttgcttcccg agcgtctgcg ttcgtgatga aaaaggtaac 780
ccagtatctt ggtctatcac cgaccagttc gcgaccatgt gtcatggcta caccctgcct 840
gaacaccgtc gtaagggtta ctctcgtctg gttgcgctga ccctggcgcg taagctgcag 900
tctcgtggtt ttccgtccca gggtaacgtc ctggacgaca acactgcgtc catctccctg 960
ctgaagtccc tgcatgccga attcctgccg tgccgtttcc accgtctgat tctgacccca 1020
gcgaccttct ctggtctgcc gcacctgtaa gcgttaagtc tgagaggtga gagctcgaat 1080
tcggatcccg acccatttgc tgtccaccag tcatgctagc catatggctg ccgcgcggca 1140
ccaggccgct gctgtgatga tgatgatgat ggctgctgcc catggtatat ctccttctta 1200
aagttaaaca aaattatttc tagaggggaa ttgttatccg ctcacaattc ccctatagtg 1260
agtcgtatta atttcgcggg atcgagatct cgatcctcta cgccggacgc atcgtggccg 1320
gcatcaccgg cgccacaggt gcggttgctg gcgcctatat cgccgacatc accgatgggg 1380
aagatcgggc tcgccacttc gggctcatga gcgcttgttt cggcgtgggt atggtggcag 1440
gccccgtggc cgggggactg ttgggcgcca tctccttgca tgcaccattc cttgcggcgg 1500
cggtgctcaa cggcctcaac ctactactgg gctgcttcct aatgcaggag tcgcataagg 1560
gagagcgtcg agatcccgga caccatcgaa tggcgcaaaa cctttcgcgg tatggcatga 1620
tagcgcccgg aagagagtca attcagggtg gtgaatgtga aaccagtaac gttatacgat 1680
gtcgcagagt atgccggtgt ctcttatcag accgtttccc gcgtggtgaa ccaggccagc 1740
cacgtttctg cgaaaacgcg ggaaaaagtg gaagcggcga tggcggagct gaattacatt 1800
cccaaccgcg tggcacaaca actggcgggc aaacagtcgt tgctgattgg cgttgccacc 1860
tccagtctgg ccctgcacgc gccgtcgcaa attgtcgcgg cgattaaatc tcgcgccgat 1920
caactgggtg ccagcgtggt ggtgtcgatg gtagaacgaa gcggcgtcga agcctgtaaa 1980
gcggcggtgc acaatcttct cgcgcaacgc gtcagtgggc tgatcattaa ctatccgctg 2040
gatgaccagg atgccattgc tgtggaagct gcctgcacta atgttccggc gttatttctt 2100

gatgtctctg accagacacc catcaacagt attattttct cccatgaaga cggtacgcga 2160
ctgggcgtgg agcatctggt cgcattgggt caccagcaaa tcgcgctgtt agcgggccca 2220
ttaagttctg tctcggcgcg tctgcgtctg gctggctggc ataaatatct cactcgcaat 2280
caaattcagc cgatagcgga acgggaaggc gactggagtg ccatgtccgg ttttcaacaa 2340
accatgcaaa tgctgaatga gggcatcgtt cccactgcga tgctggttgc caacgatcag 2400
atggcgctgg gcgcaatgcg cgccattacc gagtccgggc tgcgcgttgg tgcggatatc 2460
tcggtagtgg gatacgacga taccgaagac agctcatgtt atatcccgcc gttaaccacc 2520
atcaaacagg attttcgcct gctggggcaa accagcgtgg accgcttgct gcaactctct 2580
cagggccagg cggtgaaggg caatcagctg ttgcccgtct cactggtgaa aagaaaaacc 2640
accctggcgc ccaatacgca aaccgcctct ccccgcgcgt tggccgattc attaatgcag 2700
ctggcacgac aggtttcccg actggaaagc gggcagtgag cgcaacgcaa ttaatgtaag 2760
ttagctcact cattaggcac cgggatctcg accgatgccc ttgagagcct tcaacccagt 2820
cagctccttc cggtgggcgc ggggcatgac tatcgtcgcc gcacttatga ctgtcttctt 2880
tatcatgcaa ctcgtaggac aggtgccggc agcgctctgg gtcattttcg gcgaggaccg 2940
ctttcgctgg agcgcgacga tgatcggcct gtcgcttgcg gtattcggaa tcttgcacgc 3000
cctcgctcaa gccttcgtca ctggtcccgc caccaaacgt ttcggcgaga agcaggccat 3060
tatcgccggc atggcggccc cacgggtgcg catgatcgtg ctcctgtcgt tgaggacccg 3120
gctaggctgg cggggttgcc ttactggtta gcagaatgaa tcaccgatac gcgagcgaac 3180
gtgaagcgac tgctgctgca aaacgtctgc gacctgagca acaacatgaa tggtcttcgg 3240
tttccgtgtt tcgtaaagtc tggaaacgcg gaagtcagcg ccctgcacca ttatgttccg 3300
gatctgcatc gcaggatgct gctggctacc ctgtggaaca cctacatctg tattaacgaa 3360
gcgctggcat tgaccctgag tgatttttct ctggtcccgc cgcatccata ccgccagttg 3420
tttaccctca caacgttcca gtaaccgggc atgttcatca tcagtaaccc gtatcgtgag 3480
catcctctct cgtttcatcg gtatcattac ccccatgaac agaaatcccc cttacacgga 3540
ggcatcagtg accaaacagg aaaaaaccgc ccttaacatg gcccgcttta tcagaagcca 3600
gacattaacg cttctggaga aactcaacga gctggacgcg gatgaacagg cagacatctg 3660
tgaatcgctt cacgaccacg ctgatgagct ttaccgcagc tgcctcgcgc gtttcggtga 3720
tgacggtgaa aacctctgac acatgcagct cccggagacg gtcacagctt gtctgtaagc 3780
ggatgccggg agcagacaag cccgtcaggg cgcgtcagcg ggtgttggcg ggtgtcgggg 3840
cgcagccatg acccagtcac gtagcgatag cggagtgtat actggcttaa ctatgcggca 3900
tcagagcaga ttgtactgag agtgcaccat atatgcggtg tgaaataccg cacagatgcg 3960

taaggagaaa ataccgcatc aggcgctctt ccgcttcctc gctcactgac tcgctgcgct 4020
cggtcgttcg gctgcggcga gcggtatcag ctcactcaaa ggcggtaata cggttatcca 4080
cagaatcagg ggataacgca ggaaagaaca tgtgagcaaa aggccagcaa aaggccagga 4140
accgtaaaaa ggccgcgttg ctggcgtttt tccataggct ccgcccccct gacgagcatc 4200
acaaaaatcg acgctcaagt cagaggtggc gaaacccgac aggactataa agataccagg 4260
cgtttccccc tggaagctcc ctcgtgcgct ctcctgttcc gaccctgccg cttaccggat 4320
acctgtccgc ctttctccct tcgggaagcg tggcgctttc tcatagctca cgctgtaggt 4380
atctcagttc ggtgtaggtc gttcgctcca agctgggctg tgtgcacgaa ccccccgttc 4440
agcccgaccg ctgcgcctta tccggtaact atcgtcttga gtccaacccg gtaagacacg 4500
acttatcgcc actggcagca gccactggta acaggattag cagagcgagg tatgtaggcg 4560
gtgctacaga gttcttgaag tggtggccta actacggcta cactagaagg acagtatttg 4620
gtatctgcgc tctgctgaag ccagttacct tcggaaaaag agttggtagc tcttgatccg 4680
gcaaacaaac caccgctggt agcggtggtt tttttgtttg caagcagcag attacgcgca 4740
gaaaaaaagg atctcaagaa gatcctttga tcttttctac ggggtctgac gctcagtgga 4800
acgaaaactc acgttaaggg attttggtca tgaacaataa aactgtctgc ttacataaac 4860
agtaatacaa ggggtgttat gagccatatt caacgggaaa cgtcttgctc taggccgcga 4920
ttaaattcca acatggatgc tgatttatat gggtataaat gggctcgcga taatgtcggg 4980
caatcaggtg cgacaatcta tcgattgtat gggaagcccg atgcgccaga gttgtttctg 5040
aaacatggca aaggtagcgt tgccaatgat gttacagatg agatggtcag actaaactgg 5100
ctgacggaat ttatgcctct tccgaccatc aagcatttta tccgtactcc tgatgatgca 5160
tggttactca ccactgcgat ccccgggaaa acagcattcc aggtattaga agaatatcct 5220
gattcaggtg aaaatattgt tgatgcgctg gcagtgttcc tgcgccggtt gcattcgatt 5280
cctgtttgta attgtccttt taacagcgat cgcgtatttc gtctcgctca ggcgcaatca 5340
cgaatgaata acggtttggt tgatgcgagt gattttgatg acgagcgtaa tggctggcct 5400
gttgaacaag tctggaaaga aatgcataaa cttttgccat tctcaccgga ttcagtcgtc 5460
actcatggtg atttctcact tgataacctt atttttgacg aggggaaatt aataggttgt 5520
attgatgttg gacgagtcgg aatcgcagac cgataccagg atcttgccat cctatggaac 5580
tgcctcggtg agttttctcc ttcattacag aaacggcttt ttcaaaaata tggtattgat 5640
aatcctgata tgaataaatt gcagtttcat ttgatgctcg atgagttttt ctaagaatta 5700
attcatgagc ggatacatat ttgaatgtat ttagaaaaat aaacaaatag gggttccgcg 5760
cacatttccc cgaaaagtgc cacctgaaat tgtaaacgtt aatattttgt taaaattcgc 5820
gttaaatttt tgttaaatca gctcattttt taaccaatag gccgaaatcg gcaaaatccc 5880

ttataaatca aaagaataga ccgagatagg gttgagtgtt gttccagttt ggaacaagag 5940

tccactatta aagaacgtgg actccaacgt caaagggcga aaaaccgtct atcagggcga 6000

tggcccacta cgtgaaccat caccctaatc aagttttttg gggtcgaggt gccgtaaagc 6060

actaaatcgg aaccctaaag ggagcccccg atttagagct tgacggggaa agccggcgaa 6120

cgtggcgaga aaggaaggga agaaagcgaa aggagcgggc gctagggcgc tggcaagtgt 6180

agcggtcacg ctgcgcgtaa ccaccacacc cgccgcgctt aatgcgccgc tacagggcgc 6240

gtcccattcg ccatccggat atagttcctc ctttcagcaa aaaacccctc aagacccgtt 6300

tagaggcccc aaggggttat gctagttatt gctcagcggt ggcagcagcc aactcagctt 6360

cctttcgggc tttgttagca gccggatctc agtggtggtg gtggtggtgc tcgagtgc 6418

<210> 17
<211> 4334
<212> DNA
<213> Escherichia coli

<220>
<221> misc_feature
<223> pBMT-3_ccdAB

<400> 17
ctgatggaca ggctgcgcct gcccacgagc ttgaccacag ggattgccca ccggctaccc 60

agccttcgac cacataccca ccggctccaa ctgcgcggcc tgcggccttg ccccatcaat 120

ttttttaatt ttctctgggg aaaagcctcc ggcctgcggc ctgcgcgctt cgcttgccgg 180

ttggacacca agtggaaggc gggtcaaggc tcgcgcagcg accgcgcagc ggcttggcct 240

tgacgcgcct ggaacgaccc aagcctatgc gagtgggggc agtcgaaggc gaagcccgcc 300

cgcctgcccc ccgagcctca cggcggcgag tgcgggggtt ccaagggggc agcgccacct 360

tgggcaaggc cgaaggccgc gcagtcgatc aacaagcccc ggaggggcca ctttttgccg 420

gagggggagc cgcgccgaag gcgtggggga accccgcagg ggtgcccttc tttgggcacc 480

aaagaactag atatagggcg aaatgcgaaa gacttaaaaa tcaacaactt aaaaaagggg 540

ggtacgcaac agctcattgc ggcacccccc gcaatagctc attgcgtagg ttaaagaaaa 600

tctgtaattg actgccactt ttacgcaacg cataattgtt gtcgcgctgc cgaaaagttg 660

cagctgattg cgcatggtgc cgcaaccgtg cggcacccta ccgcatggag ataagcatgg 720

ccacgcagtc cagagaaatc ggcattcaag ccaagaacaa gcccggtcac tgggtgcaaa 780

cggaacgcaa agcgcatgag gcgtgggccg ggcttattgc gaggaaaccc acggcggcaa 840

tgctgctgca tcacctcgtg gcgcagatgg gccaccagaa cgccgtggtg gtcagccaga 900

agacactttc caagctcatc ggacgttctt tgcggacggt ccaatacgca gtcaaggact 960

```
tggtggccga gcgctggatc tccgtcgtga agctcaacgg ccccggcacc gtgtcggcct     1020
acgtggtcaa tgaccgcgtg gcgtggggcc agccccgcga ccagttgcgc ctgtcggtgt     1080
tcagtgccgc cgtggtggtt gatcacgacg accaggacga atcgctgttg gggcatggcg     1140
acctgcgccg catcccgacc ctgtatccgg gcgagcagca actaccgacc ggccccggcg     1200
aggagccgcc cagccagccc ggcattccgg gcatggaacc agacctgcca gccttgaccg     1260
aaacggagga atgggaacgg cgcgggcagc agcgcctgcc gatgcccgat gagccgtgtt     1320
ttctggacga tggcgagccg ttggagccgc cgacacgggt cacgctgccg cgccggtagt     1380
acgtacccgg aattgccagc tggggcgccc tctggtaagg ttgggaagcc ctgcaaagta     1440
aactggatgg ctttcttgcc gccaaggatc tgatggcgca ggggatcaag ctctgatcaa     1500
gagacaggat gaggatcgtt tcgatgaagc agcgtattac agtgacagtt gacagcgaca     1560
gctatcagtt gctcaaggca tatgatgtca atatctccgg tctggtaagc acaaccatgc     1620
agaatgaagc ccgtcgtctg cgtgccgaac gctggaaagc ggaaaatcag gaagggatgg     1680
ctgaggtcgc ccggtttatt gaaatgaacg gctcttttgc tgacgagaac agggactggt     1740
gaaatgcagt ttaaggttta cacctataaa agagagagcc gttatcgtct gtttgtggat     1800
gtacagagcg atattattga cacgcccggg cgacggatgg tgatcccccct ggccagtgca     1860
cgtctgctgt cagataaagt ctcccgtgaa ctttacccgg tggtgcatat cggggatgaa     1920
agctggcgca tgatgaccac cgatatggcc agtgtgccgg tctccgttat cggggaagaa     1980
gtggctgatc tcagccaccg cgaaaatgac atcaaaaacg ccattaacct gatgttctgg     2040
ggaatataaa tcctagacga attctctagt agaggttcca actttcacca taatgaaata     2100
agatcactac cgggcgtatt ttttgagtta tcgagatttt caggagctaa ggaagctaaa     2160
atggagaaaa aaatcactgg atataccacc gttgatatat cccaatggca tcgtaaagaa     2220
cattttgagg catttcagtc agttgctcaa tgtacctata accagaccgt tcagctggat     2280
attacggcct ttttaaagac cgtaaagaaa aataagcaca agttttatcc ggcctttatt     2340
cacattcttg cccgcctgat gaatgctcat ccggaattcc gtatggcaat gaaagacggt     2400
gagctggtga tatgggatag tgttcaccct tgttacaccg ttttccatga gcaaactgaa     2460
acgttttcat cgctctggag tgaataccac gacgatttcc ggcagtttct acacatatat     2520
tcgcaagatg tggcgtgtta cggtgaaaac ctggcctatt tccctaaagg gtttattgag     2580
aatatgtttt tcgtctcagc caatccctgg gtgagtttca ccagttttga tttaaacgtg     2640
gccaatatgg acaacttctt cgcccccgtt ttcaccatgg gcaaatatta tacgcaaggc     2700
gacaaggtgc tgatgccgct ggcgattcag gttcatcatg ccgtttgtga tggcttccat     2760
gtcggcagaa tgcttaatga attacaacag tactgcgatg agtggcaggg cggggcgtaa     2820
agatctggat cccccctcaag tcaaaagcct ccggtcggag gcttttgact ttctgctatg     2880
```

gaggtcaggt atgatttaaa tggtcagtat tgagcgatat ctagagaatt cgtcaacgaa 2940

ttcaagcttg atatcattca ggacgagcct cagactccag cgtaactgga ctgaaaacaa 3000

actaaagcgc ccttgtggcg ctttagtttt gttccgctca tgataataat ggtttcttag 3060

acgtcaggtg gcacttttcg gggaaatgtg cgcgcccgcg ttcctgctgg cgctgggcct 3120

gtttctggcg ctggacttcc cgctgttccg tcagcagctt ttcgcccacg gccttgatga 3180

tcgcggcggc cttggcctgc atatcccgat tcaacggccc cagggcgtcc agaacgggct 3240

tcaggcgctc ccgaaggtct cgggccgtct cttgggcttg atcggccttc ttgcgcatct 3300

cacgcgctcc tgcggcggcc tgtagggcag gctcataccc ctgccgaacc gcttttgtca 3360

gccggtcggc cacggcttcc ggcgtctcaa cgcgctttga gattcccagc ttttcggcca 3420

atccctgcgg tgcataggcg cgtggctcga ccgcttgcgg gctgatggtg acgtggccca 3480

ctggtggccg ctccagggcc tcgtagaacg cctgaatgcg cgtgtgacgt gccttgctgc 3540

cctcgatgcc ccgttgcagc cctagatcgg ccacagcggc cgcaaacgtg gtctggtcgc 3600

gggtcatctg cgctttgttg ccgatgaact ccttggccga cagcctgccg tcctgcgtca 3660

gcggcaccac gaacgcggtc atgtgcgggc tggtttcgtc acggtggatg ctggccgtca 3720

cgatgcgatc cgccccgtac ttgtccgcca gccacttgtg cgccttctcg aagaacgccg 3780

cctgctgttc ttggctggcc gacttccacc attccgggct ggccgtcatg acgtactcga 3840

ccgccaacac agcgtccttg cgccgcttct ctggcagcaa ctcgcgcagt cggcccatcg 3900

cttcatcggt gctgctggcc gcccagtgct cgttctctgg cgtcctgctg gcgtcagcgt 3960

tgggcgtctc gcgctcgcgg taggcgtgct tgagactggc cgccacgttg cccattttcg 4020

ccagcttctt gcatcgcatg atcgcgtatg ccgccatgcc tgcccctccc ttttggtgtc 4080

caaccggctc gacgggggca gcgcaaggcg gtgcctccgg cgggccactc aatgcttgag 4140

tatactcact agactttgct tcgcaaagtc gtgaccgcct acggcggctg cggcgcccta 4200

cgggcttgct ctccgggctt cgccctgcgc ggtcgctgcg ctcccttgcc agcccgtgga 4260

tatgtggacg atggccgcga gcggccaccg gctggctcgc ttcgctcggc ccgtggacaa 4320

ccctgctgga caag 4334

<210> 18
<211> 5024
<212> DNA
<213> Escherichia coli

<220>
<221> misc_feature
<223> pBMT-3_ccdAB_PT7-'tesA

<400> 18

caaaaaaccc ctcaagaccc gtttagaggc cccaaggggt tatgctagtt attgctcagc 60
ggtggcagca gcctaggtta attaagctgc gctagtagac gagtccatgt gctggcgttc 120
aaatttcgca gcagcggttt ctttaccaga ctcgaaaatt caggagtcgt ggttgaccag 180
cggctgcagt tgcttggcca tccagtcggc gatgaacggc tgcgcgtcgc ggttcgggtg 240
gatgccgtcg tcctgcatcc actgcggctt caggtagact tcctccatga agaacggcag 300
cagcggcacg tcgaattcct tggccagctt cgggtagatc gcggagaagg cctcgttgta 360
gcgacggccg tagttcgccg gcaggcggat ctgcatcagc agcggctcgg cgttcgcggc 420
cttcacgtcc tgcaggatct ggcgcagcgt ctgttcggtc tgctgcggct ggaagccgcg 480
caggccgtcg ttgccgccca gctcgaccag cacccagcgc ggctggtgct gcttcagcag 540
cgccggcagg cgggccaggc cctgctggct cgtgtcgccg ctgatcgagg cgttgaccac 600
cgaggtcttg gactgccact tgtcgttcag cagcgccggc cacgccgccg acgccgacat 660
gcggtagccc gccgacaggg agtcgcccag gatcagcagg gtgtccgccg ccatggtata 720
tctccttatt aaagttaaac aaaattattt ctacagggga attgttatcc gctcacaatt 780
cccctatagt gagtcgtatt aatttcctaa tgcaggagtc gatcattcag gacgagcctc 840
agactccagc gtaactggac tgaaaacaaa ctaaagcgcc cttgtggcgc tttagttttg 900
ttccgctcat gataataatg gtttcttaga cgtcaggtgg cacttttcgg ggaaatgtgc 960
gcgcccgcgt tcctgctggc gctgggcctg tttctggcgc tggacttccc gctgttccgt 1020
cagcagcttt tcgcccacgg ccttgatgat cgcggcggcc ttggcctgca tatcccgatt 1080
caacggcccc agggcgtcca gaacgggctt caggcgctcc cgaaggtctc gggccgtctc 1140
ttgggcttga tcggccttct tgcgcatctc acgcgctcct gcggcggcct gtagggcagg 1200
ctcatacccc tgccgaaccg cttttgtcag ccggtcggcc acggcttccg gcgtctcaac 1260
gcgctttgag attcccagct tttcggccaa tccctgcggt gcataggcgc gtggctcgac 1320
cgcttgcggg ctgatggtga cgtggcccac tggtggccgc tccagggcct cgtagaacgc 1380
ctgaatgcgc gtgtgacgtg ccttgctgcc ctcgatgccc cgttgcagcc ctagatcggc 1440
cacagcggcc gcaaacgtgg tctggtcgcg ggtcatctgc gctttgttgc cgatgaactc 1500
cttggccgac agcctgccgt cctgcgtcag cggcaccacg aacgcggtca tgtgcgggct 1560
ggtttcgtca cggtggatgc tggccgtcac gatgcgatcc gccccgtact tgtccgccag 1620
ccacttgtgc gccttctcga agaacgccgc ctgctgttct tggctggccg acttccacca 1680
ttccgggctg gccgtcatga cgtactcgac cgccaacaca gcgtccttgc gccgcttctc 1740
tggcagcaac tcgcgcagtc ggcccatcgc ttcatcggtg ctgctggccg cccagtgctc 1800
gttctctggc gtcctgctgg cgtcagcgtt gggcgtctcg cgctcgcggt aggcgtgctt 1860
gagactggcc gccacgttgc ccattttcgc cagcttcttg catcgcatga tcgcgtatgc 1920

cgccatgcct gcccctccct tttggtgtcc aaccggctcg acgggggcag cgcaaggcgg 1980
tgcctccggc gggccactca atgcttgagt atactcacta gactttgctt cgcaaagtcg 2040
tgaccgccta cggcggctgc ggcgccctac gggcttgctc tccgggcttc gccctgcgcg 2100
gtcgctgcgc tcccttgcca gcccgtggat atgtggacga tggccgcgag cggccaccgg 2160
ctggctcgct tcgctcggcc cgtggacaac cctgctggac aagctgatgg acaggctgcg 2220
cctgcccacg agcttgacca cagggattgc ccaccggcta cccagccttc gaccacatac 2280
ccaccggctc caactgcgcg gcctgcggcc ttgccccatc aattttttta attttctctg 2340
gggaaaagcc tccggcctgc ggcctgcgcg cttcgcttgc cggttggaca ccaagtggaa 2400
ggcgggtcaa ggctcgcgca gcgaccgcgc agcggcttgg ccttgacgcg cctggaacga 2460
cccaagccta tgcgagtggg ggcagtcgaa ggcgaagccc gcccgcctgc cccccgagcc 2520
tcacggcggc gagtgcgggg gttccaaggg ggcagcgcca ccttgggcaa ggccgaaggc 2580
cgcgcagtcg atcaacaagc cccggagggg ccactttttg ccggaggggg agccgcgccg 2640
aaggcgtggg ggaaccccgc aggggtgccc ttctttgggc accaaagaac tagatatagg 2700
gcgaaatgcg aaagacttaa aaatcaacaa cttaaaaaag gggggtacgc aacagctcat 2760
tgcggcaccc cccgcaatag ctcattgcgt aggttaaaga aaatctgtaa ttgactgcca 2820
cttttacgca acgcataatt gttgtcgcgc tgccgaaaag ttgcagctga ttgcgcatgg 2880
tgccgcaacc gtgcggcacc ctaccgcatg gagataagca tggccacgca gtccagagaa 2940
atcggcattc aagccaagaa caagcccggt cactgggtgc aaacggaacg caaagcgcat 3000
gaggcgtggg ccgggcttat tgcgaggaaa cccacggcgg caatgctgct gcatcacctc 3060
gtggcgcaga tgggccacca gaacgccgtg gtggtcagcc agaagacact ttccaagctc 3120
atcggacgtt ctttgcggac ggtccaatac gcagtcaagg acttggtggc cgagcgctgg 3180
atctccgtcg tgaagctcaa cggccccggc accgtgtcgg cctacgtggt caatgaccgc 3240
gtggcgtggg gccagccccg cgaccagttg cgcctgtcgg tgttcagtgc cgccgtggtg 3300
gttgatcacg acgaccagga cgaatcgctg ttggggcatg gcgacctgcg ccgcatcccg 3360
accctgtatc cgggcgagca gcaactaccg accggccccg gcgaggagcc gcccagccag 3420
cccggcattc cgggcatgga accagacctg ccagccttga ccgaaacgga ggaatgggaa 3480
cggcgcgggc agcagcgcct gccgatgccc gatgagccgt gttttctgga cgatggcgag 3540
ccgttggagc cgccgacacg ggtcacgctg ccgcgccggt agtacgtacc cggaattgcc 3600
agctggggcg ccctctggta aggttgggaa gccctgcaaa gtaaactgga tggctttctt 3660
gccgccaagg atctgatggc gcaggggatc aagctctgat caagagacag gatgaggatc 3720
gtttcgatga agcagcgtat tacagtgaca gttgacagcg acagctatca gttgctcaag 3780

gcatatgatg tcaatatctc cggtctggta agcacaacca tgcagaatga agcccgtcgt 3840

ctgcgtgccg aacgctggaa agcggaaaat caggaaggga tggctgaggt cgccccggttt 3900

attgaaatga acggctcttt tgctgacgag aacagggact ggtgaaatgc agtttaaggt 3960

ttacacctat aaaagagaga gccgttatcg tctgtttgtg gatgtacaga gcgatattat 4020

tgacacgccc gggcgacgga tggtgatccc cctggccagt gcacgtctgc tgtcagataa 4080

agtctcccgt gaactttacc cggtggtgca tatcggggat gaaagctggc gcatgatgac 4140

caccgatatg gccagtgtgc cggtctccgt tatcggggaa gaagtggctg atctcagcca 4200

ccgcgaaaat gacatcaaaa acgccattaa cctgatgttc tggggaatat aaatcctaga 4260

cgaattctct agtagaggtt ccaactttca ccataatgaa ataagatcac taccgggcgt 4320

atttttgag ttatcgagat tttcaggagc taaggaagct aaaatggaga aaaaaatcac 4380

tggatatacc accgttgata tatcccaatg gcatcgtaaa gaacattttg aggcatttca 4440

gtcagttgct caatgtacct ataaccagac cgttcagctg gatattacgg cctttttaaa 4500

gaccgtaaag aaaaataagc acaagtttta tccggccttt attcacattc ttgcccgcct 4560

gatgaatgct catccggaat tccgtatggc aatgaaagac ggtgagctgg tgatatggga 4620

tagtgttcac ccttgttaca ccgttttcca tgagcaaact gaaacgtttt catcgctctg 4680

gagtgaatac cacgacgatt tccggcagtt tctacacata tattcgcaag atgtggcgtg 4740

ttacggtgaa aacctggcct atttccctaa agggtttatt gagaatatgt ttttcgtctc 4800

agccaatccc tgggtgagtt tcaccagttt tgatttaaac gtggccaata tggacaactt 4860

cttcgccccc gttttcacca tgggcaaata ttatacgcaa ggcgacaagg tgctgatgcc 4920

gctggcgatt caggttcatc atgccgtttg tgatggcttc catgtcggca gaatgcttaa 4980

tgaattacaa cagtactgcg atgagtggca gggcggggcg taaa 5024

<210> 19
<211> 8268
<212> DNA
<213> Escherichia coli

<220>
<221> misc_feature
<223> pBMT-3_ccdAB_PT7-nphT7-hbd-crt-ter

<400> 19
ccttgccagc ccgtggatat gtggacgatg gccgcgagcg gccaccggct ggctcgcttc 60

gctcggcccg tggacaaccc tgctggacaa gctgatggac aggctgcgcc tgcccacgag 120

cttgaccaca gggattgccc accggctacc cagccttcga ccacataccc accggctcca 180

actgcgcggc ctgcggcctt gccccatcaa ttttttttaat tttctctggg gaaaagcctc 240

cggcctgcgg cctgcgcgct tcgcttgccg gttggacacc aagtggaagg cgggtcaagg 300

ctcgcgcagc gaccgcgcag cggcttggcc ttgacgcgcc tggaacgacc caagcctatg 360
cgagtggggg cagtcgaagg cgaagcccgc ccgcctgccc cccgagcctc acggcggcga 420
gtgcgggggt tccaaggggg cagcgccacc ttgggcaagg ccgaaggccg cgcagtcgat 480
caacaagccc cggaggggcc actttttgcc ggaggggag ccgcgccgaa ggcgtggggg 540
aaccccgcag gggtgccctt ctttgggcac caaagaacta gatatagggc gaaatgcgaa 600
agacttaaaa atcaacaact taaaaaaggg gggtacgcaa cagctcattg cggcaccccc 660
cgcaatagct cattgcgtag gttaaagaaa atctgtaatt gactgccact tttacgcaac 720
gcataattgt tgtcgcgctg ccgaaaagtt gcagctgatt gcgcatggtg ccgcaaccgt 780
gcggcaccct accgcatgga gataagcatg gccacgcagt ccagagaaat cggcattcaa 840
gccaagaaca agcccggtca ctgggtgcaa acggaacgca aagcgcatga ggcgtgggcc 900
gggcttattg cgaggaaacc cacggcggca atgctgctgc atcacctcgt ggcgcagatg 960
ggccaccaga acgccgtggt ggtcagccag aagacacttt ccaagctcat cggacgttct 1020
ttgcggacgg tccaatacgc agtcaaggac ttggtggccg agcgctggat ctccgtcgtg 1080
aagctcaacg gccccggcac cgtgtcggcc tacgtggtca atgaccgcgt ggcgtggggc 1140
cagccccgcg accagttgcg cctgtcggtg ttcagtgccg ccgtggtggt tgatcacgac 1200
gaccaggacg aatcgctgtt ggggcatggc gacctgcgcc gcatcccgac cctgtatccg 1260
ggcgagcagc aactaccgac cggccccggc gaggagccgc ccagccagcc cggcattccg 1320
ggcatggaac cagacctgcc agccttgacc gaaacggagg aatgggaacg gcgcgggcag 1380
cagcgcctgc cgatgcccga tgagccgtgt ttctggacg atggcgagcc gttggagccg 1440
ccgacacggg tcacgctgcc gcgccggtag tacgtacccg gaattgccag ctggggcgcc 1500
ctctggtaag gttgggaagc cctgcaaagt aaactggatg gctttcttgc cgccaaggat 1560
ctgatggcgc aggggatcaa gctctgatca agagacagga tgaggatcgt ttcgatgaag 1620
cagcgtatta cagtgacagt tgacagcgac agctatcagt tgctcaaggc atatgatgtc 1680
aatatctccg gtctggtaag cacaaccatg cagaatgaag cccgtcgtct gcgtgccgaa 1740
cgctggaaag cggaaaatca ggaagggatg gctgaggtcg cccggtttat tgaaatgaac 1800
ggctcttttg ctgacgagaa cagggactgg tgaaatgcag tttaaggttt acacctataa 1860
aagagagagc cgttatcgtc tgtttgtgga tgtacagagc gatattattg acacgcccgg 1920
gcgacggatg gtgatccccc tggccagtgc acgtctgctg tcagataaag tctcccgtga 1980
actttacccg gtggtgcata tcggggatga aagctggcgc atgatgacca ccgatatggc 2040
cagtgtgccg gtctccgtta tcggggaaga agtggctgat ctcagccacc gcgaaaatga 2100
catcaaaaac gccattaacc tgatgttctg gggaatataa atcctagacg aattctctag 2160

tagaggttcc aactttcacc ataatgaaat aagatcacta ccgggcgtat tttttgagtt 2220

atcgagattt tcaggagcta aggaagctaa aatggagaaa aaaatcactg gatataccac 2280

cgttgatata tcccaatggc atcgtaaaga acattttgag gcatttcagt cagttgctca 2340

atgtacctat aaccagaccg ttcagctgga tattacggcc tttttaaaga ccgtaaagaa 2400

aaataagcac aagttttatc cggcctttat tcacattctt gcccgcctga tgaatgctca 2460

tccggaattc cgtatggcaa tgaaagacgg tgagctggtg atatgggata gtgttcaccc 2520

ttgttacacc gttttccatg agcaaactga aacgttttca tcgctctgga gtgaatacca 2580

cgacgatttc cggcagtttc tacacatata ttcgcaagat gtggcgtgtt acggtgaaaa 2640

cctggcctat ttccctaaag ggtttattga gaatatgttt ttcgtctcag ccaatccctg 2700

ggtgagtttc accagttttg atttaaacgt ggccaatatg gacaacttct tcgcccccgt 2760

tttcaccatg ggcaaatatt atacgcaagg cgacaaggtg ctgatgccgc tggcgattca 2820

ggttcatcat gccgtttgtg atggcttcca tgtcggcaga atgcttaatg aattacaaca 2880

gtactgcgat gagtggcagg gcggggcgta aaaaatccct tatgcgactc ctgcattagg 2940

aaattaatac gactcactat aggggaattg tgagcggata acaattcccc tgtagaaata 3000

attttgttta actttaataa ggagatatac catgacggat gtccgctttc gtatcattgg 3060

cacgggcgct tacgtcccgg aacgcattgt cagcaacgac gaagtcggcg caccggctgg 3120

tgtggatgac gattggatta cgcgtaaaac cggtatccgc cagcgtcgct gggcggccga 3180

cgatcaagca acgagcgatc tggctaccgc agctggtcgt gcagcactga aagcagctgg 3240

cattacgccg gaacagctga ccgtcatcgc agtggctacc tctacgccgg accgtccgca 3300

gccgccgacc gcggcctatg ttcaacatca cctgggtgca accggcacgg cagcttttga 3360

tgttaacgct gtgtgcagcg gtacggtttt cgcgctgagc tctgttgccg gcaccctggt 3420

ctatcgtggc ggttacgcac tggtcattgg tgctgatctg tactcacgta tcctgaatcc 3480

ggcggaccgc aaaaccgtgg ttctgttcgg cgatggtgcg ggcgcgatgg tgctgggccc 3540

gaccagcacc ggcaccggtc cgattgtgcg tcgcgttgca ctgcatacgt ttggcggtct 3600

gaccgatctg atccgtgttc cggccggcgg ttcccgccag ccgctggaca ccgatggtct 3660

ggacgcaggc ctgcaatatt ttgcgatgga tggccgcgaa gtccgtcgct tcgtgaccga 3720

acatctgccg cagctgatta aaggttttct gcacgaagcg ggcgtggatg cggcggatat 3780

tagccatttc gtgccgcacc aagccaacgg tgtgatgctg gacgaagttt ttggcgaact 3840

gcatctgccg cgtgcaacca tgcaccgtac ggtggaaacc tacggtaata cgggcgcagc 3900

tagtattccg atcacgatgg atgcggccgt tcgtgcaggt tccttccgtc cgggcgaact 3960

ggttctgctg gcgggctttg gcggcggtat ggcggcttcg tttgctctga ttgaatggtg 4020

acctaatgca ggctgcaggc ggatacgagg aggaataaac catgaaaaag gtatgtgtta 4080

taggtgcagg tactatgggt tcaggaattg ctcaggcatt tgcagctaaa ggatttgaag 4140
tagtattaag agatattaaa gatgaatttg ttgatagagg attagatttt atcaataaaa 4200
atctttctaa attagttaaa aaaggaaaga tagaagaagc tactaaagtt gaaatcttaa 4260
ctagaatttc cggaacagtt gaccttaata tggcagctga ttgcgattta gttatagaag 4320
cagctgttga aagaatggat attaaaaagc agatttttgc tgacttagac aatatatgca 4380
agccagaaac aattcttgca tcaaatacat catcactttc aataacagaa gtggcatcag 4440
caactaaaag acctgataag gttataggta tgcatttctt taatccagct cctgttatga 4500
agcttgtaga ggtaataaga ggaatagcta catcacaaga aacttttgat gcagttaaag 4560
agacatctat agcaatagga aaagatcctg tagaagtagc agaagcacca ggatttgttg 4620
taaatagaat attaatacca atgattaatg aagcagttgg tatattagca gaaggaatag 4680
cttcagtaga agacatagat aaagctatga aacttggagc taatcaccca atgggaccat 4740
tagaattagg tgattttata ggtcttgata tatgtcttgc tataatggat gttttatact 4800
cagaaactgg agattctaag tatagaccac atacattact taagaagtat gtaagagcag 4860
gatggcttgg aagaaaatca ggaaaaggtt tctacgatta ttcaaaataa gtttacagga 4920
tctgcaggga ggaggaaatc atggagttga acaacgttat tctggagaaa gaaggcaagg 4980
tggcggttgt caccattaac cgtccaaagg ccctgaacgc tctgaactcg gataccctga 5040
aagagatgga ttacgttatt ggcgagattg agaatgacag cgaagtgctg gctgtgattc 5100
tgaccggtgc gggtgagaag agctttgtcg cgggtgcgga catcagcgag atgaaagaaa 5160
tgaacaccat cgaaggccgt aagttcggta ttctgggcaa caaggtgttt cgtcgtctgg 5220
aactgctgga gaaacctgtc attgctgccg tgaacggttt cgcgctgggc ggtggttgcg 5280
agatcgctat gagctgcgat attcgtatcg catcgtccaa cgcacgcttt ggtcaaccgg 5340
aggtcggtct gggtatcact ccgggtttcg gcggtacgca acgtctgagc cgcctggttg 5400
gcatgggcat ggcgaaacag ttgattttca cggcacagaa cattaaggcg gatgaggcgc 5460
tgcgtattgg tctggtgaat aaggtcgttg agccaagcga actgatgaat accgcgaaag 5520
aaattgcgaa caagatcgtt agcaatgccc cggtggccgt taagctgtcg aaacaggcaa 5580
tcaaccgtgg catgcagtgt gacatcgaca ccgccctggc gtttgagagc gaggcgtttg 5640
gtgagtgctt ctccaccgag gaccaaaagg atgcgatgac cgcgttcatt gagaaacgca 5700
agatcgaggg tttcaagaat cgttaataga ggaggatagg aggttttcat atgattgtga 5760
aaccgatggt ccgtaataat atctgtctga atgctcaccc gcagggctgt aaaaaaggcg 5820
tggaagatca aattgaatat accaaaaaac gtattacggc agaagtgaaa gccggcgcaa 5880
aagctccgaa aaacgtgctg gttctgggtt gcagcaatgg ctatggtctg gcttctcgca 5940

ttaccgcggc ctttggctac ggtgcagcta cgatcggcgt tagtttcgaa aaagcaggtt 6000
ccgaaaccaa atatggcacg ccgggttggt acaacaatct ggcttttgat gaagcggcca 6060
aacgtgaagg cctgtatagt gtcaccattg atggtgacgc gttctccgat gaaattaaag 6120
cacaggtgat cgaagaagcg aagaaaaaag gcattaaatt tgacctgatc gtttacagcc 6180
tggcatctcc ggtccgtacc gatccggaca cgggtatcat gcataaatct gtgctgaaac 6240
cgtttggcaa aaccttcacg ggtaaaaccg ttgatccgtt cacgggcgaa ctgaaagaaa 6300
ttagcgcgga accggccaac gatgaagaag cagctgcgac cgtcaaagtg atgggcggtg 6360
aagactggga acgttggatc aaacagctga gtaaagaagg cctgctggaa gaaggttgca 6420
ttaccctggc gtattcctac atcggcccgg aagcaaccca agctctgtat cgcaaaggca 6480
cgattggtaa agcgaaagaa catctggaag cgaccgccca ccgtctgaac aaagaaaatc 6540
cgtcaatccg cgccttcgtt tcggtcaata aaggtctggt tacccgtgca tcagctgtga 6600
ttccggttat cccgctgtac ctggcatcgc tgtttaaagt catgaaagaa aaaggcaacc 6660
atgaaggttg tattgaacag atcacccgcc tgtatgccga acgtctgtac cgcaaagatg 6720
gtacgattcc ggtggacgaa gaaaatcgta ttcgcatcga tgactgggaa ctggaagaag 6780
atgtccaaaa agccgtgagc gccctgatgg aaaaagttac cggcgaaaac gcggaatctc 6840
tgacggatct ggccggttat cgtcacgact ttctggcgag taatggtttt gatgttgaag 6900
gcattaacta cgaagctgaa gtggaacgct ttgatcgcat ttgatctaga gaattcgtca 6960
acgaattcaa gcttgatatc attcaggacg agcctcagac tccagcgtaa ctggactgaa 7020
aacaaactaa agcgcccttg tggcgcttta gttttgttcc gctcatgata ataatggttt 7080
cttagacgtc aggtggcact tttcggggaa atgtgcgcgc ccgcgttcct gctggcgctg 7140
ggcctgtttc tggcgctgga cttcccgctg ttccgtcagc agcttttcgc ccacggcctt 7200
gatgatcgcg gcggccttgg cctgcatatc ccgattcaac ggccccaggg cgtccagaac 7260
gggcttcagg cgctcccgaa ggtctcgggc cgtctcttgg gcttgatcgg ccttcttgcg 7320
catctcacgc gctcctgcgg cggcctgtag ggcaggctca tacccctgcc gaaccgcttt 7380
tgtcagccgg tcggccacgg cttccggcgt ctcaacgcgc tttgagattc ccagcttttc 7440
ggccaatccc tgcggtgcat aggcgcgtgg ctcgaccgct tgcgggctga tggtgacgtg 7500
gcccactggt ggccgctcca gggcctcgta gaacgcctga atgcgcgtgt gacgtgcctt 7560
gctgccctcg atgccccgtt gcagccctag atcggccaca gcggccgcaa acgtggtctg 7620
gtcgcgggtc atctgcgctt tgttgccgat gaactccttg gccgacagcc tgccgtcctg 7680
cgtcagcggc accacgaacg cggtcatgtg cgggctggtt tcgtcacggt ggatgctggc 7740
cgtcacgatg cgatccgccc cgtacttgtc cgccagccac ttgtgcgcct tctcgaagaa 7800
cgccgcctgc tgttcttggc tggccgactt ccaccattcc gggctggccg tcatgacgta 7860

ctcgaccgcc aacacagcgt ccttgcgccg cttctctggc agcaactcgc gcagtcggcc 7920

catcgcttca tcggtgctgc tggccgccca gtgctcgttc tctggcgtcc tgctggcgtc 7980

agcgttgggc gtctcgcgct cgcggtaggc gtgcttgaga ctggccgcca cgttgcccat 8040

tttcgccagc ttcttgcatc gcatgatcgc gtatgccgcc atgcctgccc ctcccttttg 8100

gtgtccaacc ggctcgacgg gggcagcgca aggcggtgcc tccggcgggc cactcaatgc 8160

ttgagtatac tcactagact ttgcttcgca aagtcgtgac cgcctacggc ggctgcggcg 8220

ccctacgggc ttgctctccg ggcttcgccc tgcgcggtcg ctgcgctc 8268

<210> 20
<211> 8959
<212> DNA
<213> Escherichia coli

<220>
<221> misc_feature
<223> pBMT-3_ccdAB_PT7-?tesA_PT7-nphT7-hbd-crt-ter

<220>
<221> misc_feature
<223> pBMT-3_ccdAB_PT7-'tesA_PT7-nphT7-hbd-crt-ter

<400> 20
ccttgccagc ccgtggatat gtggacgatg gccgcgagcg gccaccggct ggctcgcttc 60

gctcggcccg tggacaaccc tgctggacaa gctgatggac aggctgcgcc tgcccacgag 120

cttgaccaca gggattgccc accggctacc cagccttcga ccacataccc accggctcca 180

actgcgcggc ctgcggcctt gccccatcaa ttttttaat tttctctggg gaaaagcctc 240

cggcctgcgg cctgcgcgct tcgcttgccg gttggacacc aagtggaagg cgggtcaagg 300

ctcgcgcagc gaccgcgcag cggcttggcc ttgacgcgcc tggaacgacc caagcctatg 360

cgagtggggg cagtcgaagg cgaagcccgc ccgcctgccc cccgagcctc acggcggcga 420

gtgcgggggt tccaaggggg cagcgccacc ttgggcaagg ccgaaggccg cgcagtcgat 480

caacaagccc cggaggggcc actttttgcc ggaggggag ccgcgccgaa ggcgtggggg 540

aacccccgcag gggtgccctt ctttgggcac caaagaacta gatatagggc gaaatgcgaa 600

agacttaaaa atcaacaact taaaaaaggg gggtacgcaa cagctcattg cggcaccccc 660

cgcaatagct cattgcgtag gttaaagaaa atctgtaatt gactgccact tttacgcaac 720

gcataattgt tgtcgcgctg ccgaaaagtt gcagctgatt gcgcatggtg ccgcaaccgt 780

gcggcaccct accgcatgga gataagcatg gccacgcagt ccagagaaat cggcattcaa 840

gccaagaaca agcccggtca ctgggtgcaa acggaacgca aagcgcatga ggcgtgggcc 900

gggcttattg cgaggaaacc cacggcggca atgctgctgc atcacctcgt ggcgcagatg 960

ggccaccaga acgccgtggt ggtcagccag aagacacttt ccaagctcat cggacgttct 1020
ttgcggacgg tccaatacgc agtcaaggac ttggtggccg agcgctggat ctccgtcgtg 1080
aagctcaacg gccccggcac cgtgtcggcc tacgtggtca atgaccgcgt ggcgtggggc 1140
cagccccgcg accagttgcg cctgtcggtg ttcagtgccg ccgtggtggt tgatcacgac 1200
gaccaggacg aatcgctgtt ggggcatggc gacctgcgcc gcatcccgac cctgtatccg 1260
ggcgagcagc aactaccgac cggcccccggc gaggagccgc ccagccagcc cggcattccg 1320
ggcatggaac cagacctgcc agccttgacc gaaacggagg aatgggaacg gcgcgggcag 1380
cagcgcctgc cgatgcccga tgagccgtgt tttctggacg atggcgagcc gttggagccg 1440
ccgacacggg tcacgctgcc gcgccggtag tacgtacccg gaattgccag ctggggcgcc 1500
ctctggtaag gttgggaagc cctgcaaagt aaactggatg gctttcttgc cgccaaggat 1560
ctgatggcgc aggggatcaa gctctgatca agagacagga tgaggatcgt ttcgatgaag 1620
cagcgtatta cagtgacagt tgacagcgac agctatcagt tgctcaaggc atatgatgtc 1680
aatatctccg gtctggtaag cacaaccatg cagaatgaag cccgtcgtct gcgtgccgaa 1740
cgctggaaag cggaaaatca ggaagggatg gctgaggtcg cccggtttat tgaaatgaac 1800
ggctcttttg ctgacgagaa cagggactgg tgaaatgcag tttaaggttt acacctataa 1860
aagagagagc cgttatcgtc tgtttgtgga tgtacagagc gatattattg acacgcccgg 1920
gcgacggatg gtgatccccc tggccagtgc acgtctgctg tcagataaag tctcccgtga 1980
actttacccg gtggtgcata tcggggatga aagctggcgc atgatgacca ccgatatggc 2040
cagtgtgccg gtctccgtta tcggggaaga agtggctgat ctcagccacc gcgaaaatga 2100
catcaaaaac gccattaacc tgatgttctg gggaatataa atcctagacg aattctctag 2160
tagaggttcc aactttcacc ataatgaaat aagatcacta ccgggcgtat tttttgagtt 2220
atcgagattt tcaggagcta aggaagctaa aatggagaaa aaaatcactg gatataccac 2280
cgttgatata tcccaatggc atcgtaaaga acattttgag gcatttcagt cagttgctca 2340
atgtacctat aaccagaccg ttcagctgga tattacggcc tttttaaaga ccgtaaagaa 2400
aaataagcac aagttttatc cggcctttat tcacattctt gcccgcctga tgaatgctca 2460
tccggaattc cgtatggcaa tgaaagacgg tgagctggtg atatgggata gtgttcaccc 2520
ttgttacacc gttttccatg agcaaactga aacgttttca tcgctctgga gtgaatacca 2580
cgacgatttc cggcagtttc tacacatata ttcgcaagat gtggcgtgtt acggtgaaaa 2640
cctggcctat ttccctaaag ggtttattga gaatatgttt ttcgtctcag ccaatccctg 2700
ggtgagtttc accagttttg atttaaacgt ggccaatatg gacaacttct tcgcccccgt 2760
tttcaccatg ggcaaatatt atacgcaagg cgacaaggtg ctgatgccgc tggcgattca 2820
ggttcatcat gccgtttgtg atggcttcca tgtcggcaga atgcttaatg aattacaaca 2880

gtactgcgat gagtggcagg gcggggcgta aagatcttct cgacgctctc ccttatgcga 2940
ctcctgcatt aggaaattaa tacgactcac tataggggaa ttgtgagcgg ataacaattc 3000
ccctgtagaa ataattttgt ttaactttaa taaggagata taccatggcg gcggacaccc 3060
tgctgatcct gggcgactcc ctgtcggcgg gctaccgcat gtcggcgtcg gcggcgtggc 3120
cggcgctgct gaacgacaag tggcagtcca agacctcggt ggtcaacgcc tcgatcagcg 3180
gcgacacgag ccagcagggc ctggcccgcc tgccggcgct gctgaagcag caccagccgc 3240
gctgggtgct ggtcgagctg ggcggcaacg acggcctgcg cggcttccag ccgcagcaga 3300
ccgaacagac gctgcgccag atcctgcagg acgtgaaggc cgcgaacgcc gagccgctgc 3360
tgatgcagat ccgcctgccg gcgaactacg gccgtcgcta caacgaggcc ttctccgcga 3420
tctacccgaa gctggccaag gaattcgacg tgccgctgct gccgttcttc atggaggaag 3480
tctacctgaa gccgcagtgg atgcaggacg acggcatcca cccgaaccgc gacgcgcagc 3540
cgttcatcgc cgactggatg gccaagcaac tgcagccgct ggtcaaccac gactcctgaa 3600
tttaaatccc ttatgcgact cctgcattag gaaattaata cgactcacta taggggaatt 3660
gtgagcggat aacaattccc ctgtagaaat aattttgttt aactttaata aggagatata 3720
ccatgacgga tgtccgcttt cgtatcattg gcacgggcgc ttacgtcccg gaacgcattg 3780
tcagcaacga cgaagtcggc gcaccggctg gtgtggatga cgattggatt acgcgtaaaa 3840
ccggtatccg ccagcgtcgc tgggcggccg acgatcaagc aacgagcgat ctggctaccg 3900
cagctggtcg tgcagcactg aaagcagctg gcattacgcc ggaacagctg accgtcatcg 3960
cagtggctac ctctacgccg gaccgtccgc agccgccgac cgcggcctat gttcaacatc 4020
acctgggtgc aaccggcacg gcagcttttg atgttaacgc tgtgtgcagc ggtacggttt 4080
tcgcgctgag ctctgttgcc ggcaccctgg tctatcgtgg cggttacgca ctggtcattg 4140
gtgctgatct gtactcacgt atcctgaatc cggcggaccg caaaaccgtg gttctgttcg 4200
gcgatggtgc gggcgcgatg gtgctgggcc cgaccagcac cggcaccggt ccgattgtgc 4260
gtcgcgttgc actgcatacg tttggcggtc tgaccgatct gatccgtgtt ccggccggcg 4320
gttcccgcca gccgctggac accgatggtc tggacgcagg cctgcaatat tttgcgatgg 4380
atggccgcga agtccgtcgc ttcgtgaccg aacatctgcc gcagctgatt aaaggttttc 4440
tgcacgaagc gggcgtggat gcggcggata ttagccattt cgtgccgcac caagccaacg 4500
gtgtgatgct ggacgaagtt tttggcgaac tgcatctgcc gcgtgcaacc atgcaccgta 4560
cggtggaaac ctacggtaat acgggcgcag ctagtattcc gatcacgatg gatgcggccg 4620
ttcgtgcagg ttccttccgt ccgggcgaac tggttctgct ggcgggcttt ggcggcggta 4680
tggcggcttc gtttgctctg attgaatggt gacctaatgc aggctgcagg cggatacgag 4740

gaggaataaa ccatgaaaaa ggtatgtgtt ataggtgcag gtactatggg ttcaggaatt 4800
gctcaggcat ttgcagctaa aggatttgaa gtagtattaa gagatattaa agatgaattt 4860
gttgatagag gattagattt tatcaataaa aatctttcta aattagttaa aaaaggaaag 4920
atagaagaag ctactaaagt tgaaatctta actagaattt ccggaacagt tgaccttaat 4980
atggcagctg attgcgattt agttatagaa gcagctgttg aaagaatgga tattaaaaag 5040
cagatttttg ctgacttaga caatatatgc aagccagaaa caattcttgc atcaaataca 5100
tcatcacttt caataacaga agtggcatca gcaactaaaa gacctgataa ggttataggt 5160
atgcatttct ttaatccagc tcctgttatg aagcttgtag aggtaataag aggaatagct 5220
acatcacaag aaacttttga tgcagttaaa gagacatcta tagcaatagg aaaagatcct 5280
gtagaagtag cagaagcacc aggatttgtt gtaaatagaa tattaatacc aatgattaat 5340
gaagcagttg gtatattagc agaaggaata gcttcagtag aagacataga taaagctatg 5400
aaacttggag ctaatcaccc aatgggacca ttagaattag gtgattttat aggtcttgat 5460
atatgtcttg ctataatgga tgttttatac tcagaaactg gagattctaa gtatagacca 5520
catacattac ttaagaagta tgtaagagca ggatggcttg gaagaaaatc aggaaaaggt 5580
ttctacgatt attcaaaata agtttacagg atctgcaggg aggaggaaat catggagttg 5640
aacaacgtta ttctggagaa agaaggcaag gtggcggttg tcaccattaa ccgtccaaag 5700
gccctgaacg ctctgaactc ggataccctg aaagagatgg attacgttat tggcgagatt 5760
gagaatgaca gcgaagtgct ggctgtgatt ctgaccggtg cgggtgagaa gagctttgtc 5820
gcgggtgcgg acatcagcga gatgaaagaa atgaacacca tcgaaggccg taagttcggt 5880
attctgggca acaaggtgtt tcgtcgtctg gaactgctgg agaaacctgt cattgctgcc 5940
gtgaacggtt tcgcgctggg cggtggttgc gagatcgcta tgagctgcga tattcgtatc 6000
gcatcgtcca acgcacgctt tggtcaaccg gaggtcggtc tgggtatcac tccgggtttc 6060
ggcggtacgc aacgtctgag ccgcctggtt ggcatgggca tggcgaaaca gttgattttc 6120
acggcacaga acattaaggc ggatgaggcg ctgcgtattg gtctggtgaa taaggtcgtt 6180
gagccaagcg aactgatgaa taccgcgaaa gaaattgcga acaagatcgt tagcaatgcc 6240
ccggtggccg ttaagctgtc gaaacaggca atcaaccgtg gcatgcagtg tgacatcgac 6300
accgccctgg cgtttgagag cgaggcgttt ggtgagtgct tctccaccga ggaccaaaag 6360
gatgcgatga ccgcgttcat tgagaaacgc aagatcgagg gtttcaagaa tcgttaatag 6420
aggaggatag gaggttttca tatgattgtg aaaccgatgg tccgtaataa tatctgtctg 6480
aatgctcacc cgcagggctg taaaaaaggc gtggaagatc aaattgaata taccaaaaaa 6540
cgtattacgg cagaagtgaa agccggcgca aaagctccga aaaacgtgct ggttctgggt 6600
tgcagcaatg gctatggtct ggcttctcgc attaccgcgg cctttggcta cggtgcagct 6660

acgatcggcg ttagtttcga aaaagcaggt tccgaaacca aatatggcac gccgggttgg 6720
tacaacaatc tggcttttga tgaagcggcc aaacgtgaag gcctgtatag tgtcaccatt 6780
gatggtgacg cgttctccga tgaaattaaa gcacaggtga tcgaagaagc gaagaaaaaa 6840
ggcattaaat ttgacctgat cgtttacagc ctggcatctc cggtccgtac cgatccggac 6900
acgggtatca tgcataaatc tgtgctgaaa ccgtttggca aaaccttcac gggtaaaacc 6960
gttgatccgt tcacgggcga actgaaagaa attagcgcgg aaccggccaa cgatgaagaa 7020
gcagctgcga ccgtcaaagt gatgggcggt gaagactggg aacgttggat caaacagctg 7080
agtaaagaag gcctgctgga agaaggttgc attaccctgg cgtattccta catcggcccg 7140
gaagcaaccc aagctctgta tcgcaaaggc acgattggta aagcgaaaga acatctggaa 7200
gcgaccgccc accgtctgaa caaagaaaat ccgtcaatcc gcgccttcgt ttcggtcaat 7260
aaaggtctgg ttacccgtgc atcagctgtg attccggtta tcccgctgta cctggcatcg 7320
ctgtttaaag tcatgaaaga aaaaggcaac catgaaggtt gtattgaaca gatcacccgc 7380
ctgtatgccg aacgtctgta ccgcaaagat ggtacgattc cggtggacga agaaaatcgt 7440
attcgcatcg atgactggga actggaagaa gatgtccaaa aagccgtgag cgccctgatg 7500
gaaaaagtta ccggcgaaaa cgcggaatct ctgacggatc tggccggtta tcgtcacgac 7560
tttctggcga gtaatggttt tgatgttgaa ggcattaact acgaagctga agtggaacgc 7620
tttgatcgca tttgatctag agaattcgtc aacgaattca agcttgatat cattcaggac 7680
gagcctcaga ctccagcgta actggactga aaacaaacta aagcgccctt gtggcgcttt 7740
agttttgttc cgctcatgat aataatggtt tcttagacgt caggtggcac ttttcgggga 7800
aatgtgcgcg cccgcgttcc tgctggcgct gggcctgttt ctggcgctgg acttcccgct 7860
gttccgtcag cagcttttcg cccacggcct tgatgatcgc ggcggccttg gcctgcatat 7920
cccgattcaa cggccccagg gcgtccagaa cgggcttcag gcgctcccga aggtctcggg 7980
ccgtctcttg ggcttgatcg gccttcttgc gcatctcacg cgctcctgcg gcggcctgta 8040
gggcaggctc ataccccctgc cgaaccgctt ttgtcagccg gtcggccacg gcttccggcg 8100
tctcaacgcg ctttgagatt cccagctttt cggccaatcc ctgcggtgca taggcgcgtg 8160
gctcgaccgc ttgcgggctg atggtgacgt ggcccactgg tggccgctcc agggcctcgt 8220
agaacgcctg aatgcgcgtg tgacgtgcct tgctgccctc gatgccccgt tgcagcccta 8280
gatcggccac agcggccgca aacgtggtct ggtcgcgggt catctgcgct ttgttgccga 8340
tgaactcctt ggccgacagc ctgccgtcct gcgtcagcgg caccacgaac gcggtcatgt 8400
gcgggctggt ttcgtcacgg tggatgctgg ccgtcacgat gcgatccgcc ccgtacttgt 8460
ccgccagcca cttgtgcgcc ttctcgaaga acgccgcctg ctgttcttgg ctggccgact 8520

tccaccattc cgggctggcc gtcatgacgt actcgaccgc caacacagcg tccttgcgcc 8580

gcttctctgg cagcaactcg cgcagtcggc ccatcgcttc atcggtgctg ctggccgccc 8640

agtgctcgtt ctctggcgtc ctgctggcgt cagcgttggg cgtctcgcgc tcgcggtagg 8700

cgtgcttgag actggccgcc acgttgccca ttttcgccag cttcttgcat cgcatgatcg 8760

cgtatgccgc catgcctgcc cctccctttt ggtgtccaac cggctcgacg gggcagcgc 8820

aaggcggtgc ctccggcggg ccactcaatg cttgagtata ctcactagac tttgcttcgc 8880

aaagtcgtga ccgcctacgg cggctgcggc gccctacggg cttgctctcc gggcttcgcc 8940

ctgcgcggtc gctgcgctc 8959

<210> 21
<211> 5367
<212> DNA
<213> Escherichia coli

<220>
<221> misc_feature
<223> pET-28b(empty vector)

<400> 21
ggcgaatggg acgcgccctg tagcggcgca ttaagcgcgg cgggtgtggt ggttacgcgc 60

agcgtgaccg ctacacttgc cagcgcccta gcgcccgctc ctttcgcttt cttcccttcc 120

tttctcgcca cgttcgccgg ctttccccgt caagctctaa atcgggggct ccctttaggg 180

ttccgattta gtgctttacg gcacctcgac cccaaaaaac ttgattaggg tgatggttca 240

cgtagtgggc catcgccctg atagacggtt tttcgccctt tgacgttgga gtccacgttc 300

tttaatagtg gactcttgtt ccaaactgga acaacactca accctatctc ggtctattct 360

tttgatttat aagggatttt gccgatttcg gcctattggt taaaaaatga gctgatttaa 420

caaaaattta acgcgaattt taacaaaata ttaacgttta caatttcagg tggcactttt 480

cggggaaatg tgcgcggaac ccctatttgt ttatttttct aaatacattc aaatatgtat 540

ccgctcatga attaattctt agaaaaactc atcgagcatc aaatgaaact gcaatttatt 600

catatcagga ttatcaatac catatttttg aaaaagccgt ttctgtaatg aaggagaaaa 660

ctcaccgagg cagttccata ggatggcaag atcctggtat cggtctgcga ttccgactcg 720

tccaacatca atacaaccta ttaatttccc ctcgtcaaaa ataaggttat caagtgagaa 780

atcaccatga gtgacgactg aatccggtga gaatggcaaa agtttatgca tttctttcca 840

gacttgttca acaggccagc cattacgctc gtcatcaaaa tcactcgcat caaccaaacc 900

gttattcatt cgtgattgcg cctgagcgag acgaaatacg cgatcgctgt taaaaggaca 960

attacaaaca ggaatcgaat gcaaccggcg caggaacact gccagcgcat caacaatatt 1020

ttcacctgaa tcaggatatt cttctaatac ctggaatgct gttttcccgg ggatcgcagt 1080

ggtgagtaac catgcatcat caggagtacg gataaaatgc ttgatggtcg gaagaggcat 1140
aaattccgtc agccagttta gtctgaccat ctcatctgta acatcattgg caacgctacc 1200
tttgccatgt ttcagaaaca actctggcgc atcgggcttc ccatacaatc gatagattgt 1260
cgcacctgat tgcccgacat tatcgcgagc ccatttatac ccatataaat cagcatccat 1320
gttggaattt aatcgcggcc tagagcaaga cgtttcccgt tgaatatggc tcataacacc 1380
ccttgtatta ctgtttatgt aagcagacag ttttattgtt catgaccaaa atcccttaac 1440
gtgagttttc gttccactga gcgtcagacc ccgtagaaaa gatcaaagga tcttcttgag 1500
atcctttttt tctgcgcgta atctgctgct tgcaaacaaa aaaaccaccg ctaccagcgg 1560
tggtttgttt gccggatcaa gagctaccaa ctctttttcc gaaggtaact ggcttcagca 1620
gagcgcagat accaaatact gtccttctag tgtagccgta gttaggccac cacttcaaga 1680
actctgtagc accgcctaca tacctcgctc tgctaatcct gttaccagtg gctgctgcca 1740
gtggcgataa gtcgtgtctt accgggttgg actcaagacg atagttaccg gataaggcgc 1800
agcggtcggg ctgaacgggg ggttcgtgca cacagcccag cttggagcga acgacctaca 1860
ccgaactgag atacctacag cgtgagctat gagaaagcgc cacgcttccc gaagggagaa 1920
aggcggacag gtatccggta agcggcaggg tcggaacagg agagcgcacg agggagcttc 1980
cagggggaaa cgcctggtat ctttatagtc ctgtcgggtt tcgccacctc tgacttgagc 2040
gtcgattttt gtgatgctcg tcaggggggc ggagcctatg gaaaaacgcc agcaacgcgg 2100
cctttttacg gttcctggcc ttttgctggc cttttgctca catgttcttt cctgcgttat 2160
cccctgattc tgtggataac cgtattaccg cctttgagtg agctgatacc gctcgccgca 2220
gccgaacgac cgagcgcagc gagtcagtga gcgaggaagc ggaagagcgc ctgatgcggt 2280
attttctcct tacgcatctg tgcggtattt cacaccgcat atatggtgca ctctcagtac 2340
aatctgctct gatgccgcat agttaagcca gtatacactc cgctatcgct acgtgactgg 2400
gtcatggctg cgccccgaca cccgccaaca cccgctgacg cgccctgacg ggcttgtctg 2460
ctcccggcat ccgcttacag acaagctgtg accgtctccg ggagctgcat gtgtcagagg 2520
ttttcaccgt catcaccgaa acgcgcgagg cagctgcggt aaagctcatc agcgtggtcg 2580
tgaagcgatt cacagatgtc tgcctgttca tccgcgtcca gctcgttgag tttctccaga 2640
agcgttaatg tctggcttct gataaagcgg gccatgttaa gggcggtttt ttcctgtttg 2700
gtcactgatg cctccgtgta agggggattt ctgttcatgg gggtaatgat accgatgaaa 2760
cgagagagga tgctcacgat acgggttact gatgatgaac atgcccggtt actggaacgt 2820
tgtgagggta aacaactggc ggtatggatg cggcgggacc agagaaaaat cactcagggt 2880
caatgccagc gcttcgttaa tacagatgta ggtgttccac agggtagcca gcagcatcct 2940

gcgatgcaga tccggaacat aatggtgcag ggcgctgact tccgcgtttc cagactttac 3000

gaaacacgga aaccgaagac cattcatgtt gttgctcagg tcgcagacgt tttgcagcag 3060

cagtcgcttc acgttcgctc gcgtatcggt gattcattct gctaaccagt aaggcaaccc 3120

cgccagccta gccgggtcct caacgacagg agcacgatca tgcgcacccg tggggccgcc 3180

atgccggcga taatggcctg cttctcgccg aaacgtttgg tggcgggacc agtgacgaag 3240

gcttgagcga gggcgtgcaa gattccgaat accgcaagcg acaggccgat catcgtcgcg 3300

ctccagcgaa agcggtcctc gccgaaaatg acccagagcg ctgccggcac ctgtcctacg 3360

agttgcatga taaagaagac agtcataagt gcggcgacga tagtcatgcc ccgcgcccac 3420

cggaaggagc tgactgggtt gaaggctctc aagggcatcg gtcgagatcc cggtgcctaa 3480

tgagtgagct aacttacatt aattgcgttg cgctcactgc ccgctttcca gtcgggaaac 3540

ctgtcgtgcc agctgcatta atgaatcggc caacgcgcgg ggagaggcgg tttgcgtatt 3600

gggcgccagg gtggtttttc ttttcaccag tgagacgggc aacagctgat tgcccttcac 3660

cgcctggccc tgagagagtt gcagcaagcg gtccacgctg gtttgcccca gcaggcgaaa 3720

atcctgtttg atggtggtta acggcgggat ataacatgag ctgtcttcgg tatcgtcgta 3780

tcccactacc gagatatccg caccaacgcg cagcccggac tcggtaatgg cgcgcattgc 3840

gcccagcgcc atctgatcgt tggcaaccag catcgcagtg ggaacgatgc cctcattcag 3900

catttgcatg gtttgttgaa aaccggacat ggcactccag tcgccttccc gttccgctat 3960

cggctgaatt tgattgcgag tgagatattt atgccagcca gccagacgca gacgcgccga 4020

gacagaactt aatgggcccg ctaacagcgc gatttgctgg tgacccaatg cgaccagatg 4080

ctccacgccc agtcgcgtac cgtcttcatg ggagaaaata atactgttga tgggtgtctg 4140

gtcagagaca tcaagaaata acgccggaac attagtgcag gcagcttcca cagcaatggc 4200

atcctggtca tccagcggat agttaatgat cagcccactg acgcgttgcg cgagaagatt 4260

gtgcaccgcc gctttacagg cttcgacgcc gcttcgttct accatcgaca ccaccacgct 4320

ggcacccagt tgatcggcgc gagatttaat cgccgcgaca atttgcgacg gcgcgtgcag 4380

ggccagactg gaggtggcaa cgccaatcag caacgactgt ttgcccgcca gttgttgtgc 4440

cacgcggttg ggaatgtaat tcagctccgc catcgccgct tccacttttt cccgcgtttt 4500

cgcagaaacg tggctggcct ggttcaccac gcgggaaacg gtctgataag agacaccggc 4560

atactctgcg acatcgtata acgttactgg tttcacattc accaccctga attgactctc 4620

ttccgggcgc tatcatgcca taccgcgaaa ggttttgcgc cattcgatgg tgtccgggat 4680

ctcgacgctc tcccttatgc gactcctgca ttaggaagca gcccagtagt aggttgaggc 4740

cgttgagcac cgccgccgca aggaatggtg catgcaagga gatggcgccc aacagtcccc 4800

cggccacggg gcctgccacc atacccacgc cgaaacaagc gctcatgagc ccgaagtggc 4860

```
gagcccgatc ttccccatcg gtgatgtcgg cgatataggc gccagcaacc gcacctgtgg     4920
cgccggtgat gccggccacg atgcgtccgg cgtagaggat cgagatctcg atcccgcgaa     4980
attaatacga ctcactatag gggaattgtg agcggataac aattcccctc tagaaataat     5040
tttgtttaac tttaagaagg agatatacca tgggcagcag ccatcatcat catcatcaca     5100
gcagcggcct ggtgccgcgc ggcagccata tggctagcat gactggtgga cagcaaatgg     5160
gtcgggatcc gaattcgagc tccgtcgaca agcttgcggc cgcactcgag caccaccacc     5220
accaccactg agatccggct gctaacaaag cccgaaagga agctgagttg gctgctgcca     5280
ccgctgagca ataactagca taaccccttg gggcctctaa acgggtcttg aggggttttt     5340
tgctgaaagg aggaactata tccggat                                         5367
```

## Description

The present invention relates to a cell expressing an amino acid-N-acyl-transferase, which is preferably recombinant, and an acyl-CoA synthetase, which is preferably recombinant, wherein the cell has a reduced fatty acid degradation capacity, a method for producing acyl amino acids, comprising the step contacting an amino acid and fatty acid-CoA in the presence of an amino acid-N-acyl-transferase, which is preferably isolated and/or recombinant, wherein the amino acid-N-acyl-transferase is preferably a human amino acid-N-acyl-transferase, or culturing the cell, and a reaction mixture comprising an amino acid-N-acyl-transferase, which is preferably isolated and/or recombinant, an acyl-CoA synthetase, which is preferably isolated and/or recombinant, an amino acid and either a fatty acid-CoA or a fatty acid and an acyl-CoA-synthase.

Acyl amino acids are a class of surface-active agents with a variety of uses, for example as detergents for washing purposes, emulsifiers in food products and as essential ingredients in various personal care products such as shampoos, soaps, moisturizing agents and the like. In addition to having both hydrophobic and hydrophilic regions, a prerequisite for use as a surfactant, the compounds are made of naturally occurring molecules, more specifically amino acids and fatty acids, which are not only non-hazardous and environmentally acceptable but may be readily produced at a large scale using inexpensive biological raw materials. In pharmacological research, acyl amino acids are used as neuromodulators and probes for new drug targets.

Acyl amino acids have been isolated from a multitude of biological sources and are believed to have a range of functions, for example as signaling molecules in mammalian tissues (Tan, B., O'Dell, D. K., Yu, Y. W., Monn, M. F., Hughes, H. V., Burstein, S., Walker, J.M. (2010), Identification of endogenous acyl amino acids based on a targeted lipidomics approach, J. Lipid Res. 51(1), 112-119)), as building blocks for antibiotics in bacterial cultures (Clardy, J., and Brady, S. F. (2007), Cyclic AMP directly activates NasP, an N-acyl amino acid antibiotic biosynthetic enzyme cloned from an uncultured beta-proteobacterium, J. Bacteriol. 189(17), 6487-6489) or as compounds involved in bacterial protein sorting (Craig, J. W., Cherry, M. A., Brady, S. F.(2011), Long-chain N-acyl amino acid synthases are linked to the putative PEP-CTERM/exosortase protein-sorting system in Gram-negative bacteria, J. Bacteriol. 193(20), 5707-5715).

Traditionally acyl amino acids have been produced at an industrial scale starting with materials derived from petrochemicals. More specifically, activated fatty acids provided in the form of acid chlorides may be used to acylate amino acids in an aqueous alkaline medium as described in GB 1 483 500. Shortcomings of such approaches include the need to add hazardous chemicals such as sulphuric acid or anhydrides thereof. Other synthetic approaches are associated with the accumulation of by-products such as chloride salts which have undesirable effect on surfactancy.

A range of biotechnological routes towards production of acyl amino acids has been described. However, none of them is adequate for the commercial large-scale production of acyl amino acids owing to low yields, insufficient purities and the need for multi-step purification procedures. In particular only a small proportion of the carbon substrates fed to biotechnologically useful organisms is actually converted to the sought-after product, whilst much of it is consumed by reactions of the primary metabolism.

Another problem associated with biotechnological routes is the fact that a mixture of products is obtained and thus the composition is difficult to control. More specifically, a range of fatty acids may be converted to acyl amino acids, even though production of a single adduct may be desirable. Since the mixture comprises compounds highly related in terms of chemical structure, purifying or at least enriching a single component in an efficient and straightforward manner is usually beyond technical feasibility.

These problems may be solved by the subject matter of the attached claims.

In particular, the present invention may provide an efficient biotechnological route towards acyl amino acids. In particular, the yield and purity of the product of the present invention, in terms of catalysts or unwanted by-products, may be improved compared to the processes in the state of the art.

The present invention also provides a method for making acyl amino acids, wherein the spectrum of fatty acids converted to acyl amino acids is broader than the processes in the state of the art. In particular, the method of the present invention may be suitable for converting short and unsaturated fatty acids to acyl amino acids.

The present invention may also provide a biotechnological method for making acyl amino acids, wherein the length of the acyl residue in the acyl amino acid product may be controlled, preferably such that lauryl is enriched or prevalent.

In a first aspect the present invention provides a cell expressing an amino acid-N-acyl-transferase, which is preferably recombinant, and an acyl-CoA synthetase, which is preferably recombinant, wherein the cell has a reduced fatty acid degradation capacity.

In a first embodiment of the first aspect, the fatty acid degradation capacity of said cell is reduced owing to a decrease in activity, compared to the wild type cell, of at least one enzyme selected from the group consisting of acyl-CoA dehydrogenase, 2,4-dienoyl-CoA reductase, enoyl-CoA hydratase and 3-ketoacyl-CoA thiolase, preferably acyl-CoA dehydrogenase.

In a second embodiment, which is also an embodiment of the first embodiment, wherein the amino acid-N-acyl-transferase is a human amino acid-N-acyl-transferase, preferably SEQ ID NO: 4, SEQ ID NO: 5 or a variant thereof.

In a third embodiment, which is also an embodiment of the first to second embodiment, the cell is a bacterial cell, preferably an enterobacterial cell, more preferably *E. coli.*

In a fourth embodiment, which is also an embodiment of the first to third embodiments, the cell is capable of making proteinogenic amino acids and/or fatty acids.

In a fifth embodiment, which is also an embodiment of the first to fourth embodiments, the cell expresses an acyl-CoA thioesterase, which is preferably recombinant and is more preferably SEQ ID NO: 1 or a variant thereof.

In a sixth embodiment, which is also an embodiment of the first to fifth embodiments, the acyl-CoA synthetase is SEQ ID NO: 6 or a variant thereof.

In a second aspect the present invention provides a method for producing acyl amino acids, comprising the step of

b) contacting an amino acid and an acyl CoA in the presence of an amino acid-N-acyl-transferase, which is preferably isolated and/or recombinant,
wherein the amino acid-N-acyl-transferase is preferably a human amino acid-N-acyl-transferase, more preferably SEQ ID NO 4, SEQ ID NO 5 or a variant thereof, and
wherein the acyl-CoA synthetase is preferably SEQ ID NO 6 or a variant thereof,
or culturing the cell according to the first aspect or any embodiment thereof.

In a first embodiment of the second aspect, the method comprises, in addition to step b), the steps of

a) converting a fatty acid to an acyl CoA using an acyl-CoA synthetase, which is preferably isolated and/or recombinant and/or
c) hydrogenating.

In a second embodiment, which is also an embodiment of the first embodiment of the second aspect, at least one of the enzymes, preferably all of them, selected from the group consisting of amino acid-N-acyl-transferase and acyl-CoA synthetase is provided in the form of a cell expressing said enzyme or enzymes.

In a third embodiment, which is also an embodiment of the first or second embodiments of the second aspect, the cell expressing said enzyme or enzymes is the cell according to the first aspect or any embodiment thereof.

In a third aspect the present invention provides a reaction mixture comprising
an amino acid-N-acyl-transferase, which is preferably isolated and/or recombinant,
an acyl-CoA synthetase, which is preferably isolated and/or recombinant,
an amino acid and/or
either an acyl CoA or a fatty acid and an acyl-CoA-synthetase,
wherein the amino acid-N-acyl-transferase is preferably a human amino acid-N-acyl-transferase, more preferably SEQ ID NO: 4, SEQ ID NO: 5 or a variant thereof, and wherein the acyl-CoA synthetase is preferably SEQ ID NO: 6 or a variant thereof.

In a first embodiment of the third aspect, at least one of the enzymes, preferably all of them, selected from the group consisting of an amino acid-N-acyl-transferase and an acyl-CoA synthetase is provided in the form of a cell, preferably the cell according to the first aspect or any embodiment thereof.

In a second embodiment, which is also an embodiment of the first embodiment, the amino acid is a proteinogenic amino acid, preferably selected from the group consisting of glycine, glutamine, glutamate, asparagine and alanine and is more preferably glycine.

In a third embodiment, which is also an embodiment of the first to second embodiments, the fatty acid is an unsaturated fatty acid and is preferably selected from the group consisting of myristoleic acid, lauroleic acid, palmitoleic acid and cis-vaccenic acid.

In another embodiment of the second or third aspect, the fatty acid is a saturated fatty acid and is preferably selected from the group consisting of laurate, myristate and palmitate.

In another embodiment of the second or third aspect, the fatty acid is provided in the form of an organic phase comprising a liquid organic solvent and the fatty acid, wherein the organic solvent is preferably an ester of the fatty acid.

In a fourth aspect, the present invention provides a composition comprising
a first acyl amino acid consisting of a saturated acyl having 8 to 16 carbon atoms and glycine,
a second acyl amino acid consisting of an unsaturated acyl having 10 to 18 carbon atoms and glycine,
and optionally a third acyl amino acid consisting of a saturated or unsaturated acyl having 12 carbon atoms and an amino acid selected from the group consisting of glutamine, glutamic acid, alanine and asparagine.

In a first embodiment of the fourth aspect, the first acyl amino acid consists of a saturated acyl having 12 carbon atoms, preferably lauryl, and glycine.

In a second embodiment of the fourth aspect, which is also an embodiment of the first embodiment of the fourth aspect, the second acyl amino acid consists of an unsaturated acyl having 12 or 14 carbon atoms and glycine. According to any aspect of the present invention, the acyl amino acids formed may be a mixture of amino acids. The mixture of amino acids may comprise at least two proteinogenic amino acids as defined below. In particular, the mixture of amino acids may have 3, 4, 5, 6, 7, 8, 9, 10, 11, 12, 13, 14, 15, 16, 17, 18, 19, 20, or 21 proteinogenic amino acids. In one example, the mixture of amino acids may be used to form cocoyl glycine and salts thereof.

The present invention is based on the surprising finding that the combination of amino acid-N-acyl transferase and an acyl-CoA synthetase, preferably expressed by a cell having reduced fatty acid degradation capacity, may be used to convert a variety of fatty acids, more preferably a mixture comprising unsaturated and saturated fatty acids, to acyl amino acids.

Moreover, the present invention is based on the surprising finding that amino acid-N-acyl transferases exist that may be used to convert to an acyl amino acid short unsaturated fatty acids such as lauroleic acid.

Moreover, the present invention is based on the surprising finding that employing an amino acid-N-acyl-transferase capable of converting to an acyl amino acid a variety of fatty acids including short unsaturated fatty acids such as lauroleic acid may increase the yields of acyl amino acids produced.

Moreover, the present invention is based on the surprising finding that the composition of acyl amino acids produced in a cell, more specifically the length of fatty acids incorporated into such acyl amino acids, may be controlled by introducing into the cell one or more specific acyl-CoA thioesterases or altering the expression of one or more acyl-CoA thioesterases endogenously expressed by the cell.

The present invention centers around the use of an amino acid-N-acyl transferase and an acyl-CoA synthetase for making acyl amino acids. In a preferred embodiment, the term "amino acid-N-acyl transferase", as used herein, refers to an enzyme capable of catalysing the conversion of acyl-CoA, preferably the CoA ester of lauroleic acid, and an amino acid, preferably a proteinogenic amino acid, more preferably glycine, to an acyl amino acid. Suitable amino acid-N-acyl transferases have been described in the prior art, for example in Waluk, D. P., Schultz, N., and Hunt, M. C. (2010), Identification of glycine N-acyltransferase-like 2 (GLYATL2) as a transferase that produces N-acyl glycines in humans, FASEB J. 24, 2795-2803. In a preferred embodiment, the amino acid-N-acyl transferase comprises a nucleotide sequence of SEQ ID NO:4. In particular, the amino acid sequence of amino acid-N-acyl transferase may be selected from the group consisting of NP_001010904.1,NP_659453.3, XP_001147054.1, AAH16789.1, AAO73139.1, XP_003275392.1, XP_002755356.1, XP_003920208.1, XP_004051278.1, XP_006147456.1, XP_006214970.1, XP_003801413.1, XP_006189704.1, XP_003993512.1, XP_005862181.1, XP_007092708.1, XP_006772167.1, XP_006091892.1, XP_005660936.1, XP_005911029.1, NP_001178259.1, XP_004016547.1, XP_005954684.1, ELR45061.1, XP_005690354.1, XP_004409352.1, XP_007519553.1, XP_004777729.1, XP_005660935.1, XP_004824058.1, XP_006068141.1, XP_006900486.1, XP_007497585.1, XP_002821801.2, XP_007497583.1, XP_003774260.1, XP_001377648.2, XP_003909843.1, XP_003801448.1, XP_001091958.1, XP_002821798.1, XP_005577840.1, XP_001092197.1, NP_001207423.1, NP_001207425.1, XP_003954287.1, NP_001271595.1, XP_003909848.1, XP_004087850.1, XP_004051279.1, XP_003920209.1, XP_005577835.1, XP_003774402.1, XP_003909846.1, XP_004389401.1, XP_002821802.1, XP_003774401.1, XP_007497581.1, EHH21814.1, XP_003909845.1, XP_005577839.1, XP_003774403.1, XP_001092427.1, XP_003275395.2, NP_542392.2, XP_001147271.1, XP_005577837.1, XP_003826420.1, XP_004051281.1, XP_001147649.2, XP_003826678.1, XP_003909847.1, XP_004682812.1, XP_004682811.1, XP_003734315.1, XP_004715052.1, BAG62195.1, XP_003777804.1, XP_003909849.1, XP_001092316.2, XP_006167891.1, XP_540580.2, XP_001512426.1, EAW73833.1, XP_003464217.1, XP_007519551.1, XP_003774037.1, XP_005954680.1, XP_003801411.1, NP_803479.1, XP_004437460.1, XP_006875830.1, XP_004328969.1, XP_004264206.1, XP_004683490.1, XP_004777683.1, XP_005954681.1, XP_003480745.1, XP_004777682.1, XP_004878093.1, XP_007519550.1, XP_003421399.1, EHH53167.1, XP_006172214.1 ,XP_003993453.1 ,AAI12537.1, XP_006189705.1 ,Q2KIR7.2, XP_003421465.1, NP_001009648.1, XP_003464328.1, XP_001504745.1, ELV11036.1, XP_005690351.1, XP_005216632.1, EPY77465.1, XP_005690352.1, XP_004016544.1, XP_001498276.2, XP_004264205.1, XP_005690353.1, XP_005954683.1, XP_004667759.1, XP_004479306.1, XP_004645843.1, XP_004016543.1, XP_002928268.1, XP_006091904.1, XP_005331614.1, XP_007196549.1, XP_007092705.1, XP_004620532.1, XP_004869789.1, EHA98800.1, XP_004016545.1, XP_004479307.1, XP_004093105.1, NP_001095518.1, XP_005408101.1, XP_004409350.1, XP_001498290.1, XP_006056693.1, XP_005216639.1, XP_007455745.1, XP_005352049.1, XP_004328970.1, XP_002709220.1, XP_004878092.1, XP_007196553.1, XP_006996816.1, XP_005331615.1, XP_006772157.1, XP_007196552.1, XP_004016546.1, XP_007628721.1, NP_803452.1, XP_004479304.1, DAA21601.1, XP_003920207.1, XP_006091906.1, XP_003464227.1, XP_006091903.1, XP_006189706.1, XP_007455744.1, XP_004585544.1, XP_003801410.1, XP_007124812.1, XP_006900488.1, XP_004777680.1, XP_005907436.1, XP_004389356.1, XP_007124811.1, XP_005660937.1, XP_007628724.1, XP_003513512.1, XP_004437813.1, XP_007628723.1, ERE78858.1, EPQ15380.1, XP_005862178.1, XP_005878672.1, XP_540581.1, XP_002928267.1, XP_004645845.1, EPQ05184.1, XP_003513511.1, XP_006214972.1, XP_007196545.1, XP_007196547.1, XP_006772160.1, XP_003801409.1, NP_001119750.1, XP_003801412.1, XP_006772159.1, EAW73832.1, XP_006091897.1, XP_006772163.1, XP_006091898.1, XP_005408105.1, XP_006900487.1, XP_003993454.1, XP_003122754.3, XP_007455746.1, XP_005331618.1, XP_004585337.1, XP_005063305.1, XP_006091895.1, XP_006772156.1, XP_004051276.1, XP_004683488.1, NP_666047.1, NP_001013784.2, XP_006996815.1, XP_006996821.1, XP_006091893.1, XP_006173036.1, XP_006214971.1, EPY89845.1, XP_003826423.1, NP_964011.2, XP_007092707.1, XP_005063858.1, BAL43174.1, XP_001161154.2, XP_007124813.1, NP_083826.1XP_003464239.1, XP_003275394.1, ELK23978.1, XP_004878097.1, XP_004878098.1, XP_004437459.1, XP_004264204.1, XP_004409351.1, XP_005352047.1, Q5RFP0.1, XP_005408107.1, XP_007659164.1, XP_003909852.1, XP_002755355.1, NP_001126806.1, AAP92593.1, NP_001244199.1, BAA34427.1, XP_005063859.1, NP_599157.2, XP_004667761.1, XP_006900489.1, XP_006215013.1, XP_005408100.1, XP_007628718.1, XP_003514769.1, XP_006160935.1, XP_004683489.1, XP_003464329.1, XP_004921258.1, XP_003801447.1, XP_006167892.1, XP_004921305.1, AAH89619.1, XP_004706162.1, XP_003583243.1, EFB16804.1, XP_006728603.1, EPQ05185.1, XP_002709040.1, XP_006875861.1, XP_005408103.1, XP_004391425.1, EDL41477.1, XP_006772158.1, EGW06527.1, AAH 15294.1, XP_006772162.1, XP_005660939.1, XP_005352050.1, XP_006091901.1, XP_005878675.1, XP_004051323.1, EHA98803.1, XP_003779925.1, EDM 12924.1, XP_003421400.1, XP_006160939.1, XP_006160938.1, XP_006160937.1, XP_006160936.1, XP_005702185.1, XP_005313023.1, XP_003769190.1, XP_002714424.1, XP_004715051.1, XP_007661593.1, XP_004590594.1, ELK23975.1, XP_004674085.1, XP_004780477.1, XP_006231186.1, XP_003803573.1, XP_004803176.1, EFB16803.1, XP_006056694.1, XP_005441626.1, XP_005318647.1XP_004605904.1, XP_005862182.1, XP_003430682.1, XP_004780478.1, XP_005239278.1, XP_003897760.1, XP_007484121.1, XP_004892683.1, XP_004414286.1, XP_006927013.1, XP_003923145.1, XP_852587.2, AAP97178.1, EHH53105.1, XP_005408113.1, XP_002915474.1, XP_005377590.1, XP_527404.2, XP_005552830.1, XP_004044211.1, NP_001180996.1, XP_003513513.2, XP_001498599.2, XP_002746654.1, XP_005072349.1, XP_006149181.1, EAX04334.1, XP_003833230.1, XP_005216635.1, XP_003404197.1, XP_007523363.1, XP_007433902.1, XP_003254235.1, XP_004471242.1, XP_005216634.1, XP_006860675.1, XP_004771956.1, XP_006038833.1, NP_001138534.1, XP_007068532.1, XP_003510714.1, ERE87950.1, XP_003986313.1, XP_006728644.1, XP_004878099.1, XP_003468014.1, XP_007095614.1, XP_004648849.1, XP_004869795.1, XP_004018927.1, XP_005696454.1, XP_006201985.1, XP_005960697.1, XP_004813725.1, XP_005496926.1, ELR45088.1, XP_004696625.1, XP_005860982.1, XP_005911003.1, XP_006260162.1, EPQ04414.1, XP_006099775.1, NP_001138532.1, XP_006190795.1, XP_004649775.1, XP_004424497.1, XP_004390885.1, XP_005911004.1, XP_003777803.1, XP_004312259.1, XP_005529140.1, XP_005314582.1, XP_006926523.1, XP_006926522.1, XP_004683491.1, XP_003826680.1, XP_003215018.1, XP_003215087.1, EGW12611.1, XP_006113023.1, XP_006882182.1, XP_007425200.1, XP_006041342.1, NP_001138533.1, EMP27694.1, XP_007497753.1, XP_006034252.1,
or a variant thereof. Throughout this application, any data base code, unless specified to the contrary, refers to a sequence available from the NCBI data bases, more specifically the version online on 5th August 2013, and comprises, if such sequence is a nucleotide sequence, the polypeptide sequence obtained by translating the former.

In a preferred embodiment, the term "acyl amino acid", as used herein, refers to the product of the reaction catalysed by an amino acid-N-acyl transferase, more preferably a compound represented by the formula acyl-CO-NH-CHR-COOH, wherein R is the side chain of a proteinogenic amino acid, and wherein the term "acyl" refers to the acyl residue of a fatty acid. In a preferred embodiment of the present invention, the term "fatty acid", as used herein, means a carboxylic acid, preferably alkanoic acid, with at least 6, preferably 8, more preferably 10, most preferably 12 carbon atoms. In a preferred embodiment it is a linear fatty acid, in another embodiment it is branched. In a preferred embodiment it is a saturated fatty acid. In an especially preferred embodiment it is unsaturated. In another preferred embodiment it is a linear fatty acid with at least 12 carbon atoms comprising a double bond, preferably at position 9. In another preferred embodiment it is a simple unsaturated fatty acid having one double bond, which double bond is located at position 9 or 11. In the most preferred embodiment it is lauroleic acid (9-dodecenoic acid). In an especially preferred embodiment it is a fatty acid with 6, 7, 8, 9, 10, 11, 12, 13, 14, 15, 16, 17, 18, 19, 20, 21, 22, 23, 24, 25, 26, 27, 28, 29, 30 carbon atoms, preferably 12 carbon atoms.

Throughout this application, a formula referring to a chemical group that represents the dissociated or undissociated state of a compound capable of dissociating in an aqueous solution, comprises both the dissociated and the undissociated state and the various salt forms of the group. For example, the residue -COOH comprises both the protonated (-COOH) as well as the unprotonated ($-COO^-$) carboxylic acid.

The acyl-CoA substrate consumed in the inventive reaction may be purified from a cell, chemically synthesised or produced using an acyl-CoA synthetase, the latter being the preferred option. In a preferred embodiment, the term "acyl-CoA synthetase", as used herein, refers to an enzyme capable of catalysing the ATP-dependent conversion of a fatty acid and CoA to acyl CoA.

According to any aspect of the present invention, the term 'acyl-CoA synthetase' may refer to an acyl-CoA/ACP synthetase that may be capable of producing acyl glycinates and/or catalysing the following reaction:

$$\text{fatty acid} + \text{CoA/ACP} + \text{ATP} \rightarrow \text{acyl-CoA/ACP} + \text{ADP} + \text{Pi}$$

Examples of acyl-CoA/ACP synthetases may include EC 6.2.1.3, EC 6.2.1.10, EC 6.2.1.15, EC 6.2.1.20 and the like. The state of the art describes various methods to detect acyl-CoA synthetase activity. For example, the activity of an acyl-CoA synthetase may be assayed by incubating the sample of interest in 100 mM Tris-HCl at pH 8 in the presence of 250 $\mu$M lauroyl-CoA, 500 $\mu$M glycine and DTNB (5,5'-dithiobis-2-nitrobenzoic acid, also referred to as Ellman's reagent) and spectrophotometrically monitoring the absorbance at 410 nm following release of free thiol groups in the form of CoASH as the reaction progresses and reaction with Ellman's reagent.

Various acyl-CoA synthetases have been described in the state of the art, for example YP_001724804.1, WP_001563489.1 and NP_707317.1. In a preferred embodiment, the acyl-CoA synthetase comprises SEQ ID NO 6 or YP_001724804.1 or a variant thereof. The activity of an acyl-CoA synthetase may be assayed as described in the state of the art, for example Kang, Y., Zarzycki-Siek, J., Walton, C. B., Norris, M. H., and Hoang, T. T. (2010), Multiple FadD Acyl-CoA Synthetases Contribute to Differential Fatty Acid Degradation and Virulence in Pseudomonas aeruginosa, PLOS ONE 5 (10), e13557. Briefly, the amount of free thiol in the form of unreacted CoASH is determined by adding Ellmann's reagent and spectrophotometrically monitoring the absorbance at 410 nm, preferably in a reaction buffer comprising 150 mM Tris-HCl (pH 7.2), 10 mM $MgCl_2$, 2 mM EDTA, 0.1 % Triton X-100, 5 mM ATP, 0.5 mM coenzyme A (CoASH) and a fatty acid (30 to 300 mM).

In one example, the cell according to any aspect of the present invention may be genetically modified to overexpress at least the enzymes amino acid-N-acyl transferase and acyl-CoA synthetase. In particular, the cell may over express enzymes glycine N-acyl transferase and acyl-CoA/ACP synthetase.

The teachings of the present invention may not only be carried out using biological macromolecules having the exact amino acid or nucleic acid sequences referred to in this application explicitly, for example by name or accession number, or implicitly, but also using variants of such sequences. In a preferred embodiment, the term "variant", as used herein, comprises amino acid or nucleic acid sequences, respectively, that are at least 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner to the effect that the biological activity of the reference sequence or a molecule derived therefrom is preserved. The state of the art comprises algorithms that may be used to align two given nucleic acid or amino acid sequences and to calculate the degree of identity, see Arthur Lesk (2008), Introduction to bioinformatics, 3rd edition, Thompson et al., Nucleic Acids Research 22, 4637-4680, 1994, and Katoh et al., Genome Information, 16(1), 22-33, 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In a preferred embodiment, the term "variant", with regard to amino acid sequence, comprises, preferably in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In a preferred embodiment, the term "variant" of an amino acid sequence or nucleic acid sequence comprises, preferably in addition to the above degree of sequence identity, any active portion and/or fragment of the amino acid sequence or nucleic acid sequence, respectively, or any nucleic acid sequence encoding an active portion and/or fragment of an amino acid sequence. In a preferred embodiment, the term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease are capable of hydrolysing peptide bonds in polypeptides. In a preferred embodiment, the term "retains at least some of its essential biological activity", as used herein, means that the amino acid sequence in question has a biological activity exceeding and distinct from the background activity and the kinetic parameters characterising said activity, more specifically $k_{cat}$ and $K_M$, are preferably within 3, more preferably 2, most preferably one order of magnitude of the values displayed by the reference molecule with respect to a specific substrate. In a preferred embodiment, the term "variant" of a nucleic acid comprises nucleic acids the complementary strand of which hybridises, preferably under stringent conditions, to the reference or wild type nucleic acid. Stringency of hybridisation reactions is readily determinable by one of ordinary skilled in the art, and in generally is an empirical calculation dependent on probe length, washing temperature and salt concentration. In general longer probes require higher temperatures for proper annealing, while shorter probes need lower temperatures. Hybridisation generally depends on the ability of denatured DNA to reanneal to complementary strands are present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995), Current Protocols in Molecular Biology. John Wiley & Sons, Inc. Moreover, the person skilled take in the art may follow the instructions given in the manual "The DIG System Users Guide for Filter Hybridization", Boehringer Mannheim GmbH, Mannheim, Germany, 1993 and in Liebl et al. (International Journal of Systematic Bacteriology 41: 255-260 (1991) on how to identify DNA sequences by means of hybridisation. In a preferred embodiment, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50 °C - 68 °C, approximately 52 °C - 68 °C, approximately 54 °C - 68 °C, approximately 56 °C - 68 °C, approximately 58 °C - 68 °C, approximately 60 °C - 68 °C, approximately 62 °C - 68 °C, approximately 64 °C - 68 °C, approximately 66 °C - 68 °C. In a particularly preferred embodiment, the temperature is approximately 64 °C - 68 °C or approximately 66 °C - 68 °C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. In a preferred embodiment, the term "homologue" of a nucleic acid sequence, as used herein, refers to any nucleic acid sequence that encodes the same amino acid sequence as the reference nucleic acid sequence, in line with the degeneracy of the genetic code. If one or more of the enzymes used to practise the present invention is provided in the form of a cell, it is preferred that the cell has a reduced fatty acid degradation capacity. In a preferred embodiment, the term "having a reduced fatty acid degradation capacity", as used in herein, means that the respective cell degrades fatty acids, preferably those taken up from the environment, at a lower rate than a comparable cell or wild type cell having normal fatty acid degradation capacity would under identical conditions. In a preferred embodiment, the fatty acid degradation of such a cell is lower on account of deletion, inhibition or inactivation of at least one gene encoding an enzyme involved in the β-oxidation pathway. In a preferred embodiment of the present invention, at least one enzyme involved in the β-oxidation pathway has lost, in order of increasing preference, 5, 10, 20, 40, 50, 75, 90 or 99 % activity relative to the activity of the same enzyme under comparable conditions in the respective wild type microorganism. The person skilled in the art is familiar with various techniques that may be used to delete a gene encoding an enzyme or reduce the activity of such an enzyme in a cell, for example by exposition of cells to radioactivity followed by accumulation or screening of the resulting mutants, site-directed introduction of point mutations or knock out of a chromosomally integrated gene encoding for an active enzyme, as described in Sambrook/Fritsch/Maniatis (1989). In addition, the transcriptional repressor FadR may be over expressed to the effect that expression of enzymes involved in the β-oxidation pathway is repressed (Fujita, Y., Matsuoka, H., and Hirooka, K. (2007) Mol. Microbiology 66(4), 829-839). In a preferred embodiment, the term "deletion of a gene", as used herein, means that the nucleic acid sequence encoding said gene is modified such that the expression of active polypeptide encoded by said gene is reduced. For example, the gene may be deleted by removing in-frame a part of the sequence comprising the sequence encoding for the catalytic active centre of the polypeptide. Alternatively, the ribosome binding site may be altered such that the ribosomes no longer translate the corresponding RNA. Moreover, the person skilled in the art is able to routinely measure the activity of enzymes expressed by living cells using standard essays as described in enzymology text books, for example Cornish-Bowden (1995), Fundamentals of Enzyme Kinetics, Portland Press Limited, 1995.

Degradation of fatty acids is accomplished by a sequence of enzymatically catalysed reactions. First of all, fatty acids are taken up and translocated across the cell membrane *via* a transport/acyl-activation mechanism involving at least one outer membrane protein and one inner membrane-associated protein which has fatty acid-CoA ligase activity, referred to in the case of *E. coli* as FadL and FadD / FadK, respectively. Inside the cell, the fatty acid to be degraded is subjected to enzymes catalysing other reactions of the β-oxidation pathway. The first intracellular step involves the conversion of acyl-CoA to enoyl-CoA through acyl-CoA dehydrogenase, the latter referred to as FadE in the case of *E. coli.* The activity of an acyl-CoA dehydrogenase may be assayed as described in the state of art, for example by monitoring the concentration of NADH spectrophotometrically at 340 nm in 100 mM MOPS, pH 7.4, 0.2 mM Enoyl-CoA, 0.4 mM $NAD^+$. The resulting enoyl-CoA is converted to 3-ketoacyl-CoA *via 3*-hydroxylacyl-CoA through hydration and oxidation, catalysed by enoyl-CoA hydratase/3-hydroxyacyl-CoA dehydrogenase, referred to as FadB and FadJ in *E. coli.* Enoyl-CoA hydratase/3-hydroxyacyl-CoA dehydrogenase activity, more specifically formation of the product NADH may be assayed spectrophotometrically as described in the state of the art, for example as outlined for FadE. Finally, 3-ketoacyl-CoA thiolase, FadA and FadI in *E. coli,* catalyses the cleavage of 3-ketoacyl-CoA, to give acetyl-CoA and the input acyl-CoA shortened by two carbon atoms. The activity of ketoacyl-CoA thiolase may be assayed as described in the state of the art, for example in Antonenkov, V., D.. Van Veldhoven, P., P., Waelkens, E., and Mannaerts, G.P. (1997) Substrate specificities of 3-oxoacyl-CoA thiolase and sterol carrier protein 2/3-oxoacyl-coa thiolase purified from normal rat liver peroxisomes. Sterol carrier protein 2/3-oxoacyl-CoA thiolase is involved in the metabolism of 2-methyl-branched fatty acids and bile acid intermediates. J. Biol. Chem. 1997, 272:26023-26031. In a preferred embodiment, the term "a cell having a reduced fatty acid degradation capacity", as used herein, refers to a cell having a reduced capability of taking up and/or degrading fatty acids, preferably those having at least eight carbon chains. The fatty acid degradation capacity of a cell may be reduced in various ways. In a preferred embodiment, the cell has, compared to its wild type, a reduced activity of an enzyme involved in the β-oxidation pathway. In a preferred embodiment, the term "enzyme involved in the β-oxidation pathway", as used herein, refers to an enzyme that interacts directly with a fatty acid or a derivative thereof formed as part of the degradation of said fatty acid *via* the β-oxidation pathway the sequence of reactions effecting the conversion of a fatty acid to acetyl-CoA and the CoA ester of the shortened fatty acid, preferably by recognizing the fatty acid or derivative thereof as a substrate, and converts it to a metabolite formed as a part of the β-oxidation pathway. For example, the acyl-CoA dehydrogenase is an enzyme involved in the β-oxidation pathway as it interacts with fatty acid-CoA and converts fatty acid-CoA ester to enoyl-CoA, which is a metabolite formed as part of the β-oxidation. In a preferred embodiment, the term "enzyme involved in the β-oxidation pathway", as used herein, comprises any polypeptide from the group comprising acyl-CoA dehydrogenase, enoyl-CoA hydratase, 3-hydroxyacyl-CoA dehydrogenase and 3-keto-acyl-CoA thiolase. Subsequently, the acyl-CoA synthetase may catalyse the conversion a fatty acid to the CoA ester of a fatty acid, *i.e.* a molecule, wherein the functional group -OH of the carboxy group is replaced with -S-CoA, preferably for introducing said fatty acid into the β-oxidation pathway. For example, the polypeptides FadD and FadK in *E. coli* (access code: BAA15609.1) are acyl-CoA dehydrogenases. In a preferred embodiment, the term "acyl-CoA dehydrogenase", as used herein, is a polypeptide capable of catalysing the conversion of an acyl-CoA to enoyl-CoA, preferably as part of the β-oxidation pathway. For example, the polypeptide FadE in *E. coli* (access code: BAA77891.2) is an acyl-CoA dehydrogenase. In a preferred embodiment, the term "2,4-dienoyl-CoA reductase", as used herein, is a polypeptide capable of catalysing the conversion of the 2,4-dienoyl CoA from an unsaturated fatty acid into enoyl-CoA, preferably as part of the β-oxidation pathway. For example, the polypeptide FadH in *E. coli* is a 2,4-dienoyl-CoA reductase. In a preferred embodiment, the term "enoyl-CoA hydratase", as used herein, also referred to as 3-hydroxyacyl-CoA dehydrogenase, refers to a polypeptide capable of catalysing the conversion of enoyl-CoA to 3-ketoacyl-CoA through hydration and oxidation, preferably as part of the β-oxidation pathway. For example, the polypeptides FadB and FadJ in *E. coli* (access code: BAE77457.1) are enoyl-CoA hydratases. In a preferred embodiment, the term "ketoacyl-CoA thiolase", as used herein, refers to a polypeptide capable of catalysing the conversion of cleaving 3-ketoacyl-CoA, resulting in an acyl-CoA shortened by two carbon atoms and acetyl-CoA, preferably as the final step of the β-oxidation pathway. For example, the polypeptides FadA and FadI in *E. coli* (access code: AP009048.1) are ketoacyl-CoA thiolases. In one example, the cells according to any aspect of the present invention may be genetically modified to result in an increased activity of at least one amino acid N-acyl transferase in combination with increased activity of at least one acyl-CoA synthetase in combination with an increased activity of at least one transporter protein of the FadL and/or the AlkL. In particular, the cell according to any aspect of the present invention may be genetically modified to overexpress glycine N-acyl transferase, acyl-CoA/ACP synthetase, a transporter protein of the FadL and the AlkL compared to the wild type cell. These cells may be capable of producing acyl glycinates. In another example, the cell according to any aspect of the present invention may be genetically modified to:

- increase the expression of amino acid N-acyl transferase, acyl-CoA synthetase; and
- reduce activity of at least one enzyme selected from the group consisting of acyl-CoA dehydrogenase FadE, multifunctional 3-hydroxybutyryl-CoA epimerase, Δ3-cis-Δ 2-trans-enoyl-CoA isomerase, enoyl-CoA hydratase, 3-hydroxyacyl-CoA dehydrogenase FadB, 3-ketoacyl-CoA thiolase, electron-transfer flavoprotein

compared to the wild type cell.

In yet another example, the cells according to any aspect of the present invention may be genetically modified to result in:

- increase the expression of amino acid N-acyl transferase, acyl-CoA synthetase and at least one transporter protein of the FadL and/or the AlkL; and
- reduce activity of at least one enzyme selected from the group consisting of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein

compared to the wild type cell.

In particular, the acyl-CoA dehydrogenase FadE (EC 1.3.8.7, EC 1.3.8.8 or EC 1.3.8.9) may catalyse the reaction acyl-CoA + electron-transfer flavoprotein = trans-2,3-dehydroacyl-CoA + reduced electron-transfer flavoprotein; the multifunctional 3-hydroxybutyryl-CoA epimerase (EC 5.1.2.3), Δ3-cis-Δ2-trans-enoyl-CoA isomerase (EC 5.3.3.8), enoyl-CoA hydratase (EC 4.2.1.17) and 3-hydroxyacyl-CoA dehydrogenase (EC 1.1.1.35) FadB, may catalyse the reactions (S)-3-hydroxybutanoyl-CoA → (R)-3-hydroxybutanoyl-CoA, (3Z)-3-enoyl-CoA → (2E)-2-enoyl-CoA, (3S)-3-hydroxyacyl-CoA → trans-2-enoyl-CoA + H2O, (S)-3-hydroxyacyl-CoA + NAD+ = 3-oxoacyl-CoA + NADH + H+; 3-ketoacyl-CoA thiolase (EC 2.3.1.16), may catalyse the reaction acyl-CoA + acetyl-CoA → CoA + 3-oxoacyl-CoA; and electron-transfer flavoprotein (EC 1.5.5.1), may catalyse the following reaction: reduced electron-transferring flavoprotein + ubiquinone → electron-transferring flavoprotein + ubiquinol.

More in particular, the cell according to any aspect of the present invention may be genetically modified to increase the expression of glycine N-acyl transferase, acyl-CoA/ACP synthetase, a transporter protein of the FadL and the AlkL; and reduce activity of acyl-CoA dehydrogenase FadE, multifunctional 3-hydroxybutyryl-CoA epimerase, Δ3-cis- Δ 2-trans-enoyl-CoA isomerase, enoyl-CoA hydratase, 3-hydroxyacyl-CoA dehydrogenase FadB, 3-ketoacyl-CoA thiolase, electron-transfer flavoprotein, compared to the wild-type of the cell.

In one example, the cell according to any aspect of the present invention may be genetically modified to:

- increase the expression of amino acid N-acyl transferase, acyl-CoA synthetase; and
- reduce activity of at least one enzyme selected from the group consisting of glycine cleavage system H protein, glycine cleavage system P protein, glycine cleavage system L protein, glycine cleavage system T protein, threonine aldolase, serine hydroxylmethyltransferase.

In another example, the cell according to any aspect of the present invention may be genetically modified to:

- increase the expression of amino acid N-acyl transferase, acyl-CoA synthetase and at least one transporter protein of the FadL and/or the AlkL; and
- reduce activity of at least one enzyme selected from the group consisting of acyl-CoA dehydrogenase FadE, multifunctional 3-hydroxybutyryl-CoA epimerase, Δ3-*cis*-Δ *2-trans*-enoyl-CoA isomerase, enoyl-CoA hydratase, 3-hydroxyacyl-CoA dehydrogenase FadB, 3-ketoacyl-CoA thiolase, electron-transfer flavoprotein

compared to the wild type cell.

In yet another example, the cell according to any aspect of the present invention may be genetically modified to:

- increase the expression of amino acid N-acyl transferase, acyl-CoA synthetase and at least one transporter protein of the FadL and/or the AlkL;
- reduce activity of at least one enzyme selected from the group consisting of acyl-CoA dehydrogenase FadE, multifunctional 3-hydroxybutyryl-CoA epimerase, Δ3-cis-Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein; and
- reduce activity of at least one enzyme selected from the group consisting of glycine cleavage system H protein, glycine cleavage system P protein, glycine cleavage system L protein, glycine cleavage system T protein, threonine aldolase, serine hydroxylmethyltransferase

compared to the wild type cell.

In particular, the cell according to any aspect of the present invention may be genetically modified to increase the expression of glycine N-acyl transferase, acyl-CoA/ACP synthetase, a transporter protein of the FadL and the AlkL; reduce activity of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein; and reduce activity of glycine cleavage system H protein, glycine cleavage system P protein, glycine cleavage system L protein, glycine cleavage system T protein, threonine aldolase, serine hydroxylmethyltransferase, compared to the wild-type of the cell.

More in particular, the glycine cleavage system H protein, carrying lipoic acid and interacts with the glycine cleavage system proteins P, L and T; the glycine cleavage system P protein (EC 1.4.4.2), catalyses the reaction glycine + [glycine-cleavage complex H protein]-N(6)-lipoyl-L-lysine → [glycine-cleavage complex H protein]-S-aminomethyl-N(6)-dihydrolipoyl-L-lysine + CO2; glycine cleavage system L protein (EC 1.8.1.4), catalyses the reaction protein N6-(dihydrolipoyl)lysine + NAD+ → protein N6-(lipoyl)lysine + NADH + H+; glycine cleavage system T protein (EC 2.1.2.10), catalyses the reaction [protein]-S8-aminomethyldihydrolipoyllysine + tetrahydrofolate → [protein]-dihydrolipoyllysine + 5,10-methylenetetrahydrofolate + NH3; threonine aldolase (EC 4.2.1.48), catalyses the reaction L-threonine → glycine + acetaldehyde; serine hydroxylmethyltransferase (EC 2.1.2.1), catalyses the reaction 5,10-methylenetetrahydrofolate + glycine + $H_2O$ + tetrahydrofolate + L-serine.

In one example, the cell according to any aspect of the present invention may be genetically modified to increase the expression of amino acid N-acyl transferase, acyl-CoA synthetase, and a genetic modification in the cell capable of producing at least one fatty acid from at least one carbohydrate. A list of non-limiting genetic modification to enzymes or enzymatic activities is provided below in Table 1. The cells according to any aspect of the present invention may comprise a combination of genetic modification that produce fatty acids and convert the fatty acids to N-acyl amino acids.

In particular, the cell according to any aspect of the present invention may be genetically modified to increase the expression of amino acid N-acyl transferase, acyl-CoA synthetase and comprise any of the genetic modifications listed in Table 1. More in particular, the cell may be genetically modified to increase the expression of N-acyl transferase, acyl-CoA synthetase, a transporter protein of the FadL and the AlkL and comprise any of the genetic modifications listed in Table 1. Even more in particular, the cell may be genetically modified to increase the expression of N-acyl transferase, acyl-CoA synthetase, a transporter protein of the FadL and the AlkL, reduce activity of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein; reduce activity of glycine cleavage system H protein, glycine cleavage system P protein, glycine cleavage system L protein, glycine cleavage system T protein, threonine aldolase, serine hydroxylmethyltransferase, and comprise any of the genetic modifications listed in Table 1.

In one example, the cell according to any aspect of the present invention may be genetically modified to increase the expression of amino acid N-acyl transferase, acyl-CoA/ACP synthetase and decrease the expression of at least one enzyme selected from the group consisting of β-ketoacyl-ACP synthase I, 3-oxoacyl-ACP-synthase I, Malonyl-CoA-ACP transacylase, enoyl ACP reductase, and β-ketoacyl-ACP synthase III. In particular, the genetic modification in the cell according to any aspect of the present invention may comprise an increase in the expression of amino acid N-acyl transferase, acyl-CoA/ACP synthetase and a decrease in the expression of β-ketoacyl-ACP synthase I, 3-oxoacyl-ACP-synthase I, Malonyl-CoA-ACP transacylase, enoyl ACP reductase, and β-ketoacyl-ACP synthase III.

The inventive teachings may be carried out using a wide range of cells. In a preferred embodiment, the term "cell", as used herein, refers to any permanently unicellular organism comprising bacteria archaea, fungi, algae and the like. In a preferred embodiment, the cell is a bacterial cell, more preferably one from the group comprising *Pseudomonas, Corynebacterium, Bacillus* and *Escherichia,* most preferably *Escherichia coli.* In another preferred embodiment, the cell is a lower eukaryote, more preferably a fungus from the group comprising *Saccharomyces, Candida, Pichia, Schizosaccharomyces* and *Yarrowia,* and is most preferably *Saccharomyces cerevisiae.* The microorganism may be an isolated cell, in other words a pure culture of a single strain, or may comprise a mixture of at least two strains. Biotechnologically relevant cells are commercially available, for example from the American Type Culture Collection (ATCC) or the German Collection of Microorganisms and Cell Cultures (DSMZ). Particles for keeping and modifying cells are available from the prior art, for example Sambrook/Fritsch/Maniatis (1989): Molecular cloning - A Laboratory Manual, Cold Spring Harbour Press, 2nd editi*on,* Fuchs/Schlegel (2007), Allgemeine Mikrobiologie, 2008, Georg Thieme Verlag.

Although the inventive teachings may be practiced using wild type cells, it is preferred that at least one of the enzymes involved, in particular at least one or all from the group comprising amino acid amino acid N-acyl-transferase, acyl-CoA synthetase and acyl-CoA thioesterase, is recombinant. In a preferred embodiment, the term "recombinant" as used herein, refers to a molecule or is encoded by such a molecule, preferably a polypeptide or nucleic acid that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

Whether or not a nucleic acid molecule, polypeptide, more specifically an enzyme required to practice the invention, is recombinant or not has not necessarily implications for the level of its expression. However, it is preferred that one or more recombinant nucleic acid molecules, polypeptides or enzymes required to practice the invention are overexpressed. In a preferred embodiment, the term "overexpressed", as used herein, means that the respective polypeptide encoded or expressed is expressed at a level higher or at higher activity than would normally be found in the cell under identical conditions in the absence of genetic modifications carried out to increase the expression, for example in the respective wild type cell. The person skilled in the art is familiar with numerous ways to bring about overexpression. For example, the nucleic acid molecule to be overexpressed or encoding the polypeptide or enzyme to be overexpressed may be placed under the control of a strong inducible promoter such as the lac promoter. The state of the art describes standard plasmids that may be used for this purpose, for example the pET system of vectors exemplified by pET-3a (commercially available from Novagen). Whether or not a nucleic acid or polypeptide is overexpressed may be determined by way of quantitative PCR reaction in the case of a nucleic acid molecule, SDS polyacrylamide electrophoreses, Western blotting or comparative activity assays in the case of a polypeptide. Genetic modifications may be directed to transcriptional, translational, and/or post-translational modifications that result in a change of enzyme activity and/or selectivity under selected and/or identified culture conditions. Thus, in various examples of the present invention, to function more efficiently, a microorganism may comprise one or more gene deletions. Gene deletions may be accomplished by mutational gene deletion approaches, and/or starting with a mutant strain having reduced or no expression of one or more of these enzymes, and/or other methods known to those skilled in the art.

Besides, any of the enzymes required to practice the inventive teachings, in particular at least one or all from the group comprising N-acyl-transferase, acyl-CoA synthetase and acyl-CoA thioesterase, may be an isolated enzyme.

In any event, any enzyme required to practice the present invention is preferably used in an active state and in the presence of all cofactors, substrates, auxiliary and/or activating polypeptides or factors essential for its activity. In a preferred embodiment, the term "isolated", as used herein, means that the enzyme of interest is enriched compared to the cell in which it occurs naturally. Whether or not an enzyme is enriched may be determined by SDS polyacrylamide electrophoresis and/or activity assays. For example, the enzyme of interest may constitute more than 5, 10, 20, 50, 75, 80, 85, 90, 95 or 99 percent of all the polypeptides present in the preparation as judged by visual inspection of a polyacrylamide gel following staining with Coomassie blue dye.

If a cell expressing an amino acid-N-acyl-transferase and an acyl-CoA synthetase and having a reduced fatty acid degradation capacity is used, it is preferred that the cell be capable of making proteinogenic amino acids and/or fatty acids. In a preferred embodiment, the term "proteinogenic amino acid", as used herein, refers to an amino acid selected from the group comprising alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine. Proteinogenic amino acids and fatty acids are synthesized as part of the primary metabolism of many wild type cells in reactions and using enzymes that have been described in detail in biochemistry textbooks, for example Jeremy M Berg, John L Tymoczko, and Lubert Stryer, Biochemistry, 5th edition, W. H. Freeman, 2002.

In a preferred embodiment, the cell used to practice the inventive teachings expresses an acyl-CoA thioesterase. In a more preferred embodiment, the term "acyl-CoA thioesterase", as used herein, refers to an enzyme capable of hydrolyzing acyl-CoA. In a preferred embodiment the acyl-CoA thioesterase comprises a sequence from the group comprising SEQ ID NO 1, AEM72521.1 and AAC49180.1 or a variant thereof, more preferably SEQ ID NO 1 or a variant thereof. The activity of acyl-CoA thioesterase may be assayed using various assays described in the state of the art. Briefly, the reaction of Ellman's reagent, which reacts with free thiol groups associated with CoASH formed upon hydrolysis of acyl-CoA may be detected by spectophotometrically monitoring absorbance at 412 nm.

In a preferred embodiment, the term "contacting", as used herein, means bringing about direct contact between the amino acid, the acyl CoA and the amino acid-N-acyl transferase or the inventive cell and/or any other reagents required to carry out the inventive teachings, preferably in an aqueous solution. For example, the cell, the amino acid and the acyl CoA may not be in different compartments separated by a barrier such as an inorganic membrane. If the amino acid or fatty acid is soluble and may be taken up by the cell or can diffuse across biological membranes, it may simply be added to the inventive cell in an aqueous solution. In case it is insufficiently soluble, it may be solved in a suitable organic solvent prior to addition to the aqueous solution. The person skilled in the art is able to prepare aqueous solutions of amino acids or fatty acids having insufficient solubility by adding suitable organic and/or polar solvents. Such solvents are preferably provided in the form of an organic phase comprising liquid organic solvent. In a preferred embodiment, the organic solvent or phase is considered liquid when liquid at 25 °C and standard atmospheric pressure. In another preferred embodiment, a fatty acid is provided in the form of a fatty acid ester such as the respective methyl or ethyl ester. For example, the fatty acid laurate may be solved in lauric acid methyl ester as described in EP11191520.3. According to the present invention, the compounds and catalysts may be contacted *in vitro,* i.e. in a more or less enriched or even purified state, or may be contacted *in situ,* i.e. they are made as part of the metabolism of the cell and subsequently react inside the cell.

The term "an aqueous solution" comprises any solution comprising water, preferably mainly water as solvent that may be used to keep the inventive cell, at least temporarily, in a metabolically active and/or viable state and comprises, if such is necessary, any additional substrates. The person skilled in the art is familiar with the preparation of numerous aqueous solutions, usually referred to as media that may be used to keep inventive cells, for example LB medium in the case of *E. coli.* It is advantageous to use as an aqueous solution a minimal medium, *i.e.* a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium.

It is a particular strength of the present invention that not only saturated fatty acids, but also unsaturated fatty acids may be converted to acyl amino acids. In case the end product sought-after is to comprise a higher yield of saturated acyl residues than is present after step b), it may be possible to complement the process by hydrogenating the acyl residues of the acyl amino acids following step b). In this case, the inventive composition according to the fifth aspect of the present invention, which composition comprises a mixture of acyl amino acids having unsaturated acyl residues, constitutes an obligatory intermediate which may be converted to the final product, i.e. a mixture of acyl amino acids having saturated acyl residues. The hydrogenation may be carried out according to various state of the art processes, for example those described in US5734070. Briefly, the compound to be hydrogenated may be incubated at 100 °C in the presence of hydrogen and a suitable catalyst, for example a nickel catalyst on silicon oxide as a support.

The fatty acids that are to be converted to acyl amino acids may be produced by the cell according to the present invention. In one example, the very cell that produces the acyl amino acids is capable of producing the fatty acids from which the acyl amino acids are produced. In particular, the cells may be genetically modified to be able to produce fatty acids. In one example, the genetic modification may be to decrease a specific enzymatic activity and this may be done by a gene disruption or a genetic modification. The genetic modification may also increase a specific enzymatic activity. In particular, the genetic modification may increase microbial synthesis of a selected fatty acid or fatty acid derived chemical product above a rate of a control or wild type cell. This control or wild type cell may lack this genetic modification to produce a selected chemical product. A list of non-limiting genetic modification to enzymes or enzymatic activities is provided below in Table 1.

Table 1. Examples of genetic modifications in cells of microorganisms for production of fatty acids

| Genetic Modifications | | | |
|---|---|---|---|
| ENZYME FUNCTION | E.C. CLASSIFICATION No. | GENE NAME IN *E. COLI* | COMMENTS |
| Glucose transporter | N/A | galP | Increase function |
| Pyruvate dehydrogenase E1p | 1.2.4.1 | accE | Increase function |
| Lipoate acetyltransferase/dihydrolipoamide acetyltransferase | 2.3.1.12 | aceF | Increase function |
| Pyruvalte dehydrogenase E3 (lipoamide dehydrogenase) | 1.8.1.4 | Ipd | Increase function or alter such as by mutation to increase resistance to NADH inhibition, |
| Lactate dehydrogenase | 1.1.1.28 | IdhA | Decrease function, including by mutation |
| Pyruvate formate lyase (B "inactive") | 1..3.1._ | pflB | Decrease function, |
| Pyruvate oxidase | 1.2.2.2 | poxB | Decrease function, including by mutation |
| Phosphate acetyltransferase | 2.3.1.8 | Pta | Decrease function, including by mutation |
| acetate kinase | 2.7.2.15 2.7.2.1 | ackA | Decrease function, including by mutation |
| methylglyoxal synthase | 4.2.3.3 | mgsA | Decrease function, including by mutation |
| Heat stable, histidyl Phosphorylatable protein (of PTS) | N/A | ptsH (HPr) | Decrease function, including by mutation |
| Phosphoryl transfer protein (of PTS) | N/A | ptsI | Decrease function, including by mutation |
| Polypeptide chain (of PTS) | N/A | Crr | Decrease function, including by mutation |
| 3-oxoacyl-ACP synthase I<br>3-oxoacyl-ACP synthase II | 2.3.1.1 79<br>2.3.1.41 | fabF | Decrease function, including by mutation |
| β-ketoacyl-ACP synthase I, 3-oxoacyl-ACP-synthase I | 2.3.1.41<br>2.3.1. | fabB | Decrease function, including by mutation |
| Malonyl-CoA-ACp transacylase | 2.3.1.39 | fabD | Decrease function, including by mutation |
| enoyl acyl carrier protein reductase | 1.3.1.9. 1.3.1.10 | fabI | Decrease function, including by mutation |
| β-ketoacyl carrier protein synthase III | 2.3.1.1 80 | fabH | Decrease function, including by mutation |
| Carboxyl transferase subunit α subunit | 6.4.1.2 | accA | Increase function |
| Biotin carboxyl carrier protein | 6.4.1.2 | accB | Increase function |
| Biotin carboxylase subunit | 6.3.4.14 | accC | Increase function |
| Carboxyl transferase subunit β subunit | 6.4.1.2 | accD | Increase function |
| long chain fatty acyl thioesterase I | 3.1.2.2. 3.1.1.5 | tesA | Increase function as well as alter by mutation to express in cytoplasm or deletion |
| acyl-CoA synthase | 2.3.1.86 | fadD | Decrease via deletionor mutation |

(continued)

| Genetic Modifications | | | |
|---|---|---|---|
| ENZYME FUNCTION | E.C. CLASSIFICATION No. | GENE NAME IN *E. COLI* | COMMENTS |
| acetate CoA-transferase | 2.8.3.8 | atoD | Decrease via deletion or mutation |
| acetate CoA-transferase | 2.8.3.8 | atoA | Decrease via deletion or mutation |
| Transporter | N/A | atoE | Decrease via deletion or mutation |
| acetyl-CoA acetylransferase | 2.3.1.9 | | or mutation |
| pantothenate kinase | 2.7.1.33 | coaA | Increase via expression or feedback resistant mutation |
| lactose repressor | N/A | lacI | Decrease via deletion or mutation |
| $\gamma$-glutamyl-$\gamma$-aminobutyraldehyde dehydrogenase | 1.2.1. | puuC | Decrease via deletion or mutation |
| malate synthase A | 2.3.3.9 | accA | Decrease via deletion or mutation |
| isocitrate lyase | 4.1.3.1 | aceA | Decrease via deletion or mutation |
| isocitrate dehydrogenase phosphatase/isocitrate dehydrogenase kinase | 2.7.11.5. | aceK | Decrease via deletion or mutation |
| pyruvate formate-lyase deactivase | 1.2.1.10 1.1.1.1 | adhE | Decrease via deletion or mutation |
| aldehyde dehydrogenase A, NAD-linked | 1.2.1.21 1.2.1.22 | aldA | Decrease via deletion or mutation |
| acetaldehyde dehydrogenase | 1.2.1.4 | aldB | Decrease via deletion or mutation |
| Lambda phage DE3 lysogen | N/A | $\lambda$DE3 | Increase |
| T7 mRNA polymerase | N/A | T7pol | Increase |
| trigger factor | 5.2.1.8 | tig | Decrease via deletion or mutation |
| 3-ketoacyl-CoA thiolase | 2.3.1.16 | fadA | Increase |
| doddecenoyl.CoA $\delta$-isomerase, enoyl-CoA hydratase, 3-hydroxybutyryl-CoA epimerase, 3-hydroxyacyl-CoA dehydrogenase | 5.3.3.8 1.1.1.35 5.1.2.3 4.2.1.17 | fadB | Increase |
| Sucrose permease | N/A | cscB | Increase |
| Invertase | 3.2.1.26 | cscA | Increase |
| fructokinase | 2.7.1.4 | cscK | Increase |
| carbonic anhydrase | 4.2.1.1 | cynT | Increase |
| carbonic anhydrase | 4.2.1.1 | can | Increase |
| pyridine nucleotide transhydrogenase | 1.6.1.2 | pntAB | Increase |
| pyridine nucleotide transhydrogenase | 1.6.1.1 | udhA | Increase |
| acyl-CoA thioesterase | 3.1.2.20 3.1.2.2 | yciA | Increase and or decrease |
| thioesterase II | 3.1.2.20 3.1.2.2 | tesB | Increase and or decrease |
| thioesterase III | 3.1.2.— | fadM | Increase and or decrease |
| hydroxyphenylacetyl-CoA thioesterase | N/A | paaI | Increase and or decrease |
| esterase/thioesterase | 3.1.2.28 | ybgC | Increase and or decrease |

(continued)

| Genetic Modifications | | | |
|---|---|---|---|
| ENZYME FUNCTION | E.C. CLASSIFICATION No. | GENE NAME IN *E. COLI* | COMMENTS |
| proofreading thioesterase in enterobactin biosynthesis | | entH | Increase and or decrease |
| acetoacetyl-CoA synthase | 2.3.1.194 | npth07 | Increase |
| 3-ketoacyl-CoA synthase/elongase | 2.3.1 | Elo1 | Increase |
| 3-ketoacyl-CoA synthase/elongase | 2.3.1 | Elo2 | Increase |
| 3-Hydroxybutyryl-CoA dehydrogenase | 1.1.1.157 | hbd | Increase |
| 3-oxoacyl-coA reductase | 1.1.1.100 | fabG | Increase |
| enoyl-CoA hydratase | 4.2.1.17 | crt | Increase |
| enoyl-CoA hydratase | 4.2.1.17 | ech2 | Increase |
| Trans-2-enoyl-reductase | 1.3.1.9 | ter | Increase |
| thioesterase | 3.1.2.20 | paaI | Decrease |
| E.C. No. - "Enzyme Commission number" | | | |

In one example, the genetic modification in a cell of the present invention may include any of the enzymes above in combination with a genetic modification in at least one enzyme selected from the group consisting of β-ketoacyl-ACP synthase I, 3-oxoacyl-ACP-synthase I, Malonyl-CoA-ACP transacylase, enoyl ACP reductase, and β-ketoacyl-ACP synthase III. In particular, the genetic modification in the cell according to any aspect of the present invention may comprise any of the enzymes listed in Table 1 in combination with the following enzymes β-ketoacyl-ACP synthase I, 3-oxoacyl-ACP-synthase I, Malonyl-CoA-ACP transacylase, enoyl ACP reductase, and β-ketoacyl-ACP synthase III.

Accordingly, the cells and methods of the present invention may comprise providing a genetically modified microorganism that comprises both a production pathway to a fatty acid or fatty acid derived product, and a modified polynucleotide that encodes an enzyme of the malonyl-ACP dependent fatty acid synthase system that exhibits reduced activity, so that utilization of malonyl-CoA shifts toward the production pathway compared with a comparable (control) microorganism lacking such modifications. The methods involve producing the chemical product using a population of such genetically modified microorganism in a vessel, provided with a nutrient media. Other genetic modifications described herein, to other enzymes, such as acetyl-CoA carboxylase and/or NADPH-dependent transhydrogenase, may be present in some such examples. Providing additional copies of polynucleotides that encode polypeptides exhibiting these enzymatic activities is shown to increase a fatty acid or fatty acid derived product production. Other ways to increase these respective enzymatic activities is known in the art and may be applied to various examples of the present invention.

Also, without being limiting, a first step in some multi-phase method embodiments of making a fatty acid may be exemplified by providing into a vessel, such as a culture or bioreactor vessel, a nutrient media, such as a minimal media as known to those skilled in the art, and an inoculum of a genetically modified microorganism so as to provide a population of such microorganism, such as a bacterium, and more particularly a member of the family *Enterobacteriaceae,* such as *E. coli,* where the genetically modified microorganism comprises a metabolic pathway that converts malonyl-CoA to a fatty acid. This inoculum is cultured in the vessel so that the cell density increases to a cell density suitable for reaching a production level of a fatty acid or fatty acid derived product that meets overall productivity metrics taking into consideration the next step of the method. In various alternative embodiments, a population of these genetically modified microorganisms may be cultured to a first cell density in a first, preparatory vessel, and then transferred to the noted vessel so as to provide the selected cell density. Numerous multi-vessel culturing strategies are known to those skilled in the art. Any such embodiments provide the selected cell density according to the first noted step of the method.

Also without being limiting, a subsequent step may be exemplified by two approaches, which also may be practiced in combination in various embodiments. A first approach provides a genetic modification to the genetically modified microorganism such that its enoyl-ACP reductase enzymatic activity may be controlled. As one example, a genetic modification may be made to substitute a temperature-sensitive mutant enoyl-ACP reductase (e.g., $fabI^{TS}$ in *E. coli*) for the native enoyl-ACP reductase. The former may exhibit reduced enzymatic activity at temperatures above 30° C. but normal enzymatic activity at 30° C., so that elevating the culture temperature to, for example to 34° C., 35° C., 36° C., 37° C. or even 42° C., reduces enzymatic activity of enoyl-ACP reductase. In such case, more malonyl-CoA is converted to a fatty acid or fatty acid derived product or another chemical product than at 30° C., where conversion of malonyl-CoA to fatty acids is not impeded by a less effective enoyl-ACP reductase.

Other genetic modifications that may be useful in the production of fatty acids and/or amino acids may be included in the cell. For example, the ability to utilize sucrose may be provided, and this would expand the range of feed stocks that can be utilized to produce a fatty acid or fatty acid derived product or other chemical products. Common laboratory and industrial strains of *E. coli,* such as the strains described herein, are not capable of utilizing sucrose as the sole carbon source. Since sucrose, and sucrose-containing feed stocks such as molasses, are abundant and often used as feed stocks for the production by microbial fermentation, adding appropriate genetic modifications to permit uptake and use of sucrose may be practiced in strains having other features as provided herein. Various sucrose uptake and metabolism systems are known in the art (for example, U.S. Pat. No. 6,960,455).

Also, genetic modifications may be provided to add functionality for breakdown of more complex carbon sources, such as cellulosic biomass or products thereof, for uptake, and/or for utilization of such carbon sources. For example, numerous cellulases and cellulase-based cellulose degradation systems have been studied and characterized (Beguin, P and Aubert, J-P (1994) FEMS Microbial. Rev. 13: 25-58; Ohima, K. et al. (1997) Biotechnol. Genet. Eng. Rev. 14: 365414).

In some examples, genetic modifications increase the pool and availability of the cofactor NADPH, and/or, consequently, the NADPH/$NADP^+$ ratio may also be provided. For example, in *E. coli,* this may be done by increasing activity, such as by genetic modification, of one or more of the following genes: *pgi* (in a mutated form), *pntAB,* overexpressed, *gapA:gapN* substitution/replacement, and disrupting or modifying a soluble transhydrogenase such as *sthA,* and/or genetic modifications of one or more of *zwf, gnd,* and *edd.*

Any such genetic modifications may be provided to species not having such functionality, or having a less than desired level of such functionality. More generally, and depending on the particular metabolic pathways of a microorganism selected for genetic modification, any subgroup of genetic modifications may be made to decrease cellular production of fermentation product(s) selected from the group consisting of acetate, acetoin, acetone, acrylic, malate, fatty acid ethyl esters, isoprenoids, glycerol, ethylene glycol, ethylene, propylene, butylene, isobutylene, ethyl acetate, vinyl acetate, other acetates, 1,4-butanediol, 2,3-butanediol, butanol, isobutanol, sec-butanol, butyrate, isobutyrate, 2-OH-isobutryate, 3-OH-butyrate, ethanol, isopropanol, D-lactate, L-lactate, pyruvate, itaconate, levulinate, glucarate, glutarate, caprolactam, adipic acid, propanol, isopropanol, fusel alcohols, and 1,2-propanediol, 1,3-propanediol, formate, fumaric acid, propionic acid, succinic acid, valeric acid, and maleic acid. Gene deletions may be made as disclosed generally herein, and other approaches may also be used to achieve a desired decreased cellular production of selected fermentation products.

The inventions is further illustrated by the following figures and non-limiting examples from which further embodiments, aspects and advantages of the present invention may be taken.

**Figure. 1** depicts a total ion chromatogram of the 48 h sample from the *E. coli* W3110 ΔfadE pJ294{Ptac}[synUcTE] / pCDF{Ptac}[hGLYAT2(co_Ec)_fadD_Ec] fermentation.

**Figure. 2** depicts a total ion chromatogram of the 48 h sample from the *E. coli* W3110 ΔfadE pJ294{Ptac}[synUcTE] / pCDF{Ptac}[hGLYAT3(co_Ec)_fadD_Ec] fermentation.

**Figure. 3** depicts a total ion chromatogram of the 48 h sample from the *E. coli* W3110 ΔfadE pJ294{Ptac}[synUcTE] / pCDFDuet-1 fermentation (negative control).

**Examples**

<u>Sequence ID NOs:</u>

Throughout this application a range of SEQ ID NOs are used. These are shown in Table 2 below.

<u>Table 2. Sequences used in the examples.</u>

| SEQ ID NO: | Comment |
|---|---|
| 1 | *Umbellularia californica synUcTE* (an acyl-CoA thioesterase) gene (codon-optimized) |
| 2 | tac promoter |
| 3 | Vector pJ294[Ptac-synUcTE], see example 1 |
| 4 | *Homo sapiens* genes hGLYAT2 (an amino acid N-acyl transferase) |
| 5 | *Homo sapiens* genes hGLYAT3 (another amino acid N-acyl transferase) |

(continued)

| SEQ ID NO: | Comment |
|---|---|
| 6 | *Escherichia coli fadD* (an acyl-CoA synthetase) |
| 7 | Vector pCDF[atfA1_Ab(co_Ec)-fadD_Ec], see example 2 |
| 8 | Vector pCDF{Ptac}[hGLYAT2(co_Ec)-fadD_Ec], see example 2 |
| 9 | Vector pCDF{Ptac}[hGLYAT3(co_Ec)-fadD_Ec], see example 2 |
| 10 | alkL (an importer facilitating transport of hydrophobic acyl across cell membranes) gene, see example 3 |
| 11 | lacuv5 promoter, see example 3 |
| 12 | Vector pCDF[alkLmod1], see example 3 |
| 13 | Vector pCDF{Ptac}[hGLYAT2(co_Ec)-fadD_Ec]{Placuv5}[alkLmod1], see example 3 |
| 14 | Vector pET-28b, see example 10 |
| 15 | Vector pET-28b{Ptac}[hGLYAT2(co_Ec)], see example 10 |
| 16 | pET-28b{Ptac}[hGLYAT3(co_Ec)], see example 10 |

Example 1

**Generation of an expression vector for the *Umbellularia californica* gene *synUcTE***

To generate an expression vector for the *Umbellularia californica synUcTE* gene (SEQ ID No. 1), which encodes the *Umbellularia californica* acyl CoA-thioesterase, this gene was codon-optimized for expression in *Escherichia coli.* The gene was synthesized together with a *tac* promoter (SEQ ID No. 2), and, simultaneously, one cleavage site was introduced upstream of the promoter and one cleavage site downstream of the terminator. The synthesized DNA fragment $P_{tac}$-synUcTE was digested with the restriction endonucleases *BamH*I and *Not*I and ligated into the correspondingly cut vector pJ294 (DNA2.0 Inc., Menlo Park, CA, USA). The finished *E. coli* expression vector was referred to as pJ294[Ptac-synUcTE] (SEQ ID No. 3).

Example 2

**Generation of vectors for coexpression of *Escherichia coli fadD* with either the *Homo sapiens* genes hGLYAT3 and hGLYAT2**

To generate vectors for the coexpression of the *Homo sapiens* genes hGLYAT2 (SEQ ID No. 4) or hGYLAT3 (SEQ ID No. 5), which encodes human glycine-N-acyltransferase, with *Escherichia coli fadD* (SEQ ID No. 6), which encodes the *E. coli* acyl-CoA synthetase, the genes hGLYAT2 and hGLYAT3 were codon-optimized for expression in *Escherichia coli* and synthesized. The synthesized DNA fragments were digested with the restriction endonucleases SacII and *Eco*47III and ligated into the correspondingly cut pCDF[atfA1_Ab(co_Ec)-fadD_Ec] (SEQ ID No. 7) with removal of the *aft*A1 gene. The sequence segments which were additionally removed in this process were cosynthesized during gene synthesis. The vector is a pCDF derivative which already comprises a synthetic tac promoter (SEQ ID No. 2) and the *Escherichia coli fadD* gene. The resulting expression vectors were named pCDF{Ptac}[hGLYAT2(co_Ec)-fadD_Ec] (SEQ ID No. 8) and pCDF{Ptac}[hGLYAT3(co_Ec)-fadD_Ec] (SEQ ID No. 9).

Example 3

**Generation of vectors for the coexpression of the *Homo sapiens* hGLYAT2, *Escherichia coli* fadD and *Pseudomonas putida* alkL genes**

To generate vectors for the coexpression of the hGLYAT2 genes with a modified *Pseudomonas putida* alkL gene, which encodes AlkL, an outer membrane protein that facilitates the import of hydrophobic substrates into a cell, the alkL gene (SEQ ID No. 10) was amplified together with the lacuv5 promoter (SEQ ID No. 11) from the plasmid pCDF[alkLmod1] (SEQ ID No. 12) by means of sequence-specific oligonucleotides. The PCR products were cleaved with the restriction endonucleases *Bam*HI and *Nsi*I and ligated into the correspondingly cleaved vector pCDF{Ptac}[hGLYAT2(co_Ec)-fadD_Ec] (SEQ ID No. 8). The correct insertion of the target genes was checked by restriction analysis and the authenticity of the introduced genes was verified by DNA sequencing. The resulting expression vector was named pCDF{Ptac}[hGLYAT2(co_Ec)-fadD_Ec]{Placuv5}[alkLmod1] (SEQ ID No. 13).

Example 4

**Generation of an *E. coli* strain with deletion in the *fadE* gene, which strain overexpresses the *Umbellularia californica synUcTE, Escherichia coli fadD* and *Homo sapiens* hGLYAT2 and hGLYAT3 genes**

To generate *E. coli* strains which coexpress the *Umbellularia californica synUcTE* in combination with the *Escherichia coli fadD* and *Homo sapiens* hGLYAT2 or *Homo sapiens* hGLYAT3 genes, the strain *E.coli* W3110 ΔfadE was transformed with the plasmids pJ294{Ptac}[synUcTE] and pCDF{Ptac}[hGLYAT2(co_Ec)_fadD_Ec] or pCDF{Ptac}[hGLYAT3(co_Ec)_fadD_Ec] by means of electroporation and plated onto LB-agar plates supplemented with spectinomycin (100 μg/ml) and ampicillin (100 μg/ml). Transformants were checked for the presence of the correct plasmids by plasmid preparation and analytic restriction analysis. The strains *E.coli* W3110 ΔfadE pJ294{Ptac}[synUcTE] / pCDF{Ptac}[hGLYAT2(co_Ec)_fadD_Ec] and *E.coli* W3110 ΔfadE pJ294{Ptac}[synUcTE] / pCDF{Ptac}[hGLYAT3(co_Ec)_fadD_Ec] were generated thus.

Example 5

**Generation of an *E.coli* strain with deletion in the *fadE* gene, which strain overexpresses the *Escherichia coli fadD* and either *Homo sapiens* hGLYAT2 or hGLYAT3 genes**

To generate *E. coli* strains which overexpress the *Escherichia coli fadD* gene in combination with the *Homo sapiens* hGLYAT2 or hGLYAT3 genes, electrocompetent cells of *E. coli* strain W3110 ΔfadE were generated. *E.coli* W3110 ΔfadE was transformed with the plasmid pCDF{Ptac}[hGLYAT2(co_Ec)-fadD_Ec]{Placuv5}[alkLmod1] and plated onto LB-agar plates supplemented with spectinomycin (100 μg/ml). Transformants were checked for the presence of the correct plasmids by plasmid preparation and analytic restriction analysis. The strain generated thus was named *E. coli* W3110 ΔfadE pCDF{Ptac}[hGLYAT2(co_Ec)-fadD_Ec]{Placuv5}[alkLmod1].

Example 6

**Production of fatty acid/amino acid adducts by *E. coli* strains with deletion in the *fadE* gene, which strains overexpress the *synUcTE* and *fadD* genes in combination with either *hGLYAT2* or *hGLYAT3***

The strains generated in Example 4 were used to study their ability to produce fatty acid/amino acid adducts, proceeding as follows:

Starting from a -80 °C glycerol culture, the strains to be studied were first plated onto an LB-agar plate supplemented with 100 μg/ml ampicillin and 100 μg/ml spectinomycin and incubated overnight at 37 °C. Starting from a single colony in each case, the strains were then grown as a 5-ml preculture in Luria-Bertani broth, Miller (Merck, Darmstadt) supplemented with 100 μg/ml ampicillin and 100 μg/ml spectinomycin. The further culture steps were performed in M9 medium. The medium, composed of 38 mM disodium hydrogenphosphate dihydrate, 22 mM potassium dihydrogenphosphate, 8.6 mM sodium chloride, 37 mM ammonium chloride, 2 % (w/v) glucose, 2 mM magnesium sulphate heptahydrate (all chemicals from Merck, Darmstadt) and 0.1% (v/v) trace element solution, was brought to pH 7.4 with 25 % strength ammonium hydroxide solution. The trace element solution added, composed of 9.7 mM manganese(II) chloride tetrahydrate, 6.5 mM zinc sulphate heptahydrate, 2.5 mM sodium-EDTA (Titriplex III), 4.9 mM boric acid, 1 mM sodium molybdate dihydrate, 32 mM calcium chloride dihydrate, 64 mM iron(II) sulphate heptahydrate and 0.9 mM copper(II) chloride dihydrate, dissolved in 1 M hydrochloric acid (all chemicals from Merck, Darmstadt) was filter-sterilized before being added to the M9 medium. 20 ml of M9 medium supplemented with 100 μg/ml spectinomycin and 100 μg/ml ampicillin were introduced into baffled 100-ml Erlenmeyer flasks and inoculated with 0.5 ml preculture. The flasks were cultured at 37 °C and 200 rpm in a shaker-incubator. After a culture time of 8 hours, 50 ml of M9 medium supplemented with 100 μg/ml spectinomycin and 100 μg/ml ampicillin were introduced into a baffled 250-ml Erlenmeyer flask and inoculated with the 10-ml culture to achieve an optical density (600 nm) of 0.2. The flasks were cultured at 37 °C and 200 rpm in a shaker-incubator. When an optical density (600 nm) of 0.7 to 0.8 was reached, gene expression was induced by addition of 1 mM IPTG. The strains were cultured for a further 48 hours at 30 °C and 200 rpm. Simultaneously with the induction, 1 g/l glycine was added to some of the cultures. During culturing, samples were taken, and fatty acid/amino acid adducts present were analysed. The results are shown in **Figures. 1** and **2.** It has been possible to demonstrate that both *E.coli* strain W3110 ΔfadE pJ294{Ptac}[synUcTE] / pCDF{Ptac}[hGLYAT2(co_Ec)_fadD_Ec] and *E.coli* strain W3110 ΔfadE pJ294{Ptac}[synUcTE] / pCDF{Ptac}[hGLYAT3(co_Ec)_fadD_Ec] are capable of forming various fatty acid/amino acid adducts, for example lauroyl-glutamic acid, from glucose. By contrast, no such adducts can be found in a cell that, as a negative control, lacks the plasmids (**Figure. 3**). It appears that slight sequence variations, for example amino acid substitutions that distinguish hGLYAT2 from hGLYAT3, do not compromise the ability of the cell to make the fatty acid/acyl amino acid adducts as shown in Table 3.

| Strain | $C_{lauroylglycinate}$ [mg/L] | $C_{myristoylglycinate}$ [mg/L] |
|---|---|---|
| *E.coli* W3110 ΔfadE pJ294{Ptac}[synUcTE] / pCDF{Ptac}[hGLYAT2(co_Ec)_fadD_Ec] | 111 | <2 |
| *E.coli* W3110 ΔfadE pJ294{Ptac}[synUcTE] / pCDF{Ptac}[hGLYAT2(co_Ec)_fadD_Ec] + 1 g/L glycine | 121 | 2.8 |
| *E.coli* W3110 ΔfadE pJ294{Ptac}[synUcTE] / pCDFDuet-1 | n.d. | n.d. |

Table 3: Quantitative determination of fatty acid glycinates after 48 h culture time

Example 7

**Chromatographic quantification of products by HPLC/MS**

The quantification of N-lauroylglycine, N-myristoylglycine and N-palmitoylglycine and the detection of other acylamino acids in fermentation samples was performed by HPLC-ESI/MS. The quantification was performed with the aid of an external calibration (approx. 0.1 - 50 mg/l) for the three target compounds in the "single ion monitoring" mode (SIM). In parallel, a scan was carried out over a mass range m/z = 100-1000 so as to identify further acylamino acids. The samples for the determination of the fatty acid glycinates were prepared as follows: 800 μl of solvent (acetone) and 200 μl of sample were pipetted into a 2-ml reaction vessel. The mixture was shaken in a Retsch mill for 1 minute at 30 Hz and then centrifuged for 5 min at approximately 13 000 rpm. The clear supernatant was removed using a pipette and, after suitable dilution with diluent (80 % acetonitrile/20 % water + 0.1 % formic acid), analyzed. The calibration standards used were likewise dissolved and diluted in this diluent.

The following equipment was employed:

- Surveyor HPLC system (Thermo Scientific, Waltham, Massachusetts, USA) composed of MS pump, Autosampler Plus and PDA Detector Plus
- Mass spectrometer TSQ Vantage with HESI II source (Thermo Scientific, Waltham, Massachusetts, USA)
- HPLC column: 100 x 2 mm Pursuit XRS Ultra C8; 2.8 μm (Agilent, Santa Clara, California, USA)

Chemicals:

- Water from a Millipore system
- Acetonitrile for HPLC (Merck AG, Darmstadt, Germany)
- Formic acid, p.a. grade (Merck, Darmstadt, Germany)
- N-propanol Lichrosolv (Merck, Darmstadt, Germany)

- N-lauroylglycine 99 % (Chem-Impex International, Wood Dale, IL, USA)
- N-myristoylglycine > 98 % (Santa Cruz Biotechnology, Texas, USA)
- N-palmitoylglycine > 99 % (provenance unknown)

The HPLC separation was carried out using the abovementioned HPLC column. The injection volume amounted to 2 μl, the column temperature to 40 °C, the flow rate to 0.3 ml/min. The mobile phase consisted of Eluent A (0.1 % strength (v/v) aqueous formic acid) and Eluent B (75 % acetonitrile/25 % n-propanol (v/v) with 0.1 % (v/v) formic acid). The following gradient profile was used:

Table 4. Gradient profile used in Example 7.

| Time [min] | Eluent A [%] | Eluent B [%] |
|---|---|---|
| 0 | 90 | 10 |
| 1 | 90 | 10 |
| 20 | 5 | 95 |
| 25 | 5 | 95 |

The HPLC/MS analysis was carried out under positive ionization mode with the following parameters of the ESI source:

Spray Voltage: 3500V
Vaporizer Temperature: 50 °C
Sheath Gas Pressure: 40
Aux. Gas Pressure: 10
Capillary Temperature: 250 °C
Sprayer Distance: Ring C

Detection and quantification of the three analytes were performed by "single ion monitoring" (SIM) with the following parameters shown in Table 5.

Table 5. Parameters used in SIM of Example 7.

| Analyte | Ion [M+H] [m/z] | Scan range [m/z] | Scan time [ms] | Resolution Q3 |
|---|---|---|---|---|
| N-lauroylglycine | 258.2 | 0.002 | 50 | 0.7 |
| N-myristoylglycine | 286.2 | 0.002 | 50 | 0.7 |
| N-palmitoylglycine | 314.2 | 0.002 | 50 | 0.7 |

Example 8

**Production of fatty acid amino acid adducts by *E.coli* strains with deletion in the *fadE* gene, which strains overexpress the *synUcTE* and *fadD* genes in combination with *hGLYAT2* or *hGLYAT3* in a parallel fermentation system**

The strains generated in Example 4 were used for studying their ability to produce fatty acid amino acid adducts from glucose. For this purpose, the strain was cultured both in a shake flask and in a fed-batch fermentation. The fermentation was carried out in a parallel fermentation system from DASGIP with 8 bioreactors.
The production cells were prepared as described in Example 6.

The fermentation was performed using 1 l reactors equipped with overhead stirrers and impeller blades. pH and $pO_2$ were measured online for process monitoring. OTR/CTR measurements served for estimating the metabolic activity and cell fitness, inter alia.

The pH electrodes were calibrated by means of a two-point calibration using standard solutions of pH 4.0 and pH 7.0, as specified in DASGIP's technical instructions. The reactors were provided with the necessary sensors and connections as specified in the technical instructions, and the agitator shaft was fitted. The reactors were then charged with 300 ml of water and autoclaved for 20 min at 121 °C to ensure sterility. The $pO_2$ electrodes were connected to the measuring amplifiers and polarized overnight (for at least 6 h). Thereafter, the water was removed under a clean bench and replaced by M9 medium (pH 7.4) composed of $KH_2PO_4$ 3.0 g/l, $Na_2HPO_4$ 6.79 g/l, NaCl 0.5 g/l, $NH_4Cl$ 2.0 g/l, 2 ml of a sterile 1 M $MgSO_4*7H_2O$ solution and 1 ml/l of a filter-sterilized trace element stock solution (composed of HCl (37 %) 36.50 g/l, $MnCl_2*4H_2O$ 1.91 g/l, $ZnSO_4*7H_2O$ 1.87 g/l, ethylenediaminetetraacetic acid dihydrate 0.84 g/l, $H_3BO_3$ 0.30 g/l, $Na_2MoO_4*2H_2O$ 0.25 g/l, $CaCl_2*2H_2O$ 4.70 g/l, $FeSO_4*7H_2O$ 17.80 g/l, $CuCl_2*2H_2O$ 0.15 g/l) with 15 g/l glucose as the carbon source (added by metering in 30 ml/l of a sterile feed solution composed of 500 g/l glucose, 1.3 % (w/v) $MgSO_4*7H_2O$) supplemented with 100 mg/l spectinomycin and 3 ml/l DOW1500.

Thereafter, the $pO_2$ electrodes were calibrated to 100 % with a one-point calibration (stirrer: 400 rpm/aeration: 10 sl/h air), and the feed, correction agent and induction agent lines were cleaned by "cleaning in place" as specified in the technical instructions. To this end, the tubes were rinsed first with 70 % ethanol, then with 1 M NaOH, then with sterile fully-demineralized water and, finally, filled with the respective media.

Using the *E. coli* strain of Example 4, a dilution streak was first performed with a cryoculture on an LB agar plate supplemented with 100 mg/l spectinomycin, and the plate was incubated for approximately 16 h at 37 °C. LB medium (10 ml in a 100-ml baffle flask) supplemented with 100 mg/l spectinomycin was then inoculated with a single colony and the culture was grown overnight at 37 °C and 200 rpm for approximately 16 h. Thereafter, this culture was used for a second preculture stage with an initial OD of 0.2 in 50 ml of M9 medium, composed of $KH_2PO_4$ 3.0 g/l, $Na_2HPO_4$ 6.79 g/l, NaCl 0.5 g/l, $NH_4Cl$ 2.0 g/l, 2 ml of a sterile 1 M $MgSO_4*7H_2O$ solution and 1 ml/l of a filter-sterilized trace element stock solution (composed of HCl (37 %) 36.50 g/l, $MnCl_2*4H_2O$ 1.91 g/l, $ZnSO_4*7H_2O$ 1.87 g/l, ethylenediaminetetraacetic acid dihydrate 0.84 g/l, $H_3BO_3$ 0.30 g/l, $Na_2MoO_4*2H_2O$ 0.25 g/l, $CaCl_2*2H_2O$ 4.70 g/l, $FeSO_4*7H_2O$ 17.80 g/l, $CuCl_2*2H_2O$ 0.15 g/l) supplemented with 20 g/l glucose as carbon source (added by metering in 40 ml/l of a sterile feed solution composed of 500 g/l glucose) together with the above-described antibiotics was transferred into a 500-ml baffle flask and incubated for 8-12 h at 37 °C/200 rpm. To inoculate the reactors with an optical density of 0.1, the $OD_{600}$ of the second preculture stage was measured and the amount of culture required for the inoculation was calculated. The required amount of culture was placed into the heated and aerated reactor with the aid of a 5-ml syringe through a septum.

The standard program shown in Table 6a-c was used:

a)

| DO controller | | pH controller | |
|---|---|---|---|
| Preset | 0 % | Preset | 0 ml/h |
| P | 0.1 | P | 5 |
| Ti | 300 s | Ti | 200 s |
| Min | 0 % | Min | 0 ml/h |
| Max | 100 % | Max | 40 ml/h |

b)

| N (Rotation) | from | to | XO2 (gas mixture) | from | to | F (gas flow) | from | to |
|---|---|---|---|---|---|---|---|---|
| Growth and biotrans-formation | 0 % | 30 % | Growth and biotrans-formation | 0 % | 100 % | Growth and biotrans-formation | 15 % | 80 % |
| | 400 rpm | 1500 rpm | | 21 % | 21 % | | 6 sl/h | 72 sl/h |

c)

| Script | |
|---|---|
| Trigger fires | 31 % DO (1/60h) |
| Induction IPTG | 2 h after the feed start |
| Feed trigger | 50 % DO |
| Feed rate | 3 [ml/h] |

Table 6. Standard program for use of heated and aerated reactor in Example 8

The pH was adjusted unilaterally to pH 7.0 with 12.5 % strength ammonia solution. During the growth phase and the biotransformation, the dissolved oxygen ($pO_2$ or DO) in the culture was adjusted to at least 30 % via the stirrer speed and the aeration rate. After the inoculation, the DO dropped from 100 % to these 30 %, where it was maintained stably for the remainder of the fermentation.

The fermentation was carried out as a fed batch, the feed start as the beginning of the feed phase with 5 g/l*h glucose feed, composed of 500 g/l glucose, 1.3 % (w/v) $MgSO_4*7H_2O$, being triggered via the DO peak which indicates the end of the batch phase. From the feed start onwards, the temperature was reduced from 37 °C to 30 °C. 2 h after the feed start, the expression was induced with 1 mM IPTG.

To quantify lauroyl, myristoyl and palmitoyl glycinate, samples were taken 47 h and 64 h after the start of the fermentation. These samples were prepared for analysis, and analyzed as described in Example 7.
It has been possible to demonstrate that strain *E. coli* W3110 ΔfadE pJ294{Ptac}[synUcTE] / pCDF{Ptac}[hGLYAT2(co_Ec)_fadD_Ec] is capable of forming lauroyl glycinate from glucose.

Table 9: Quantification of fatty acid glycinates after 47 and 64 h fermentation time.

| Analyte | Ion [M+H] [m/z] | Scan range [m/z] | Scan time [ms] | Resolution Q3 |
|---|---|---|---|---|
| N-Lauroylglycine | 258.2 | 0.002 | 50 | 0.7 |
| N-Myristoylglycine | 286.2 | 0.002 | 50 | 0.7 |
| N-Palmitoylglycine | 314.2 | 0.002 | 50 | 0.7 |

| Fermentation time [h] | $C_{\text{Lauroyl glycinate}}$ [g/l] | $C_{\text{Myristoyl glycinate}}$ [g/l] | $C_{\text{Palmitoyl glycinate}}$ [g/l] | $C_{\text{Glycine}}$ [g/l] | $C_{\text{Octanoic acid}}$ [g/l] | $C_{\text{Decanoic acid}}$ [g/l] | $C_{\text{Lauric acid}}$ [g/l] | $C_{\text{Myristic acid}}$ [g/l] | $C_{\text{Palmitoleic acid}}$ [g/l] | $C_{\text{Palmitic acid}}$ [g/l] | $C_{\text{Oleic acid}}$ [g/l] | $C_{\text{Stearic acid}}$ [g/l] |
|---|---|---|---|---|---|---|---|---|---|---|---|---|
| 47 | 0.63 | 0.07 | n.d. | n.d. | n.d. | 0.001 | n.d. | n.d. | n.d. | 0.002 | 0.006 | 0.001 |
| 64 | 1.06 | 0.11 | n.d. | n.d. | n.d. | n.d. | 0.001 | n.d. | n.d. | n.d. | 0.002 | n.d. |

<u>Table 10.</u> Production of fatty acids after 47 and 64 hours' fermentation time. (n. d.: not determined)

<u>Example 9</u>

The strain of Example 5 was fermented in a fed-batch fermentation to study the ability of linking lauric acid and glycine to give lauroyl glycinate. This fermentation was carried out in a parallel fermentation system from DASGIP with 8 bioreactors.

The experimental setting was as described in Example 8 except that 100 g/l glycine in demineralized water and 100 g/l laurate in lauric acid methyl ester were fed rather than glucose.. To quantify lauroyl, myristoyl and palmitoyl glycinate in fermentation samples, samples were taken 23 h and 42 h after the start of the fermentation. These samples were prepared for analysis, and analyzed as described in Example 7. The results are shown in Tables 11 and 12.

It has been possible to demonstrate that the strain *E.coli* W3110 ΔfadE pCDF{Ptac}[hGLYAT2(co_Ec)_fadD_Ec]{Plavuv5} [alkLmod1] is capable of linking lauric acid and glycine and of producing lauroyl glycinate.

| Fermentation time [h] | $C_{Lauroyl\ glycinate}$ [g/l] | $C_{Myristoyl\ glycinate}$ [g/l] | $C_{Palmitoyl\ glycinate}$ [g/l] | $C_{Glycine}$ [g/l] | $C_{Octanoic\ acid}$ [g/l] | $C_{Decanoic\ acid}$ [g/l] | $C_{Lauric\ acid}$ [g/l] | $C_{Myristic\ acid}$ [g/l] | $C_{Palmitoleic\ acid}$ [g/l] | $C_{Palmitic\ acid}$ [g/l] | $C_{Oleic\ acid}$ [g/l] | $C_{Stearic\ acid}$ [g/l] |
|---|---|---|---|---|---|---|---|---|---|---|---|---|
| 21 | 1.02 | n.d. | n.d. | n.d. | n.d. | n.d. | 6.38 | n.d. | n.d. | n.d. | n.d. | n.d. |
| 40 | 1.78 | n.d. | n.d. | n.d. | n.d. | n.d. | 6.79 | n.d. | n.d. | n.d. | n.d. | n.d. |

Table 11. Production of lauroyl glycinate after fermentation for 23 and 42 hours with feeding of lauric acid and glycine.

| Fermentation time [h] | $C_{Lauryl\ glycinate}$ [g/l] | $C_{Myristyl\ glycinate}$ [g/l] | $C_{Palmityl\ glycinate}$ [g/l] | $C_{Glycine}$ [g/l] | $C_{Octanoic\ acid}$ [g/l] | $C_{Decanoic\ acid}$ [g/l] | $C_{Lauric\ acid}$ [g/l] | $C_{Myristic\ acid}$ [g/l] | $C_{Palmitoleic\ acid}$ [g/l] | $C_{Palmitic\ acid}$ [g/l] | $C_{Oleic\ acid}$ [g/l] | $C_{Stearic\ acid}$ [g/l] |
|---|---|---|---|---|---|---|---|---|---|---|---|---|
| 21 | n.d. | n.d. | n.d. | n.d. | n.d. | n.d. | n.d. | n.d. | n.d. | n.d. | n.d. | n.d. |
| 40 | n.d. | n.d. | n.d. | n.d. | n.d. | n.d. | n.d. | n.d. | n.d. | n.d. | n.d. | n.d. |

Table 12. Production of lauroyl glycinate after fermentation for 23 and 42 hours without feeding of lauric acid and glycine (negative control).

Example 10

**Generation of vectors for expression of the *Homo sapiens* genes hGLYAT3 and hGLYAT2 in *E.coli* strains producing fatty acids via Malonyl-CoA and Acetyl-CoA**

To generate vectors for the expression of the *Homo sapiens* genes hGLYAT2 (SEQ ID NO: 4) or hGYLAT3 (SEQ ID NO: 5) in the fatty acid producing strains listed in Table 13 below.(From Table 3.2 of WO2014026162A1), the genes hGLYAT2 and hGYLAT3 were first amplified. The gene hGLYAT2 was amplified from the plasmid pCDF{Ptac}[hGLYAT2(co_Ec)-fadD_Ec]{Placuv5}[alkLmod1] (SEQ ID NO: 13) and the hGYLAT3 gene was amplified from the plasmid pCDF{Ptac}[hGLYAT3(co_Ec)-fadD_Ec] (SEQ ID No: 9) by means of sequence-specific oligonucleotides. The PCR products were cleaved with the restriction endonucleases *NotI* and *SacI* and ligated in the correspondingly cleaved vector pET-28b (SEQ ID NO: 14). The correct insertion of the target genes was checked by restriction analysis and the authenticity of the introduced genes was verified by DNA sequencing. The resulting expression vectors were named pET-28b{Ptac}[hGLYAT2(co_Ec)] (SEQ ID No: 15) and pET-28b{Ptac}[hGLYAT3(co_Ec)] (SEQ ID No: 16).

Example 11

**Production of fatty acid amino acid adducts via Malonyl-CoA and Acetyl-CoA by strains overexpressing hGLYAT2 or hGLYAT3 in a shake flask experiment**

The vectors produced according to Example 10 were then used to generate a microorganism strain from Table 13 below (OPX Biotechnologies Inc., USA) using any transformation method known in the art. In particular, the methods provided in section IV of WO2014026162A1 were used.

The strains generated were used for studying their ability to produce fatty acids, in particular amino acid adducts from glucose. For this purpose, the strains were transformed with the vectors pET-28b{Ptac}[hGLYAT2(co_Ec)] (SEQ ID NO: 15) and pET-28b{Ptac}[hGLYAT3(co_Ec)] (SEQ ID NO: 16) and cultured in shake flasks (Subsection C of section IV of WO2014026162A1). Strain BXF_031 (OPX Biotechnologies Inc., USA) harbouring the empty vector pET-28b was used as a control.

Triplicate evaluations were performed. Briefly, overnight starter cultures were made in 50 ml of Terrific Broth including the appropriate antibiotics and incubated 16-24 hours at 30°C., while shaking at 225 rpm. These cultures were used to inoculate 150 ml cultures of each strain in SM11 minimal medium to an $OD_{600}$ of 0.8 and 5% TB culture carryover as starting inoculum, and antibiotics. 1 L SM11 medium consists of: 2 ml FM10 Trace Mineral Stock, 2.26 ml 1M $MgSO_4$, 30 g glucose, 200 mM MOPS (pH 7.4), 1 g/L yeast extract, 1.25 ml VM1 Vitamin Mix, 0.329 g $K_2HPO_4$, 0.173 g $KH_2PO_4$, 3 g $(NH_4)_2SO_4$, 0.15 g citric acid (anhydrous); FM10 Trace Mineral Stock consists of: 1 ml of concentrated HCl, 4.9 g $CaCl_2*2H_2O$,0.97 g $FeCl_3*6H_2O$, 0.04 g $CoCl_2*6H_2O$, 0.27 g $CuCl_2*2H_2O$, 0.02 g $ZnCl_2$, 0.024 g $Na_2MoO_4*2H_2O$, 0.007 g $H_3BO_3$, 0.036 g MnCl2*4$H_2O$, Q.S. with DI water to 100 ml; VM1 Vitamin Mix Solution consists of: 5 g Thiamine, 5.4 g Pantothenic acid, 6.0 g Niacin, 0.06 g, Q.S. with DI water to 1000 ml. All ingredients for the culture mediums used in this example are provided in (Subsection A of section IV of WO2014026162A1).

Cultures were incubated for 2 hours at 30°C., while shaking at 225 rpm. After 2 hours, the cells were washed with SM11 (SM11 medium without phosphate). Cells were twice spun down (4,000 rpm, 15 min), the supernatant decanted, the pellet re-suspended in 150 ml of SM11 (SM11 medium without phosphate). The cultures were used to inoculate 3×50 ml of each strain in SM11 (no phosphate). The cultures were grown at 30° C. for approximately 2 h to an $OD_{600}$ of 1.0-1.5 after 2 h cells and shifted to 37° C. and samples removed periodically for product measurement over the course of 72 hrs.

The quantification of N-lauroylglycine, N-myristoylglycine and N-palmitoylglycine and the detection of other acylamino acids in fermentation samples was performed by HPLC-ESI/MS as described in Example 7.

Table 13. List of microorganism strains that were used to introduce the genes hGLYAT2 or hGLYAT3 in examples 10 and 11. The method of production and the sequences of the strains are provided in Table 3.2 of WO2014026162A1 (OPX Biotechnologies Inc., USA).

| **Strain designation** | **Host** | **Plasmid** | **SEQ ID NOs.** |
|---|---|---|---|
| BXF_0012 | BX_864 | 1)pBMT-3_ccdAB | 17 |
| BXF_0013 | BX_864 | 1)pBMT-3_ccdAB_$P_{T7}$-'tesA | 18 |
| BXF_0014 | BX_864 | 1)pBMT-3_ccdAB_$P_{T7}$-nphT7-hbd-crt-ter | 19 |
| BXF_0015 | BX_864 | 1)pBMT-3_ccdAB_$P_{T7}$-'tesA_PT7-$nph_{T7}$-hbd-crt-ter | 20 |
| BXF_0020 | BX_860 | 1)pBMT-3_ccdAB_PT7-'tesA_PT7-nphT7-hbd-crt-ter | 20 |
| BXF_0021 | BX_876 | 1)pBMT-3_ ccdAB_PT7-'tesAPT7-nphT7-hbd-crt-ter | 20 |
| BXF_0022 | BX_874 | 1)pBMT-3_ccdAB | 17 |
| BXF_0023 | BX_874 | 1)pBMT-3_ccdAB_PT7-'tesA | 18 |
| BXF_0024 | BX_874 | 1)pBMT-3_ccdAB_PT7-'tesA_PT7-nphT7-hbd-crt-ter | 20 |
| BXF_0025 | BX_875 | 1)pBMT-3_ccdAB | 17 |
| BXF_0026 | BX_875 | 1)pBMT-3_ccdAB_PT7-'tesA | 18 |

(continued)

| Strain designation | Host | Plasmid | SEQ ID NOs. |
|---|---|---|---|
| BXF_0027 | BX_875 | 1)pBMT-3_ccdAB_PT7-'tesA_PT7-nphT7-hbd-crt-ter | 20 |
| BXF_0028 | BX_878 | 1)pBMT-3ccdAB-T7-'tesA-PT7_nphT7_hbd_crt_ter | 20 |
| BXF_0028 | BX_878 | 1)pBMT-3_ccdAB_PT7-'tesA_PT7-nphT7-hbd-crt-ter | 20 |
| BXF_0029 | BX_879 | 1)pBMT-3_ccdAB_PT7-'tesA_PT7-nphT7-hbd-crt-ter | 20 |
| BXF_0030 | BX_881 | 1)pBMT-3_ccdAB_PT7-'tesA_PT7-nphT7-hbd-crt-ter | 20 |
| BXF_0031 | BX_864 | 1)pBMT-3_ccdAB_PT7-'tesA_PT7-nphT7-hbd-crt-ter<br>2)pET-28b(empty vector) | 20<br>21 |
| BXF_0033 | BX_878 | 1)pBMT-3_ccdAB_PT7-nphT7-hbd-crt-ter | 19 |
| BXF_0034 | BX_879 | 2)pBMT-3_ccdAB_PT7-nphT7-hbd-crt-ter | 19 |

## Claims

1. A cell expressing an amino acid-N-acyl-transferase, which is preferably recombinant, and an acyl-CoA synthetase, which is preferably recombinant,
wherein the cell has a reduced fatty acid degradation capacity.

2. The cell according to claim 1, wherein the fatty acid degradation capacity is reduced owing to a decrease in activity, compared to the wild type cell, of at least one enzyme selected from the group comprising acyl-CoA dehydrogenase, 2,4-dienoyl-CoA reductase, enoyl-CoA hydratase and 3-ketoacyl-CoA thiolase, preferably acyl-CoA dehydrogenase.

3. The cell according to any of claims 1 to 2, wherein the amino acid-N-acyl-transferase is a human amino acid-N-acyl-transferase, preferably SEQ ID NO 4, SEQ ID NO 5 or a variant thereof.

4. The cell according to any of claims 1 to 3, wherein the cell is a bacterial cell, preferably an enterobacterial cell, more preferably *E. coli.*

5. The cell according to any of claims 1 to 4, wherein the cell is capable of making proteinogenic amino acids and/or fatty acids.

6. The cell according to claim 5, wherein the cell comprises at least one enzyme selected from the group consisting of β-ketoacyl-ACP synthase I, 3-oxoacyl-ACP-synthase I, Malonyl-CoA-ACP transacylase, enoyl ACP reductase, and β-ketoacyl-ACP synthase III capable of making proteinogenic amino acids and/or fatty acids.

7.

8. The cell according to any of the preceding claims, wherein the cell expresses an acyl-CoA thioesterase, which is preferably recombinant and is more preferably SEQ ID NO: 1 or a variant thereof.

9. The cell according to any of the preceding claims, wherein the acyl-CoA synthetase is SEQ ID NO: 6 or a variant thereof.

10. A cell expressing an amino acid-N-acyl-transferase, an acyl-CoA synthetase, and at least one enzyme selected from the group consisting of β-ketoacyl-ACP synthase I, 3-oxoacyl-ACP-synthase I, Malonyl-CoA-ACP transacylase, enoyl ACP reductase, and β-ketoacyl-ACP synthase III, wherein the cell has a reduced fatty acid degradation capacity.

11. A method for producing acyl amino acids, comprising the steps

b) contacting an amino acid and an acyl CoA in the presence of an amino acid-N-acyl-transferase, which is preferably isolated and/or recombinant,
wherein the amino acid-N-acyl-transferase is preferably a human amino acid-N-acyl-transferase, more preferably SEQ ID NO: 4, SEQ ID NO: 5 or a variant thereof, and
wherein the acyl-CoA synthetase is preferably SEQ ID NO 6 or a variant thereof.
or culturing the cell according to any of claims 1 to 9.

**12.** The method according to claim 10, further comprising, in addition to step b), the steps

a) converting a fatty acid to an acyl CoA using an acyl-CoA synthetase, which is preferably isolated and/or recombinant,
and/or
c) hydrogenating.

**13.** The method according to any of claims 10 to 11, wherein at least one of the enzymes, preferably all of them, selected from the group comprising amino acid-N-acyl-transferase and acyl-CoA synthetase is provided in the form of a cell expressing said enzyme or enzymes.

**14.** The method according to claim 12, wherein the cell expressing said enzyme or enzymes is the cell according to any of claims 1 to 9.

**15.** A reaction mixture comprising
an amino acid-N-acyl-transferase, which is preferably isolated and/or recombinant,
an acyl-CoA synthetase, which is preferably isolated and/or recombinant,
an amino acid and
either an acyl CoA or a fatty acid and an acyl-CoA-synthetase,
wherein the amino acid-N-acyl-transferase is preferably a human amino acid-N-acyl-transferase, more preferably SEQ ID NO: 4, SEQ ID NO: 5 or a variant thereof, and wherein the acyl-CoA synthetase is preferably SEQ ID NO: 6 or a variant thereof.

**16.** The reaction mixture according to claim 14, wherein at least one of the enzymes, preferably all of them, selected from the group comprising an amino acid-N-acyl-transferase and an acyl-CoA synthetase is provided in the form of a cell, preferably the cell according to any of claims 1 to 7.

**17.** The method or reaction mixture according to any of claims 10 to 15, wherein the amino acid is a proteinogenic amino acid, preferably selected from the group comprising glycine, glutamine, glutamate, asparagine and alanine and is more preferably glycine.

**18.** The method or reaction mixture according to any of claims 10 to 16, wherein the fatty acid is an unsaturated fatty acid and is preferably selected from the group comprising myristoleic acid, lauroleic acid, palmitoleic acid and cis-vaccenic acid.

**19.** The method or reaction mixture according to any of claims 10 to 17, wherein the fatty acid is a saturated fatty acid and is preferably selected from the group comprising laurate, myristate and palmitate.

**20.** The method or reaction mixture according to any of claims 10 to 18, wherein the fatty acid is provided in the form of an organic phase comprising a liquid organic solvent and the fatty acid, wherein the organic solvent is preferably an ester of the fatty acid.

**21.** A composition comprising
a first acyl amino acid consisting of a saturated acyl having 8 to 16 carbon atoms and glycine,
a second acyl amino acid consisting of an unsaturated acyl having 10 to 18 carbon atoms and glycine,
and optionally a third acyl amino acid consisting of a saturated or unsaturated acyl having 12 carbon atoms and an amino acid selected from the group comprising glutamine, glutamic acid, alanine and asparagine.

**22.** The composition according to claim 20, wherein the first acyl amino acid consists of a saturated acyl having 12 carbon atoms, preferably lauryl, and glycine.

**23.** The composition according to any of claims 20 to 21, wherein the second acyl amino acid consists of an unsaturated acyl having 12 or 14 carbon atoms and glycine.

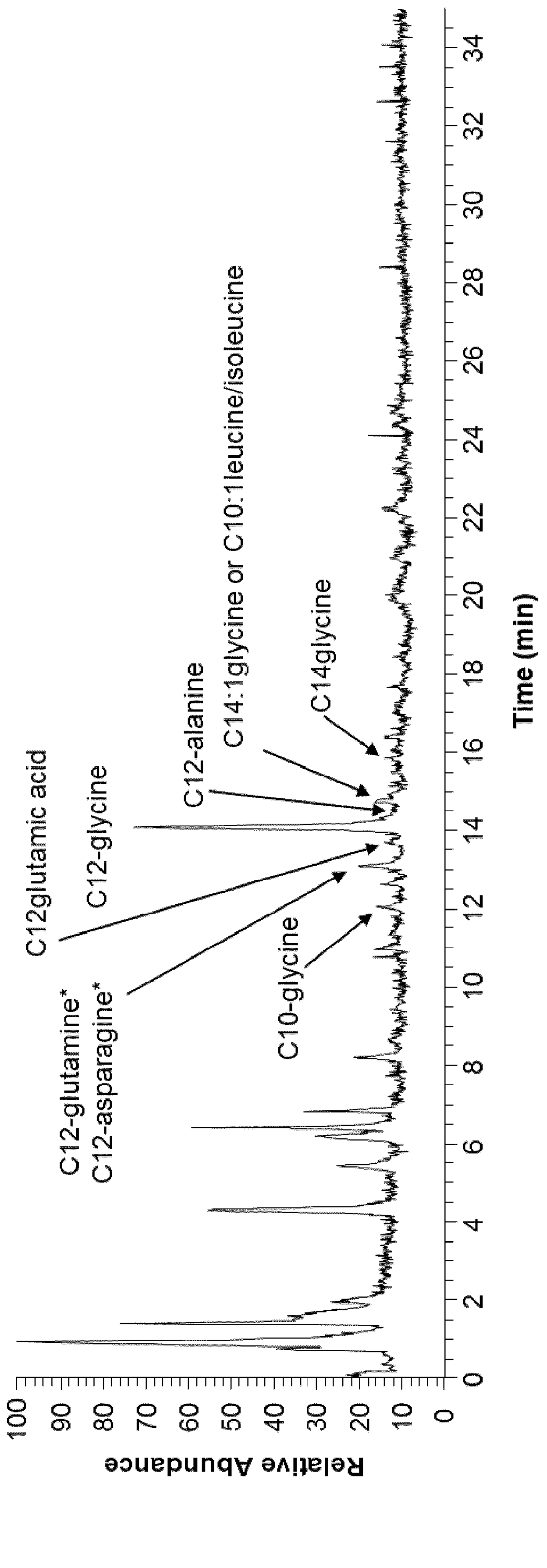
C12glutamic acid
C12-glycine
C12-alanine
C14:1glycine or C10:1leucine/isoleucine
C14glycine
C12-glutamine*
C12-asparagine*
C10-glycine
Relative Abundance
100
90
80
70
60
50
40
30
20
10
0
0
2
4
6
8
10
12
14
16
18
20
22
24
26
28
30
32
34
Time (min)

**Figure 1**

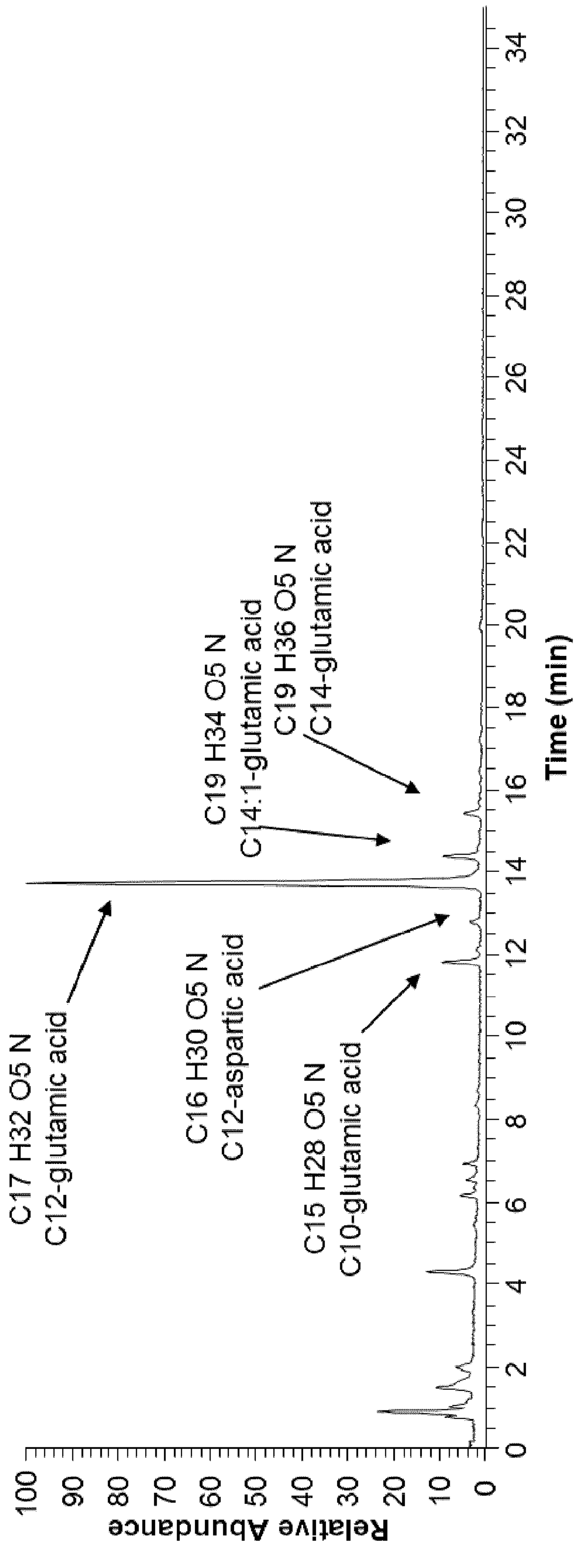
C17 H32 O5 N
C12-glutamic acid
C16 H30 O5 N
C12-aspartic acid
C15 H28 O5 N
C10-glutamic acid
C19 H34 O5 N
C14:1-glutamic acid
C19 H36 O5 N
C14-glutamic acid
Relative Abundance
Time (min)
0
10
20
30
40
50
60
70
80
90
100
0
2
4
6
8
10
12
14
16
18
20
22
24
26
28
30
32
34

Figure 2

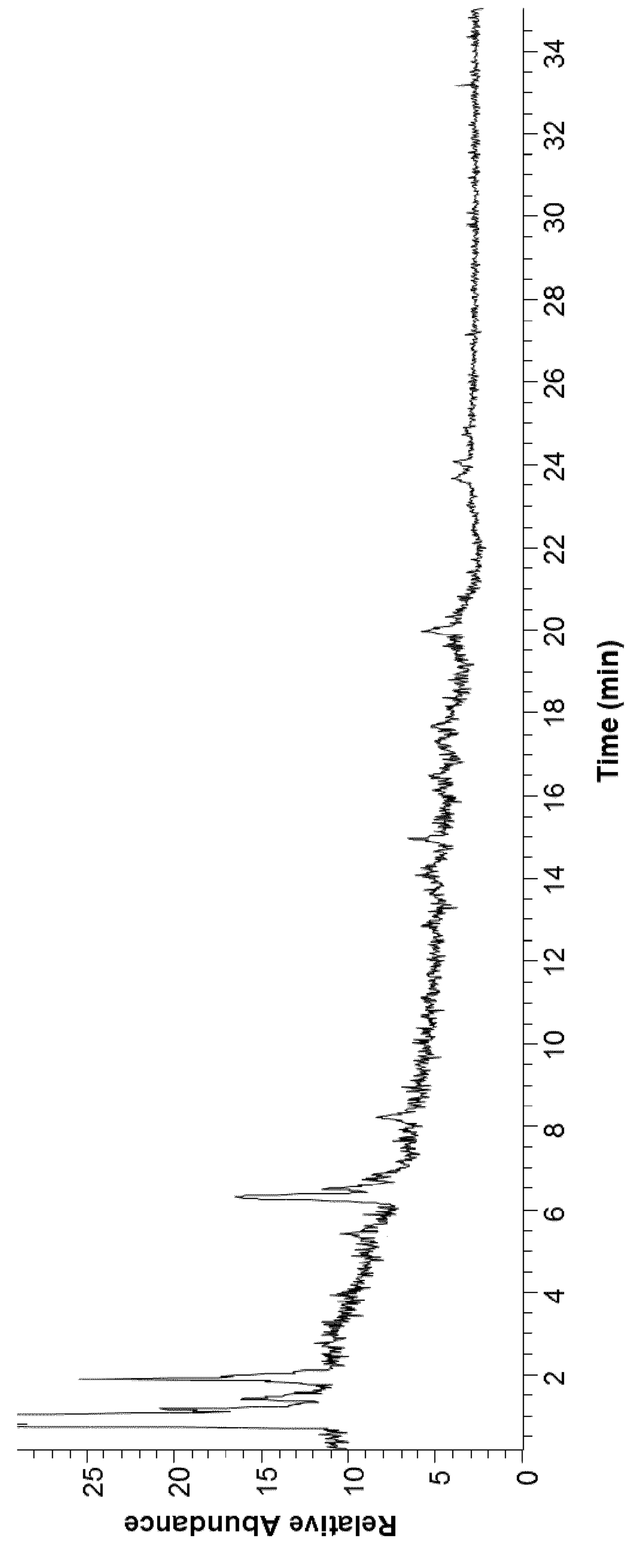
Relative Abundance
0
5
10
15
20
25
2
4
6
8
10
12
14
16
18
20
22
24
26
28
30
32
34
Time (min)

Figure 3

Europäisches Patentamt
European Patent Office
Office européen des brevets

# EUROPEAN SEARCH REPORT

Application Number
EP 14 16 9650

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/131002 A2 (MODULAR GENETICS INC [US]; JARRELL KEVIN [US]; VISHWANATH PRASHANTH [U) 30 October 2008 (2008-10-30)<br>* Whole doc., in particular see para. [0035, 0038-0041] *<br>----- | 11-23 | INV.<br>A61K8/44<br>C12N9/00<br>C12N9/10<br>C12P13/04 |
| E | WO 2014/144649 A1 (MODULAR GENETICS INC [US]) 18 September 2014 (2014-09-18)<br>* Whole doc., in particular para. [0031-0032, 0038] *<br>----- | 11 | |
| X | S. F. BRADY ET AL: "Long-Chain N-Acyltyrosine Synthases from Environmental DNA", APPLIED AND ENVIRONMENTAL MICROBIOLOGY, vol. 70, no. 11, 1 November 2004 (2004-11-01), pages 6865-6870, XP055083151, ISSN: 0099-2240, DOI: 10.1128/AEM.70.11.6865-6870.2004<br>* Whole doc., in particular p.6869; Fig.3 *<br>----- | 11-16, 18-23 | TECHNICAL FIELDS SEARCHED (IPC)<br>A61K<br>C12N<br>C12P |
| X | HUCKLE K R ET AL: "Species variations in the renal and hepatic conjugation of 3-phenoxybenzoic acid with glycine", XENOBIOTICA, TAYLOR AND FRANCIS, LONDON, GB, vol. 11, no. 9, 1 January 1981 (1981-01-01), pages 635-644, XP009181038, ISSN: 0049-8254<br>* Whole doc., in partic. abstract section 3 *<br>-----<br>-/-- | 11,13-23 | |

The present search report has been drawn up for all claims

2

| Place of search | Date of completion of the search | Examiner |
|---|---|---|
| Munich | 10 November 2014 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 16 9650

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | MHASKAR S Y ET AL: "'Synthesis of N-Acyl Amino Acids and Correlation of Structure with Surfactant Properties of Their Sodium Salt'", JOURNAL OF THE AMERICAN OIL CHEMISTS' SOCIETY. (JAOCS) ED.2, SPRINGER, DE, vol. 67, no. 12, 1 December 1990 (1990-12-01), pages 1015-1019, XP008165315, ISSN: 0003-021X, DOI: 10.1007/BF02541868 * the whole document * ----- | 21-23 | |
| X | EIKO WADA ET AL: "Enzymatic synthesis of N-acyl-l-amino acids in a glycerol-water system using acylase I from pig kidney", JOURNAL OF THE AMERICAN OIL CHEMISTS' SOCIETY, vol. 79, no. 1, 1 January 2002 (2002-01-01), pages 41-46, XP055097537, ISSN: 0003-021X, DOI: 10.1007/s11746-002-0432-7 * the whole document * ----- | 21-23 | TECHNICAL FIELDS SEARCHED (IPC) |
| X | EP 0 500 332 A2 (NAT FOOD RESEARCH INST MINISTR [JP]; CCI CORP [JP]) 26 August 1992 (1992-08-26) * the whole document * ----- | 21-23 | |
| X | WO 2012/025895 A2 (UNIV NORTHWEST [ZA]; MIENIE LODEWYK JACOBUS [ZA]; VAN DIJK ALBERDINA A) 1 March 2012 (2012-03-01) * the whole document * ----- | 11-23 | |
| X | EP 0 415 598 A1 (UNILEVER PLC [GB]; UNILEVER NV [NL]) 6 March 1991 (1991-03-06) * the whole document * ----- -/-- | 21-23 | |

2

The present search report has been drawn up for all claims

| Place of search | Date of completion of the search | Examiner |
|---|---|---|
| Munich | 10 November 2014 | Roscoe, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 16 9650

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/016944 A2 (KPSS KAO GMBH [DE]; MOLENDA MICHAEL [DE]; TIETJEN ILKA [DE]) 9 February 2012 (2012-02-09) * p.4-5, claims 8-9; the whole document * | 21-23 | |
| A | PARK S J ET AL: "Engineering of Escherichia coli fatty acid metabolism for the production of polyhydroxyalkanoates", ENZYME AND MICROBIAL TECHNOLOGY, STONEHAM, MA, US, vol. 36, no. 4, 2 March 2005 (2005-03-02), pages 579-588, XP027765871, ISSN: 0141-0229 [retrieved on 2005-03-02] * the whole document * | 10 | |
| A | HANDKE P ET AL: "Application and engineering of fatty acid biosynthesis in Escherichia coli for advanced fuels and chemicals", METABOLIC ENGINEERING, ACADEMIC PRESS, US, vol. 13, no. 1, 1 January 2011 (2011-01-01), pages 28-37, XP027575824, ISSN: 1096-7176 [retrieved on 2010-11-04] * the whole document * | 10 | TECHNICAL FIELDS SEARCHED (IPC) |
| A | US 2005/100995 A1 (WEAVER CRAIG A [US] ET AL) 12 May 2005 (2005-05-12) * the whole document * | 10 | |

The present search report has been drawn up for all claims

| Place of search | Date of completion of the search | Examiner |
|---|---|---|
| Munich | 10 November 2014 | Roscoe, Richard |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 16 9650

This annex lists the patent family members relating to the patent documents cited in the above-mentioned European search report. The members are as contained in the European Patent Office EDP file on
The European Patent Office is in no way liable for these particulars which are merely given for the purpose of information.

10-11-2014

| Patent document cited in search report | Publication date | Patent family member(s) | Publication date |
|---|---|---|---|
| WO 2008131002 A2 | 30-10-2008 | US 2011030102 A1 | 03-02-2011 |
| | | US 2013071885 A1 | 21-03-2013 |
| | | WO 2008131002 A2 | 30-10-2008 |
| WO 2014144649 A1 | 18-09-2014 | NONE | |
| EP 0500332 A2 | 26-08-1992 | DE 69225631 D1 | 02-07-1998 |
| | | DE 69225631 T2 | 17-12-1998 |
| | | EP 0500332 A2 | 26-08-1992 |
| | | US 5503776 A | 02-04-1996 |
| WO 2012025895 A2 | 01-03-2012 | AU 2011294798 A1 | 14-03-2013 |
| | | CO 6700839 A2 | 28-06-2013 |
| | | EP 2609197 A2 | 03-07-2013 |
| | | JP 2013538055 A | 10-10-2013 |
| | | KR 20130094313 A | 23-08-2013 |
| | | US 2013224175 A1 | 29-08-2013 |
| | | WO 2012025895 A2 | 01-03-2012 |
| EP 0415598 A1 | 06-03-1991 | AU 625887 B2 | 16-07-1992 |
| | | AU 6096290 A | 21-02-1991 |
| | | BR 9004038 A | 03-09-1991 |
| | | EP 0415598 A1 | 06-03-1991 |
| | | IN 171130 A1 | 25-07-1992 |
| | | JP H0386812 A | 11-04-1991 |
| | | JP H0460965 B2 | 29-09-1992 |
| | | PH 27209 A | 04-05-1993 |
| | | ZA 9006498 A | 29-04-1992 |
| WO 2012016944 A2 | 09-02-2012 | EP 2415451 A1 | 08-02-2012 |
| | | EP 2600826 A2 | 12-06-2013 |
| | | US 2013156717 A1 | 20-06-2013 |
| | | WO 2012016944 A2 | 09-02-2012 |
| US 2005100995 A1 | 12-05-2005 | AU 2005295598 A1 | 27-04-2006 |
| | | BR PI0516327 A | 02-09-2008 |
| | | CA 2584004 A1 | 27-04-2006 |
| | | CN 101437950 A | 20-05-2009 |
| | | EP 1805315 A2 | 11-07-2007 |
| | | JP 2008515461 A | 15-05-2008 |
| | | KR 20070084187 A | 24-08-2007 |
| | | US 2005100995 A1 | 12-05-2005 |
| | | US 2007266455 A1 | 15-11-2007 |
| | | US 2008026434 A1 | 31-01-2008 |
| | | US 2008026435 A1 | 31-01-2008 |
| | | US 2008026436 A1 | 31-01-2008 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 14 16 9650

This annex lists the patent family members relating to the patent documents cited in the above-mentioned European search report. The members are as contained in the European Patent Office EDP file on
The European Patent Office is in no way liable for these particulars which are merely given for the purpose of information.

10-11-2014

| Patent document cited in search report | Publication date | Patent family member(s) | Publication date |
|---|---|---|---|
| | | US 2008026437 A1 | 31-01-2008 |
| | | US 2008032296 A1 | 07-02-2008 |
| | | US 2008032338 A1 | 07-02-2008 |
| | | US 2008032367 A1 | 07-02-2008 |
| | | US 2008032368 A1 | 07-02-2008 |
| | | US 2008032369 A1 | 07-02-2008 |
| | | US 2008038798 A1 | 14-02-2008 |
| | | US 2008038799 A1 | 14-02-2008 |
| | | US 2008044874 A1 | 21-02-2008 |
| | | US 2008050791 A1 | 28-02-2008 |
| | | WO 2006044646 A2 | 27-04-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1483500 A
- EP 11191520 A
- US 5734070 A
- US 6960455 B
- WO 2014026162 A1


### Non-patent literature cited in the description


- **TAN, B. ; O'DELL, D. K. ; YU, Y. W. ; MONN, M. F. ; HUGHES, H. V. ; BURSTEIN, S. ; WALKER, J.M.** Identification of endogenous acyl amino acids based on a targeted lipidomics approach. *J. Lipid Res.,* 2010, vol. 51 (1), 112-119
- **CLARDY, J. ; BRADY, S. F.** Cyclic AMP directly activates NasP, an N-acyl amino acid antibiotic biosynthetic enzyme cloned from an uncultured beta-proteobacterium. *J. Bacteriol.,* 2007, vol. 189 (17), 6487-6489
- **CRAIG, J. W. ; CHERRY, M. A. ; BRADY, S. F.** Long-chain N-acyl amino acid synthases are linked to the putative PEP-CTERM/exosortase protein-sorting system in Gram-negative bacteria. *J. Bacteriol.,* 2011, vol. 193 (20), 5707-5715
- **WALUK, D. P. ; SCHULTZ, N. ; HUNT, M. C.** Identification of glycine N-acyltransferase-like 2 (GLYATL2) as a transferase that produces N-acyl glycines in humans. *FASEB J.,* 2010, vol. 24, 2795-2803
- **KANG, Y. ; ZARZYCKI-SIEK, J. ; WALTON, C. B. ; NORRIS, M. H. ; HOANG, T. T.** Multiple FadD Acyl-CoA Synthetases Contribute to Differential Fatty Acid Degradation and Virulence in Pseudomonas aeruginosa. *PLOS ONE,* 2010, vol. 5 (10), e13557
- **ARTHUR LESK.** Introduction to bioinformatics. 2008
- **THOMPSON et al.** *Nucleic Acids Research,* 1994, vol. 22, 4637-4680
- **KATOH et al.** *Genome Information,* 2005, vol. 16 (1), 22-33
- **F. M. AUSUBEL.** Current Protocols in Molecular Biology. John Wiley & Sons, Inc, 1995
- The DIG System Users Guide for Filter Hybridization. Boehringer Mannheim GmbH, 1993
- **LIEBL et al.** *International Journal of Systematic Bacteriology,* 1991, vol. 41, 255-260
- **FUJITA, Y. ; MATSUOKA, H. ; HIROOKA, K.** *Mol. Microbiology,* 2007, vol. 66 (4), 829-839
- **CORNISH-BOWDEN.** Fundamentals of Enzyme Kinetics. Portland Press Limited, 1995
- **ANTONENKOV, V., D. ; VAN VELDHOVEN, P., P. ; WAELKENS, E. ; MANNAERTS, G.P.** Substrate specificities of 3-oxoacyl-CoA thiolase and sterol carrier protein 2/3-oxoacyl-coa thiolase purified from normal rat liver peroxisomes. Sterol carrier protein 2/3-oxoacyl-CoA thiolase is involved in the metabolism of 2-methyl-branched fatty acids and bile acid intermediates. *J. Biol. Chem. 1997,* 1997, vol. 272, 26023-26031
- **SAMBROOK ; FRITSCH ; MANIATIS.** Molecular cloning - A Laboratory Manual. Cold Spring Harbour Press, 1989
- **FUCHS ; SCHLEGEL.** Allgemeine Mikrobiologie. Georg Thieme Verlag, 2008
- **JEREMY M BERG ; JOHN L TYMOCZKO ; LUBERT STRYER.** Biochemistry. W. H. Freeman, 2002
- **BEGUIN, P ; AUBERT, J-P.** *FEMS Microbial. Rev.,* 1994, vol. 13, 25-58
- **OHIMA, K. et al.** *Biotechnol. Genet. Eng. Rev.,* 1997, vol. 14, 365414